# EUROPEAN PATENT SPECIFICATION

(11) **EP 2 948 776 B1**
(45) Date of publication and mention of the grant of the patent: **04.12.2019**
(21) Application number: 14743533.3
(22) Date of filing: 24.01.2014
(51) Int. Cl.: G01N 33/574, C12Q 1/68

(54) **METHODS, COMPOSITIONS, KITS, AND SYSTEMS FOR SELECTIVE ENRICHMENT OF TARGET CELLS**
VERFAHREN, ZUSAMMENSETZUNGEN, KITS UND SYSTEME ZUR SELEKTIVEN ANREICHERUNG VON ZIELZELLEN
PROCÉDÉS, COMPOSITIONS, TROUSSES ET SYSTÈMES POUR L'ENRICHISSEMENT SÉLECTIF DE CELLULES CIBLES

(30) Priority: 25.01.2013 US 201361756993 P; 04.02.2013 US 201361760626 P
(43) Date of publication of application: 02.12.2015
(73) Proprietor: Xcell Biosciences, Inc., Berkeley, CA 94710 (US)
(72) Inventor: LIM, James, Oakland, CA 94608 (US)
(74) Representative: Boult Wade Tennant LLP
(86) International application number: PCT/US2014/013048
(87) International publication number: WO 2014/117021

(56) References cited:
- WO-A1-2010/056328
- US-A1- 2001 034 061
- US-A1- 2006 275 836
- US-A1- 2008 176 276
- US-A1- 2010 184 219
- US-A1- 2010 216 168
- US-A1- 2011 020 930
- US-A1- 2012 280 686
- LOUIE ELIZABETH ET AL: "Identification of a stem-like cell population by exposing metastatic breast cancer cell lines to repetitive cycles of hypoxia and reoxygenation", BREAST CANCER RESEARCH, CURRENT MEDICINE GROUP LTD, GB, vol. 12, no. 6, 10 November 2010 (2010-11-10), page R94, XP021085404, ISSN: 1465-5411, DOI: 10.1186/BCR2773
- M. YU ET AL: "Circulating tumor cells: approaches to isolation and characterization", THE JOURNAL OF CELL BIOLOGY, vol. 192, no. 3, 7 February 2011 (2011-02-07), pages 373-382, XP055020222, ISSN: 0021-9525, DOI: 10.1083/jcb.201010021
- Roberto Scatena ET AL: "Circulating tumour cells and cancer stem cells: A role for proteomics in defining the interrelationships between function, phenotype and differentiation with potential clinical applications", BBA - REVIEWS ON CANCER., vol. 1835, no. 2, 1 April 2013 (2013-04-01), pages 129-143, XP55481688, NL ISSN: 0304-419X, DOI: 10.1016/j.bbcan.2012.12.002

### SEQUENCE LISTING

<110> Xcell Biosciences, Inc.
   Lim, James
<120> METHODS, COMPOSITIONS, KITS, AND SYSTEMS FOR SELECTIVE ENRICHMENT OF TARGET CELLS
<130> 43382-701.601
<140> To be Assigned
   <141> 2014-01-22
<150> 61/756,993
   <151> 2013-01-25
<150> 61/760,626
   <151> 2013-02-04
<160> 2
<170> PatentIn version 3.5
<210> 1
   <211> 826
   <212> PRT
   <213> Homo sapiens HIF-1 gamma polypeptide
<400> 1
<210> 2
   <211> 2481
   <212> DNA
   <213> Homo sapiens HIF-1 gamma polynucleotide
<400> 2
   atggagggcg ccggcggcgc gaacgacaag aaaaagataa gttctgaacg tcgaaaagaa      60
   aagtctcgag atgcagccag atctcggcga agtaaagaat ctgaagtttt ttatgagctt     120
   gctcatcagt tgccacttcc acataatgtg agttcgcatc ttgataaggc ctctgtgatg     180
   aggcttacca tcagctattt gcgtgtgagg aaacttctgg atgctggtga tttggatatt     240
   gaagatgaca tgaaagcaca gatgaattgc ttttatttga aagccttgga tggttttgtt 300
   atggttctca cagatgatgg tgacatgatt tacatttctg ataatgtgaa caaatacatg 360
   ggattaactc agtttgaact aactggacac agtgtgtttg attttactca tccatgtgac 420
   catgaggaaa tgagagaaat gcttacacac agaaatggcc ttgtgaaaaa gggtaaagaa 480
   caaaacacac agcgaagctt ttttctcaga atgaagtgta ccctaactag ccgaggaaga 540
   actatgaaca taaagtctgc aacatggaag gtattgcact gcacaggcca cattcacgta 600
   tatgatacca acagtaacca acctcagtgt gggtataaga aaccacctat gacctgcttg 660
   gtgctgattt gtgaacccat tcctcaccca tcaaatattg aaattccttt agatagcaag 720
   actttcctca gtcgacacag cctggatatg aaattttctt attgtgatga aagaattacc 780
   gaattgatgg gatatgagcc agaagaactt ttaggccgct caatttatga atattatcat 840
   gctttggact ctgatcatct gaccaaaact catcatgata tgtttactaa aggacaagtc 900
   accacaggac agtacaggat gcttgccaaa agaggtggat atgtctgggt tgaaactcaa 960
   gcaactgtca tatataacac caagaattct caaccacagt gcattgtatg tgtgaattac 1020
   gttgtgagtg gtattattca gcacgacttg attttctccc ttcaacaaac agaatgtgtc 1080
   cttaaaccgg ttgaatcttc agatatgaaa atgactcagc tattcaccaa agttgaatca 1140
   gaagatacaa gtagcctctt tgacaaactt aagaaggaac ctgatgcttt aactttgctg 1200
   gccccagccg ctggagacac aatcatatct ttagattttg gcagcaacga cacagaaact 1260
   gatgaccagc aacttgagga agtaccatta tataatgatg taatgctccc ctcacccaac 1320
   gaaaaattac agaatataaa tttggcaatg tctccattac ccaccgctga aacgccaaag 1380
   ccacttcgaa gtagtgctga ccctgcactc aatcaagaag ttgcattaaa attagaacca 1440
   aatccagagt cactggaact ttcttttacc atgccccaga ttcaggatca gacacctagt 1500
   ccttccgatg gaagcactag acaaagttca cctgagccta atagtcccag tgaatattgt 1560
   ttttatgtgg atagtgatat ggtcaatgaa ttcaagttgg aattggtaga aaaacttttt 1620
   gctgaagaca cagaagcaaa gaacccattt tctactcagg acacagattt agacttggag 1680
   atgttagctc cctatatccc aatggatgat gacttccagt tacgttcctt cgatcagttg 1740
   tcaccattag aaagcagttc cgcaagccct gaaagcgcaa gtcctcaaag cacagttaca 1800
   gtattccagc agactcaaat acaagaacct actgctaatg ccaccactac cactgccacc 1860
   actgatgaat taaaaacagt gacaaaagac cgtatggaag acattaaaat attgattgca 1920
   tctccatctc ctacccacat acataaagaa actactagtg ccacatcatc accatataga 1980
   gatactcaaa gtcggacagc ctcaccaaac agagcaggaa aaggagtcat agaacagaca 2040
   gaaaaatctc atccaagaag ccctaacgtg ttatctgtcg ctttgagtca aagaactaca 2100
   gttcctgagg aagaactaaa tccaaagata ctagctttgc agaatgctca gagaaagcga 2160
   aaaatggaac atgatggttc actttttcaa gcagtaggaa ttggaacatt attacagcag     2220
   ccagacgatc atgcagctac tacatcactt tcttggaaac gtgtaaaagg atgcaaatct     2280
   agtgaacaga atggaatgga gcaaaagaca attattttaa taccctctga tttagcatgt     2340
   agactgctgg ggcaatcaat ggatgaaagt ggattaccac agctgaccag ttatgattgt     2400
   gaagttaatg ctcctataca aggcagcaga aacctactgc agggtgaaga attactcaga     2460
   gctttggatc aagttaactg a                                               2481

## Description

### BACKGROUND OF THE INVENTION

Enriched subpopulations of cells from a heterogeneous population of cells are often required for many clinical and research applications. Many subpopulations of cells exist in bodily fluids, including blood. Isolating and examining subpopulations of cells from blood is an attractive option due to the minimally-invasive nature of a blood draw. Examples of useful cell subpopulations found in the blood can include circulating tumor cells (CTCs), cancer stem cells (CSCs), hematopoietic stem cells (HSCs), and endothelial progenitor cells (EPCs).

Recent developments in the life sciences have highlighted the need for methods and systems to rapidly and efficiently isolate, characterize, and enrich cell subpopulations from a heterogeneous population without compromising the integrity of the cells. However, a critical problem for the field has been an inability to capture and enrich rare target cell subpopulations in sufficient quantities to conduct meaningful research. The current detection methods for isolating target cell subpopulations often rely on the use of antibodies, ability of the cells to invade a matrix, or complex microfluidic devices that have led to inconsistent findings and limited sample. For example, currently available methods for isolating cell subpopulations rely on antibodies for affinity purification or identification. Reliance on antibodies can be problematic because cells may not always express the same cell-surface antigens. Indeed, studies show that CTCs and CSCs with metastatic potential reduce EpCAM expression. Furthermore, cell subpopulations such as CTCs and CSCs can have diverse molecular phenotypes which can change over time. Therefore methods to capture, detect, and enrich for certain cell subpopulations, which do not rely on antibody markers, may have utility in a diverse range of applications.

### SUMMARY OF THE INVENTION

The invention provides a method for selective enrichment of circulating tumor cells from a heterogeneous cell population originating from a biological sample comprising a bodily fluid, the method comprising:
(a) contacting said heterogeneous cell population with a substrate; and
(b) incubating said heterogeneous cell population under conditions sufficient to allow growth of said circulating tumor cells, wherein said conditions comprise incubating said heterogeneous cell population under a pressurized condition of 103.4 kPa (15 PSIA) or higher and hypoxic conditions, wherein said incubating results in a selective enrichment of said circulating tumor cells.
Aspects of the present disclosure relate to the enrichment, identification, isolation, and/or culture of target cell subpopulations. For example, the invention provides a method to capture and enrich target cell subpopulations from a heterogeneous cell population, comprising: contacting the cells from the heterogeneous population of cells to a substrate; and incubating the cells in enrichment medium for a time sufficient to allow enrichment of the target subpopulation of cells, wherein the target subpopulation of cells are isolated based their physical and kinetic properties.

Described herein is a method to capture, enrich, and identify a target subpopulation of cells from a heterogeneous cell population, comprising: contacting the cells from the heterogeneous cell population to a substrate; incubating the target subpopulation of cells in enrichment media for a time sufficient to allow enrichment of the target subpopulation of cells; using an apparatus to obtain images of the target cell subpopulation over time; and using software to perform analysis of the images, wherein the software can automatically classify cells based on their physical and kinetic properties. In some instances, the software integrates parameters based on physical properties, wherein the parameters are chosen from a group comprising: overall cell size, cell shape, cell movement, cell nucleus size, and cell division. In some instances, the software detects fluorescent labeling of cells.

Described herein is a method to capture and enrich CTCs from a human subject, comprising: drawing blood from the human; contacting the cells contained within the blood to a substrate; incubating the cells in a media for a time sufficient to allow enrichment of CTCs; and using an apparatus to automatically perform image analysis of the CTCs and classification of CTCs wherein the classification is used to direct treatment for a cancer patient.

In practicing any of the foregoing methods, the target cell subpopulation can be selected from the group consisting of: circulating tumor cells (CTCs), cancer stem cells (CSCs), cells from a solid tumor, hematopoietic stem cells (HSCs), primary neuronal cells, fetal cells, stem cells, adult stem cells, immune cells, primary hepatocytes, primary cardiomyocytes, endothelial progenitor cells (EPCs), cancer cells, and epithelial progenitor cells.

In practicing any of the foregoing methods, the heterogeneous cell population can be comprised of a bodily fluid, or derivatives thereof. In some embodiments, the heterogeneous cell population is comprised of a bodily fluid, or derivatives thereof and cells from a different source that have the ability to be marked. In some embodiments, one or more cell from the target cell subpopulation is isolated and cultured.

In practicing any of the foregoing methods, the substrate can be selected from the group comprising: GOL Substrate; GEL Substrate; and a cell. In some embodiments, the configuration of the substrate is selected from the group comprising: single layer, two layer, middle layer(s) and inverted configuration.

In some embodiments of any of the foregoing methods, the enrichment media is selected from the group comprising: Plating Culture Medium; Type R Long Term Growth Medium; Type DF Long Term Growth Medium; Type D Long Term Growth Medium; MEF - Enrichment Medium; and any combination thereof. In some embodiments, the one or more individual cells are isolated using micromanipulation.

In some embodiments of the foregoing methods, one or more cells from the target cell population are isolated using micromanipulation.

Any of the foregoing methods of the invention can be used in a variety of clinical applications. In some embodiments, an invention method is used to monitor patients to investigate the presence of cancer. In some embodiments, serial testing of blood is conducted on a reoccurring basis to predict the likelihood of cancer relapse. In some embodiments, the isolated cell is optionally cultured used for a pharmacological assay to determine the best mode of treatment for a cancer patient. In some embodiments, the isolated cell is propagated to generate a cell culture. In some embodiments, the cell culture is used for research of cancer. In some embodiments, the classification of cells directs diagnosis, prognosis, or theranosis of a cancer patient. In some embodiments, the method comprises transmitting data relating to the classification over a network.

Described herein is a computer readable medium comprising code that, upon execution by one or more processors, implements any of the foregoing methods.

Described herein is a system for implementing the method of the invention comprising: a storage memory for storing a dataset associated with a sample; a processor communicatively coupled to the storage memory; and the dataset.

Described herein is a method for selective enrichment of a target cell subpopulation from a heterogeneous cell population originating from a biological sample, the method comprising: contacting the heterogeneous cell population with a substrate; and incubating the heterogeneous cell population under conditions sufficient to allow growth of the target cell subpopulation, wherein the conditions comprise incubating the heterogeneous cell population under positive pressure conditions and/or hypoxic conditions, wherein the incubating results in a selective enrichment of the target cell subpopulation.

In some embodiments, the method further comprises identifying a member of the target cell subpopulation upon a display of one or more morphological characteristics and/or kinetic properties by the member of the target cell subpopulation, wherein the one or more morphological characteristics and/or kinetic properties is selected from the group consisting of colony formation, proliferation, large surface area as compared to a surface area of a non-target cell, increased adherence to the substrate as compared to an adherence of a non-target cell to the substrate, rapid cell division, and multinucleation. In some embodiments, the identifying does not comprise use of an antibody or polynucleotide marker.

The target cell subpopulation may comprise primary cells. The target cell subpopulation may comprise cells selected from the group consisting of: circulating tumor cells (CTCs), cancer stem cells (CSCs), cells from a solid tumor, hematopoietic stem cells (HSCs), primary neuronal cells, fetal cells, stem cells, adult stem cells, immune cells, primary hepatocytes, primary cardiomyocytes, endothelial progenitor cells (EPCs), cancer cells, and epithelial progenitor cells. The member of the target cell subpopulation may be a CTC or CSC. In some embodiments, the CTC is a metastatic CTC. In some embodiments, the CTC is a viable CTC. In some embodiments, the CTC is an acute leukemia cell, acute t-cell leukemia cell, acute lymphocytic leukemia cell, acute myelocytic leukemia cell, myeloblastic leukemia cell, promyelocytic leukemia cell, myelomonocytic leukemia cell, monocytic leukemia cell, erythroleukemia cell, chronic leukemia cell, chronic myelocytic (granulocytic) leukemia cell, chronic lymphocytic leukemia cell, polycythemia vera cell, lymphoma cell, multiple myeloma cell, Waldenstrom's macroglobulinemia cell, heavy chain disease cell, sarcoma cell, fibrosarcoma cell, myxosarcoma cell, liposarcoma cell, chondrosarcoma cell, osteogenic sarcoma cell, lymphangiosarcoma cell, mesothelioma cell, Ewing's tumor cell, leiomyosarcoma cell, rhabdomyosarcoma cell, colon carcinoma cell, colorectal cancer cell, pancreatic cancer cell, breast cancer cell, ovarian cancer cell, prostate cancer cell, castration resistant prostate cancer cell, squamous cell carcinoma cell, basal cell carcinoma cell, adenocarcinoma cell, sweat gland carcinoma cell, sebaceous gland carcinoma cell, papillary carcinoma cell, papillary adenocarcinomas cell, cystadenocarcinoma cell, medullary carcinoma cell, bronchogenic cell, renal cell carcinoma cell, hepatoma cell, bile duct carcinoma cell, choriocarcinoma cell, seminoma cell, embryonal carcinoma cell, Wilms' tumor cell, cervical cancer cell, uterine cancer cell, testicular tumor cell, lung carcinoma cell, small cell lung carcinoma cell, bladder carcinoma cell, epithelial carcinoma cell, glioma cell, craniopharyngioma cell, ependymoma cell, pinealoma cell, hemangioblastoma cell, acoustic neuroma cell, oligodendroglioma cell, meningioma cell, melanoma cell, neuroblastoma cell, retinoblastoma cell, endometrial cancer cell, non small cell lung cancer cell, head and neck cancer cell, or kidney cancer cell. In some embodiments, the non-target primary cell subpopulation comprises white blood cells, senescent epithelial cells, fibroblasts, non-viable cells, or fragmented cells.

In some embodiments, the biological sample comprises a bodily fluid, or a derivative thereof. In some embodiments, the bodily fluid is selected from blood, plasma, serum, lymph, saliva, urine, sweat, or ascites. In some embodiments, the member of the target cell subpopulation is present at a concentration of 1 target cell/ml or less of the bodily fluid or derivative thereof. In some embodiments, the member of the target cell subpopulation is present at a concentration of 0.8 target cell/ml or less of the bodily fluid or derivative thereof. In some embodiments, the member of the target cell subpopulation is present at a concentration of 0.6 target cell/ml or less of the bodily fluid or derivative thereof. In some embodiments, the biological sample comprises a solid tissue sample.

In some embodiments, the substrate comprises a coating, wherein the coating is selected from the group consisting of a GOL substrate, GEL substrate, one or more layers of feeder cells, and any combination thereof. In some embodiments, the configuration of the substrate is selected from the group consisting of: single layer configuration, two layer configuration, middle layer(s) configuration, and inverted configuration.

In some embodiments, the method further comprises individually isolating the member of the target cell subpopulation. In some embodiments, the method comprises individually isolating and culturing the member of the target cell subpopulation. In some embodiments, the isolating comprises use of a micromanipulation apparatus.

In some embodiments, the method comprises culturing the member of the target cell subpopulation as a cell culture for over 2 weeks.

In some embodiments, the hypoxic conditions comprise culturing under O₂ levels that are less than 10%. In some embodiments, the O₂ levels are 1%.

In some embodiments, the positive pressure conditions comprise culturing the member under pressurized conditions of above 14.7 PSIA. In some embodiments, the positive pressure conditions comprise culturing the member under pressurized conditions of 15 PSIA or higher. In some embodiments, the positive pressure conditions comprise culturing the member under pressurized conditions of 15.7 PSIA or higher. In some embodiments, the positive pressure conditions comprise culturing the member under pressurized conditions of 16.7 PSIA or higher. In some embodiments, positive pressure conditions comprise culturing the member under pressurized conditions of 19.7 PSIA.

In some embodiments, the conditions further comprise culturing the member of the target cell subpopulation in a cell culture medium selected from the group consisting of: Plating Culture Medium; Type R Long Term Growth Medium; Type DF Long Term Growth Medium; Type D Long Term Growth Medium; MEF-Conditioned Medium; and any combination thereof. In some embodiments, the conditions further comprise culturing the member of the target cell subpopulation in a cell culture medium comprising EGF, hydrocortisone, progesterone, dihydrotestosterone, and/or any combination thereof.

In some embodiments, conditions comprise culturing the member in a cell culture medium comprising a compound that regulates surface tension of a cell. In some embodiments, the compound is a Rho kinase inhibitor. In some embodiments, the compound is selected from the group consisting of

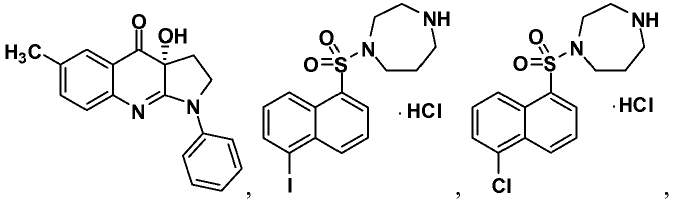

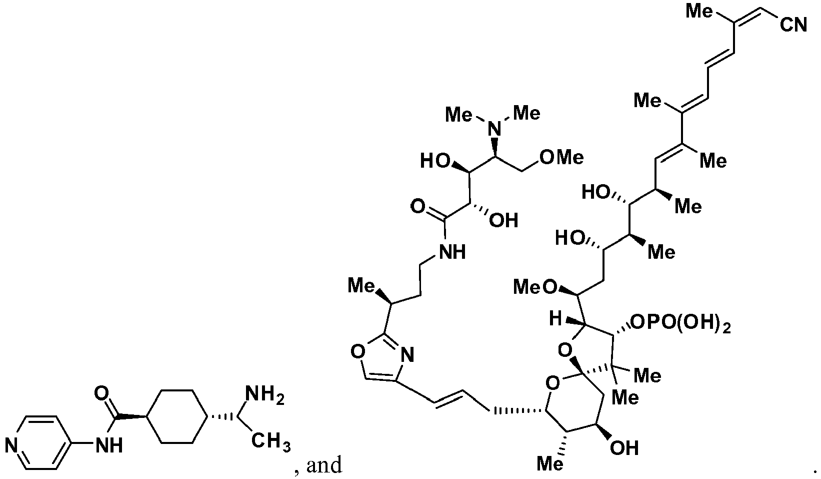

In some embodiments, the conditions comprise culturing the member with an agent that increases expression of a hypoxia-inducible factor in the member. In some embodiments, the hypoxia-inducible factor comprises Hypoxia Inducible Factor-la (HIF-1α), Hypoxia Inducible Factor-1β (HIF-1β), Hypoxia Inducible Factor-2α (HIF-2α), Hypoxia Inducible Factor-2p (HIF-2β), Hypoxia Inducible Factor-3α (HIF-3α), Hypoxia Inducible Factor-3β (HIF-3β), or any combination thereof. In some embodiments, the hypoxia-inducible factor comprises HIF-1α. In some embodiments, the agent that increases expression of a hypoxia-inducible factor comprises a vector, wherein the vector comprises a polynucleotide encoding the hypoxia-inducible factor. In some embodiments, the vector is an expression vector. In some embodiments, the hypoxia-inducible factor comprises an amino acid sequence comprising at least 70% or greater homology to SEQ. ID. NO. 1:

In some embodiments, the polynucleotide comprises at least 70% greater homology or complementarity to SEQ. ID. NO. 2.

In practicing any of the foregoing methods, the method can comprise contacting the cell culture medium with a feeder cell. In some embodiments, the feeder cell is selected from the group consisting of a cultured human umbilical vein endothelial cell (HUVEC), human lung microvascular endothelial cell (HLMVEC), and mouse embryonic fibroblast (MEF).

In some embodiments of any of the methods described herein, the method comprises sequencing the member of the target cell subpopulation. In some embodiments, the method comprises determining expression of one or more genes in the member of the target cell subpopulation. In some embodiments, the method comprises determining expression of one or more proteins in the member of the target cell subpopulation.

In some embodiments, the identifying comprises: obtaining images of the member; and using a computer readable medium to automatically classify the member as belonging to the target cell subpopulation. In some embodiments, the computer readable medium is configured to automatically classify circulating tumor cells (CTCs), cancer stem cells (CSCs), cells from a solid tumor, hematopoietic stem cells (HSCs), primary neuronal cells, fetal cells, stem cells, adult stem cells, immune cells, primary hepatocytes, primary cardiomyocytes, endothelial progenitor cells (EPCs), cancer cells, and epithelial progenitor cells. In some embodiments, the computer readable medium is configured to automatically classify circulating tumor cells (CTCs), cancer stem cells (CSCs), hematopoietic stem cells (HSCs), and endothelial progenitor cells (EPCs). In some embodiments, the computer readable medium is configured to integrate parameters based on physical properties of the target subpopulation of cells, wherein the parameters are chosen from the group consisting In some embodiments, the computer readable medium is configured to detect fluorescent labeling of cells.

Described herein is a method for treating a cancer in a subject in need thereof, comprising administering an anti-cancer agent to the subject upon detection of a target cell in a biological sample originating from the subject, wherein the target cell is detected by a method as described herein.

Described herein is a method of administering an anti-cancer agent to a subject in need thereof, comprising: administering the anti-cancer agent to the subject at multiple time points; analyzing a target cell originating from a biological sample derived from the subject upon at least a first and second time point of the administration, wherein the analyzing comprises the method of any one of the preceding claims; adjusting dosage of the anti-aggregate agent and/or selecting a different anti-aggregate agent for administration based on the analyzing.

Described herein is a method of detecting an increased likelihood of developing a metastatic cancer in a subject that has not developed the metastatic cancer, comprising; isolating a target cell subpopulation from a heterogeneous cell population originating from the subject using a method as described herein; detecting the target cell subpopulation; and designating the subject as having an increased likelihood of developing the metastatic cancer upon the detecting.

Described herein is a method of developing an anti-cancer therapy, comprising: culturing a target cell subpopulation using a method as described herein; contacting the target cell subpopulation with a test agent; assaying viability and/or growth of the target cell subpopulation; and selecting the test agent as an anti-cancer therapy if viability and/or growth of the target cell subpopulation is reduced as compared to the target cell subpopulation that has not been contacted with the test agent.

Described herein is a method of selecting a personalized disease therapy for a subject in need thereof, comprising: culturing a target cell subpopulation using a method as described herein; sequencing nucleic acids from a member of the target cell subpopulation to generate a genetic profile of the member of the target cell subpopulation; and selecting a personalized disease therapy for the subject based on the genetic profile of the member of the target cell subpopulation. In some embodiments, the method further comprises recommending the personalized disease therapy to the subject. In some embodiments, the personalized disease therapy is a personalized cancer therapy.

Described herein is a cell culture incubator, comprising an enclosed environmental chamber; wherein the cell culture incubator is configured to maintain (i) a gas composition of the enclosed environmental chamber, (ii) a user-controlled atmospheric pressure of the enclosed environmental chamber, and (iii) an internal ambient temperature of the enclosed environmental chamber. In some instances, the cell culture incubator comprises one or more control units, wherein the one or more control units is configured to maintain at least one of the gas composition, the user-controlled atmospheric pressure, and the internal ambient temperature. In some instances, the one or more control units are configured to maintain at least two of the gas composition, the user-controlled atmospheric pressure, and the internal ambient temperature. In some instances, the one or more control units are configured to maintain the gas composition, the user-controlled atmospheric pressure, and the internal ambient temperature. In some instances, at least one of the gas composition, the user-controlled atmospheric pressure, and the internal ambient temperature are configured for selective proliferation of a target primary cell subpopulation as compared to a non-target primary cell subpopulation. In some instances, the selective proliferation is evidenced by a two-fold increase in proliferation of the target primary cell subpopulation as compared to proliferation of the non-target primary cell subpopulation. In some instances, least one of the gas composition, the user-controlled atmospheric pressure, and the internal ambient temperature are configured for selective adherence of a target primary cell subpopulation as compared to a non-target primary cell subpopulation. In some instances, the selective adherence is evidenced by a two-fold increase in adherence of the target primary cell subpopulation as compared to adherence of the non-target primary cell subpopulation. In some instances, at least one of the gas composition, the user-controlled atmospheric pressure, and the internal ambient temperature are configured to promote selective colony formation of the target primary cell as compared to colony formation of the non-target primary cell subpopulation. In some instances, the selective colony formation is evidenced by a 2-fold increase in colony formation of the target primary cell subpopulation as compared to colony formation of the non-target primary cell subpopulation. In some instances, the colony formation is a two-dimensional and/or three-dimensional colony formation.

In some instances, the target primary cell subpopulation is selected from the group consisting of circulating tumor cells (CTCs), cancer stem cells (CSCs), cells from a solid tumor, hematopoietic stem cells (HSCs), primary neuronal cells, fetal cells, stem cells, adult stem cells, immune cells, primary hepatocytes, primary cardiomyocytes, endothelial progenitor cells (EPCs), cancer cells, and epithelial progenitor cells. In some instances, the target primary cell subpopulation is selected from the group consisting of circulating tumor cells (CTCs), cancer stem cells (CSCs), hematopoietic stem cells (HSCs), and endothelial progenitor cells (EPCs). In some instances, the target primary cell subpopulation is selected from the group consisting of circulating tumor cells (CTCs), cancer stem cells (CSCs), cells from a solid tumor, hematopoietic stem cells (HSCs), primary neuronal cells, primary fetal cells, primary hepatocytes, primary cardiomyocytes, and endothelial progenitor cells (EPCs). In some instances, the target primary cell subpopulation is selected from the group consisting of CTCs and CSCs. In some embodiments, the circulating tumor cell (CTC) is an acute leukemia cell, acute t-cell leukemia cell, acute lymphocytic leukemia cell, acute myelocytic leukemia cell, myeloblastic leukemia cell, promyelocytic leukemia cell, myelomonocytic leukemia cell, monocytic leukemia cell, erythroleukemia cell, chronic leukemia cell, chronic myelocytic (granulocytic) leukemia cell, chronic lymphocytic leukemia cell, polycythemia vera cell, lymphoma cell, multiple myeloma cell, Waldenstrom's macroglobulinemia cell, heavy chain disease cell, sarcoma cell, fibrosarcoma cell, myxosarcoma cell, liposarcoma cell, chondrosarcoma cell, osteogenic sarcoma cell, lymphangiosarcoma cell, mesothelioma cell, Ewing's tumor cell, leiomyosarcoma cell, rhabdomyosarcoma cell, colon carcinoma cell, colorectal cancer cell, pancreatic cancer cell, breast cancer cell, ovarian cancer cell, prostate cancer cell, castration resistant prostate cancer cell, squamous cell carcinoma cell, basal cell carcinoma cell, adenocarcinoma cell, sweat gland carcinoma cell, sebaceous gland carcinoma cell, papillary carcinoma cell, papillary adenocarcinomas cell, cystadenocarcinoma cell, medullary carcinoma cell, bronchogenic cell, renal cell carcinoma cell, hepatoma cell, bile duct carcinoma cell, choriocarcinoma cell, seminoma cell, embryonal carcinoma cell, Wilms' tumor cell, cervical cancer cell, uterine cancer cell, testicular tumor cell, lung carcinoma cell, small cell lung carcinoma cell, bladder carcinoma cell, epithelial carcinoma cell, glioma cell, craniopharyngioma cell, ependymoma cell, pinealoma cell, hemangioblastoma cell, acoustic neuroma cell, oligodendroglioma cell, meningioma cell, melanoma cell, neuroblastoma cell, retinoblastoma cell, endometrial cancer cell, non small cell lung cancer cell, head and neck cancer cell, or kidney cancer cell. In some embodiments, the non-target primary cell subpopulation comprises white blood cells, senescent epithelial cells, fibroblasts, non-viable cells, or fragmented cells.

In some instances, the gas composition comprises an O₂ level between 0.1-20%. In some instances, the cell culture incubator maintains an O₂ level of the enclosed environmental chamber of no more than 5%. In some instances, the cell culture incubator maintains an O₂ level of the enclosed environmental chamber of no more than 2%. In some instances, the cell culture incubator maintains an O₂ level of the enclosed environmental chamber of no more than 1%.

In some instances, the cell culture incubator maintains a user-controlled atmospheric pressure of the enclosed environmental chamber of 15.7 PSIA or greater. In some instances, the cell culture incubator maintains a user-controlled atmospheric pressure of the enclosed environmental chamber of 16.7 PSIA or greater. In some instances, the cell culture incubator maintains a user-controlled atmospheric pressure of the enclosed environmental chamber of 19.7A PSIA. In some instances, the cell culture incubator maintains the atmospheric pressure by controlling an inlet gas pressure.

In some instances, the cell culture incubator comprises one or more user interfaces. In some instances, the one or more user interfaces is configured to allow a user to control the gas composition. In some instances, the one or more user interfaces is configured to allow a user to control the O₂ level. In some instances, the one or more user interfaces is configured to allow a user to control the atmospheric pressure. In some instances, the one or more user interfaces is configured to provide a display of the gas composition to a user. In some instances, the one or more user interfaces is configured to provide a display of the O₂ level to a user. In some instances, the one or more user interfaces is configured to provide a display of the atmospheric pressure to a user. In some instances, the cell culture incubator is configured to maintain (iv) an internal humidity of the enclosed environmental chamber. In some instances, the enclosed environmental chamber comprises a shelf. In some instances, the enclosed environmental chamber comprises a pressure sensor. In some instances, the enclosed environmental chamber comprises an O₂ sensor. In some instances, the enclosed environmental chamber comprises an oxygen removal catalyst.

In some instances, the enclosed environmental chamber comprises a sterilization unit. In some instances, the sterilization unit is a UV sterilization unit. In some instances, the enclosed environmental chamber comprises a pressurized door. In some instances, the enclosed environmental chamber comprises a sensor which provides a detectable alarm upon detection of an O₂ level of the enclosed environmental chamber that differs by more than ±0.5% from a user-controlled O2 level. In some instances, the enclosed environmental chamber comprises a sensor which provides a detectable alarm upon detection of an atmospheric pressure of the enclosed environmental chamber that differs by more than ±0.5% from a user-controlled atmospheric pressure. In some instances, the enclosed environmental chamber comprises a user display which displays an atmospheric pressure level of the enclosed environmental chamber. In some instances, the enclosed environmental chamber comprises a user display which displays an O₂ level of the enclosed environmental chamber. In some instances, the enclosed environmental chamber comprises a user display which displays a CO₂ level of the enclosed environmental chamber. In some instances, the enclosed environmental chamber comprises a user display which displays a temperature level of the enclosed environmental chamber.

In some instances, the enclosed environmental chamber occupies no more than 6 cubic feet of space. In some instances, the enclosed environmental chamber occupies no more than 3.5 cubic feet of space. In some instances, the enclosed environmental chamber occupies no more than 2 cubic feet of space. In some instances, the enclosed environmental chamber occupies no more than 1.5 cubic feet of space. In some instances, the enclosed environmental chamber occupies no more than 1 cubic foot of space. In some instances, the enclosed environmental chamber occupies less than 1 cubic foot of space.

In some instances, the cell culture incubator further comprises a cellular imaging system within the enclosed environmental chamber. In some instances, the cellular imaging system comprises a microscope. In some instances, the microscope is an inverted microscope. In some instances, the cellular imaging system comprises a micromanipulation apparatus, wherein the micromanipulation apparatus is configured to enable isolation of a target cell. In some instances, the cellular imaging system is operably linked to a computer system, wherein the computer system comprises a computer readable medium as described herein. In some instances, the cellular imaging system is configured for live cell imaging. In some instances, the cellular imaging system is configured for high content screening analysis.

Described herein is a cell culture plate comprising a cell culture substrate, wherein the substrate is selected to promote adherence of a target cell to the substrate, wherein the target cell is an adherative and viable cell and the substrate comprises an extracellular matrix protein. In some instances, the cell culture plate is configured to promote long-term culture of the target cell when the target cell is adhered to the substrate. In some instances, the target cell is a primary cell. In some instances, the primary cell is selected from the group consisting of a circulating tumor cell (CTC), a cancer stem cells (CSC), a cell from a solid tumor, a hematopoietic stem cells (HSC), a primary neuronal cell, a fetal cells, a stem cell, an adult stem cell, an immune cell, a primary hepatocyte, a primary cardiomyocyte, an endothelial progenitor cell (EPC), a cancer cell, and an epithelial progenitor cell. In some instances, the primary cell is selected from the group consisting of a circulating tumor cell (CTC), cancer stem cell (CSC), cell from a solid tumor, hematopoietic stem cell (HSC), primary neuronal cell, primary fetal cell, primary hepatocyte, primary cardiomyocyte, and endothelial progenitor cell (EPC). In some embodiments, the circulating tumor cell (CTC) is an acute leukemia cell, acute t-cell leukemia cell, acute lymphocytic leukemia cell, acute myelocytic leukemia cell, myeloblastic leukemia cell, promyelocytic leukemia cell, myelomonocytic leukemia cell, monocytic leukemia cell, erythroleukemia cell, chronic leukemia cell, chronic myelocytic (granulocytic) leukemia cell, chronic lymphocytic leukemia cell, polycythemia vera cell, lymphoma cell, multiple myeloma cell, Waldenstrom's macroglobulinemia cell, heavy chain disease cell, sarcoma cell, fibrosarcoma cell, myxosarcoma cell, liposarcoma cell, chondrosarcoma cell, osteogenic sarcoma cell, lymphangiosarcoma cell, mesothelioma cell, Ewing's tumor cell, leiomyosarcoma cell, rhabdomyosarcoma cell, colon carcinoma cell, colorectal cancer cell, pancreatic cancer cell, breast cancer cell, ovarian cancer cell, prostate cancer cell, castration resistant prostate cancer cell, squamous cell carcinoma cell, basal cell carcinoma cell, adenocarcinoma cell, sweat gland carcinoma cell, sebaceous gland carcinoma cell, papillary carcinoma cell, papillary adenocarcinomas cell, cystadenocarcinoma cell, medullary carcinoma cell, bronchogenic cell, renal cell carcinoma cell, hepatoma cell, bile duct carcinoma cell, choriocarcinoma cell, seminoma cell, embryonal carcinoma cell, Wilms' tumor cell, cervical cancer cell, uterine cancer cell, testicular tumor cell, lung carcinoma cell, small cell lung carcinoma cell, bladder carcinoma cell, epithelial carcinoma cell, glioma cell, craniopharyngioma cell, ependymoma cell, pinealoma cell, hemangioblastoma cell, acoustic neuroma cell, oligodendroglioma cell, meningioma cell, melanoma cell, neuroblastoma cell, retinoblastoma cell, endometrial cancer cell, non small cell lung cancer cell, head and neck cancer cell, or kidney cancer cell. In some instances, the non-target primary cell subpopulation comprises white blood cells, senescent epithelial cells, fibroblasts, non-viable cells, or fragmented cells. In some instances, the substrate further comprises a positively charged molecule. In some instances, the positively charged molecule is poly-ornithine.

Described herein is a cell culture plate comprising a first cell culture substrate and at least a second cell culture substrate, wherein the first cell culture substrate is configured to promote long-term culture of a first target cell subpopulation and wherein the at least second cell culture substrate is configured to promote long-term culture of a second target cell subpopulation. In some instances, the cell culture plate comprises at least two wells, wherein a first of the two wells comprises the first cell culture substrate and wherein a second of the at least two wells comprises the at least second cell culture substrate. In some instances, the first target cell subpopulation and/or the at least second cell culture subpopulation is selected from the group consisting of circulating tumor cells (CTCs), cancer stem cells (CSCs), cells from a solid tumor, hematopoietic stem cells (HSCs), primary neuronal cells, fetal cells, stem cells, adult stem cells, immune cells, primary hepatocytes, primary cardiomyocytes, endothelial progenitor cells (EPCs), cancer cells, and epithelial progenitor cells. In some instances, the first target cell subpopulation and/or the at least second cell culture subpopulation is selected from the group consisting of circulating tumor cells (CTCs), cancer stem cells (CSCs), cells from a solid tumor, hematopoietic stem cells (HSCs), primary neuronal cells, primary fetal cells, primary hepatocytes, primary cardiomyocytes, and endothelial progenitor cells (EPCs). In some instances, the circulating tumor cell (CTC) is an acute leukemia cell, acute t-cell leukemia cell, acute lymphocytic leukemia cell, acute myelocytic leukemia cell, myeloblastic leukemia cell, promyelocytic leukemia cell, myelomonocytic leukemia cell, monocytic leukemia cell, erythroleukemia cell, chronic leukemia cell, chronic myelocytic (granulocytic) leukemia cell, chronic lymphocytic leukemia cell, polycythemia vera cell, lymphoma cell, multiple myeloma cell, Waldenstrom's macroglobulinemia cell, heavy chain disease cell, sarcoma cell, fibrosarcoma cell, myxosarcoma cell, liposarcoma cell, chondrosarcoma cell, osteogenic sarcoma cell, lymphangiosarcoma cell, mesothelioma cell, Ewing's tumor cell, leiomyosarcoma cell, rhabdomyosarcoma cell, colon carcinoma cell, colorectal cancer cell, pancreatic cancer cell, breast cancer cell, ovarian cancer cell, prostate cancer cell, castration resistant prostate cancer cell, squamous cell carcinoma cell, basal cell carcinoma cell, adenocarcinoma cell, sweat gland carcinoma cell, sebaceous gland carcinoma cell, papillary carcinoma cell, papillary adenocarcinomas cell, cystadenocarcinoma cell, medullary carcinoma cell, bronchogenic cell, renal cell carcinoma cell, hepatoma cell, bile duct carcinoma cell, choriocarcinoma cell, seminoma cell, embryonal carcinoma cell, Wilms' tumor cell, cervical cancer cell, uterine cancer cell, testicular tumor cell, lung carcinoma cell, small cell lung carcinoma cell, bladder carcinoma cell, epithelial carcinoma cell, glioma cell, craniopharyngioma cell, ependymoma cell, pinealoma cell, hemangioblastoma cell, acoustic neuroma cell, oligodendroglioma cell, meningioma cell, melanoma cell, neuroblastoma cell, retinoblastoma cell, endometrial cancer cell, non small cell lung cancer cell, head and neck cancer cell, or kidney cancer cell. In some instances, the non-target primary cell subpopulation comprises white blood cells, senescent epithelial cells, fibroblasts, non-viable cells, or fragmented cells. In some instances, the substrate comprises GOL substrate, GEL substrate, one or more layers of feeder cells, or any combination thereof. In some instances, the cell culture plate comprises 1, 6, 12, 24, 48, 96, 384, 1056, 1536, or 3456 wells. In some instances, the first target cell subpopulation comprises a metastatic castration resistant prostate cancer and the first substrate comprises an androgen and/or androgen mimic.

Described herein is a kit. In some instances, the kit comprises a cell culture incubator as described herein; and instructions for use in culturing a target cell subpopulation. In some instances, the instructions comprise a directive to maintain an atmospheric pressure of 15.7 PSIA or more within an enclosed environmental chamber of the incubator. In some instances, the instructions comprise a directive to maintain an atmospheric pressure of 16.7 PSIA or more within an enclosed environmental chamber of the incubator. In some instances, the instructions comprise a directive to maintain an atmospheric pressure of 19.7 PSIA or more within an enclosed environmental chamber of the incubator. In some instances, the instructions comprise a directive to maintain an O₂ level of no more than 10% within an enclosed environmental chamber of the incubator. In some instances, the instructions comprise the instructions comprise a directive to maintain an O₂ level of no more than 5% within an enclosed environmental chamber of the incubator. In some instances, the instructions comprise the instructions comprise a directive to maintain an O₂ level of no more than 2% within an enclosed environmental chamber of the incubator. In some instances, the instructions comprise the instructions comprise a directive to maintain an O₂ level of no more than 1% within an enclosed environmental chamber of the incubator.

Described herein is a kit, comprising: a cell culture plate comprising a substrate as described in any of the preceding claims; and instructions for use of the plate for culturing a target cell subpopulation. In some instances, the instructions comprise a directive to culture the target cell subpopulation at an O₂ level of no more than 10%. In some instances, the instructions comprise a directive to culture the target cell subpopulation at an O₂ level of no more than 5%. In some instances, the instructions comprise a directive culture the target cell subpopulation at an O₂ level of no more than 2%. In some instances, the instructions comprise a directive culture the target cell subpopulation at an O₂ level of no more than 1%. In some instances, the instructions comprise a directive to culture the target cell subpopulation at an atmospheric pressure of 15.7 PSIA or more. In some instances, the instructions comprise the instructions comprise a directive to culture the target cell subpopulation at an atmospheric pressure of 16.7 PSIA or more. In some instances, the instructions comprise a directive culture the target cell subpopulation at an atmospheric pressure of 19.7 PSIA or more.

Described herein is a kit, comprising: a compound that regulates surface tension of a cell in a suitable packaging; and instructions for preparing a cell culture medium comprising the compound for use in culturing a target cell subpopulation. In some instances, the compound is a Rho kinase inhibitor. In some instances, the compound is selected from the group consisting of

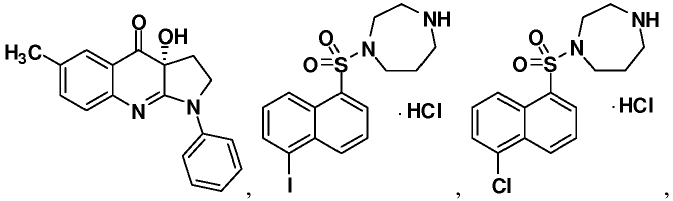

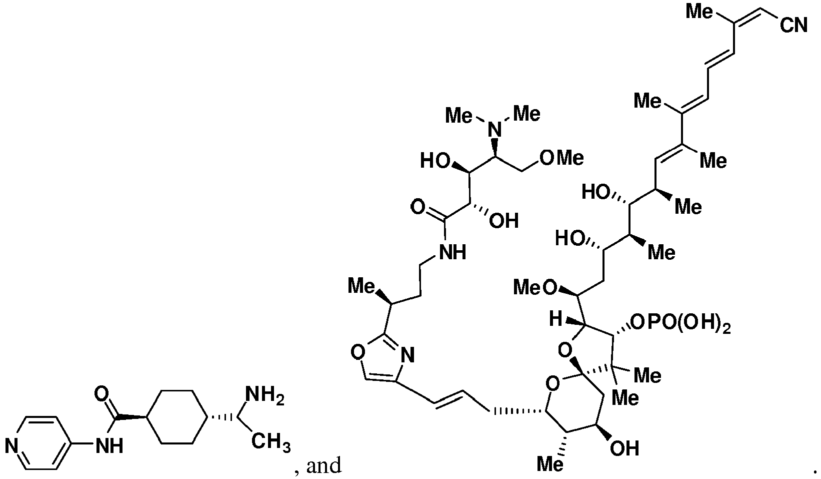

Described herein is a kit, comprising: a cell culture medium as described in any of the preceding claims; and instructions for use of the cell culture for culturing a target cell subpopulation. In some instances, the instructions comprise a directive to culture the target cell subpopulation at an O₂ level of no more than 10%. In some instances, the instructions comprise a directive to culture the target cell subpopulation at an O₂ level of no more than 5%. In some instances, the instructions comprise a directive culture the target cell subpopulation at an O₂ level of no more than 2%. In some instances, the instructions comprise a directive culture the target cell subpopulation at an O₂ level of no more than 1%. In some instances, the instructions comprise a directive to culture the target cell subpopulation at an atmospheric pressure of 15.7 PSIA or more. In some instances, the instructions comprise a directive to culture the target cell subpopulation at an atmospheric pressure of 16.7 PSIA or more. In some instances, the instructions comprise a directive culture the target cell subpopulation at an atmospheric pressure of 19.7 PSIA or more.

Described herein is a kit, comprising an agent that increases expression of a hypoxia-inducible factor in the member; and instructions for use of the agent for culturing a target cell subpopulation. In some instances, the agent that increases expression of a hypoxia-inducible factor comprises a vector, wherein the vector comprises a polynucleotide encoding the hypoxia-inducible factor. In some instances, the vector is an expression vector. In some instances, the polynucleotide comprises at least 70% greater homology or complementarity to SEQ. ID. NO. 2.

In some instances, the hypoxia-inducible factor comprises an amino acid sequence comprising at least 70% or greater homology to SEQ. ID. NO. 1.

Described herein is a computer readable medium comprising code that, upon execution by one or more processors, implements an identifying step as described by a method as described herein. For example, described herein is a computer readable medium comprising code that, upon receiving image data of one or more primary cells, implements the following steps: determining from the image data a size of the one or more primary cells; and classifying a primary cell as originating from a tumor if the size is at least 1.5X larger than a cell that does not originate from a tumor, wherein the one or more primary cells is obtained from a biological sample, and wherein the one or more primary cells is cultured under conditions sufficient to allow growth of the target cell subpopulation, wherein the conditions comprise incubating the one or more primary cells under positive pressure conditions and/or hypoxic conditions. In some instances, the computer readable medium implements a step of identifying the biological sample as comprising a tumor cell upon the classifying. In some instances, the computer readable medium implements a step of classifying a primary cell as originating from a tumor if the size is at least 2X larger than a cell that does not originate from a tumor. In some instances, the size comprises a diameter that is at least 20 µm. In some embodiments, the size comprises a surface area that is at least 900 µm2.

Described herein is a computer readable medium comprising code that, upon receiving image data of one or more primary cells obtained from a biological sample, implements the following steps: detecting from the image data a presence or absence of a three-dimensional colony adhered to a solid or semi-solid substrate; and classifying the biological sample as comprising a tumor cell upon detecting a presence of the three-dimensional colony, wherein the one or more primary cells is cultured under conditions sufficient to allow growth of the target cell subpopulation, wherein the conditions comprise incubating the one or more primary cells under positive pressure conditions and/or hypoxic conditions.

Described herein is a computer readable medium comprising code that, upon receiving image data of one or more primary cells obtained from a biological sample, implements the following steps: detecting from the image data a presence or absence of a primary cell comprising a plurality of nuclei; and classifying the biological sample as comprising a tumor cell upon detecting a presence of the plurality of nuclei, wherein the one or more primary cells is cultured under conditions sufficient to allow growth of the target cell subpopulation, wherein the conditions comprise incubating the one or more primary cells under positive pressure conditions and/or hypoxic conditions. In some instances, the computer readable medium is configured to identify a primary cell comprising a plurality of nuclei as a tumor cell.

Described herein is a computer readable medium comprising code that, upon receiving image data of one or more primary cells obtained from a biological sample, implements the following steps: detecting from the image data a presence or absence of a primary cell comprising a negative net surface charge; and classifying the biological sample as comprising a tumor cell upon detecting a presence of the primary cell comprising a negative net surface charge, wherein the net negative surface charge is evidenced by increased adherence of the primary cell to a positively charged solid or semi-solid substrate as compared to adherence of a non-tumor cell to the positively charged solid or semi-solid substrate. In some instances, the positively charged solid or semi-solid substrate comprises an extracellular matrix protein. In some instances, the extracellular matrix protein is poly-1-ornithine.

Described herein is a computer readable medium comprising code that, upon receiving image data of one or more primary cells obtained from a biological sample, implements the following steps: detecting from the image data a presence or absence of a rapidly dividing cell; and classifying the biological sample as comprising a tumor cell upon detecting a presence of the rapidly dividing cell, wherein the one or more primary cells is cultured under conditions sufficient to allow growth of the target cell subpopulation, wherein the conditions comprise incubating the one or more primary cells under positive pressure conditions and/or hypoxic conditions. In some instances, the computer readable medium is configured to identify the rapidly dividing cell as a tumor cell. In some instances, the image data comprises image data obtained at a first and second time point. In some instances, the first and second time point are three days apart or less, and wherein the presence of a rapidly dividing cell is evidenced by a 2X or more increase in cell confluency between the first and second time point. In some instances, the first and second time points are two days apart or less. In some instances, the presence of a rapidly dividing cell is evidenced by a 30X or more increase in cell confluency between the first and second time point. In some instances, the computer readable medium is configured to classify the tumor cell as a tumor-initiating cell.

In any of the foregoing instances of the computer readable medium, the biological sample is a fluid sample. In some instances, the fluid sample is a blood sample. In some instances, the blood sample is a whole blood sample. In some instances, the biological sample is a solid tissue sample. In some instances, the positive pressure conditions and/or hypoxic conditions are as described by any of the preceding claims.

Described herein is a computer system comprising: a storage memory for storing a dataset associated with a sample; a processor communicatively coupled to the storage memory, wherein the processor comprises the computer readable medium as described herein; and the dataset. In some instances, the computer system further comprises a computer configured to receive the dataset; and a network configured to transfer the data set from the system to the computer.

Described herein is a cellular imaging system, comprising: a microscope configured to produce image data from a cell; and a computer system configured to receive image data from the microscope, wherein the microscope comprises a computer readable medium as described herein. In some instances, the microscope comprises a stage, wherein the stage is configured to hold a cell culture plate as described herein. In some instances, the cellular imaging system is configured for live cell imaging. In some instances, the cellular imaging system is configured for high content screening analysis. In some instances, the cellular imaging system comprises an environmental chamber, wherein the environmental chamber is configured maintain at least one of (i) a gas composition of the environmental chamber, (ii) an atmospheric pressure of the enclosed environmental chamber, and (iii) an internal ambient temperature of the enclosed environmental chamber. In some instances, the environmental chamber is configured to maintain at least two of the gas composition, the atmospheric pressure, and the internal ambient temperature. In some instances, the gas composition comprises an O₂ level of between 1-20%. In some instances, the gas composition comprises an O₂ level of no more than 5%. In some instances, the gas composition comprises an O₂ level of no more than 2%. In some instances, the gas composition comprises an O₂ level of no more than 1%. In some instances, the atmospheric pressure of the environmental chamber is 15.7 PSIA or greater. In some instances, the atmospheric pressure of the environmental chamber is 16.7 PSIA or greater. In some instances, the atmospheric pressure of the environmental chamber is of 19.7 PSIA.

Desecribed herein is a system for culturing a target cell population, comprising: a cell culture incubator as described herein; and at least one of (i) a cell culture plate, (ii) a cell culture medium, and (iii) a cellular imaging system. In some instances, the system comprises at least two of the cell culture plate, the cell culture medium, and the cellular imaging system. In some instances, the cell culture plate is a cell culture plate as described herein. In some instances, the cell culture medium comprises Plating Culture Medium; Type R Long Term Growth Medium; Type DF Long Term Growth Medium; Type D Long Term Growth Medium; MEF - Conditioned Medium; and any combination thereof. In some instances, the cellular imaging system is as described herein.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1** depicts an exemplary method of the invention.
**FIGS. 2A** **and** **2B** depict a workflow of an exemplary method of the invention.
**FIG. 3** illustrates an overview of density-based centrifugation of a blood sample using Ficoll reagent to separate the mononuclear cellular layer.
**FIG. 4** illustrates a process by which target cell subpopulations can be plated and can adhere to the substrate.
**FIG. 5** illustrates different substrate configurations.
**FIG. 6** depicts an exemplary culture of a target cell subpopulation in a substrate having an "inverted" layer configuration.
**FIG. 7** depicts an exemplary incubator of the invention.
**FIG. 8** illustrates proliferation of subsets of a target cell subpopulation over time.
**FIG. 9** depicts a virtual grid coordinate system created by analysis software.
**FIG. 10** illustrates how target cell subpopulations can be labeled to enhance detection using a mitochondrial marker or a fluorescently labeled antibody directed against a cell-adhesion molecule.
**FIG. 11** depicts a system for software facilitation.
**FIG. 12** depicts a motor-driven micromanipulator selecting and injecting single cells.
**FIG. 13** depicts an automated cell culture system design.
**FIG. 14** depicts an exemplary cell culture system of the invention.
**FIG. 15** depicts live-cell imaging of cells from blood spiked with cancer cells; the cells have been plated onto a substrate.
**FIG. 16** depicts images of an enriched target cell subpopulation.
**FIG. 17** depicts software-aided analysis of live-cell images.
**FIG. 18** depicts an exemplary cell culture incubator of the invention.
**FIG. 19** depicts photomicrographs of CTCs isolated from a human subject with prostate cancer.
**FIG. 20** depicts results from a chemosensitivity assay.

### DETAILED DESCRIPTION OF THE INVENTION

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

### General Techniques

The practice of the present invention will employ, unless otherwise indicated, conventional techniques of immunology, biochemistry, chemistry, molecular biology, microbiology, cell biology, genomics and recombinant DNA, which are within the skill of the art. See, e.g., Sambrook, Fritsch and Maniatis, MOLECULAR CLONING: A LABORATORY MANUAL, 4th edition (2012); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M. J. MacPherson, B. D. Hames and G. R. Taylor eds. (1995)), and CULTURE OF ANIMAL CELLS: A MANUAL OF BASIC TECHNIQUE AND SPECIALIZED APPLICATIONS, 6th Edition (R. I. Freshney, ed. (2010), which are hereby incorporated by reference.

### Definitions

As used in the specification and claims, the singular forms "a", "an" and "the" include plural references unless the context clearly dictates otherwise. For example, the term "a cell" includes a plurality of cells, including mixtures thereof.

The term "anti-cancer agent" generally can refer to any agent put in contact with one or more cancer cells that directly or indirectly prohibits, stops, and/or reduces the proliferation of cancer cells and/or kills cancer cells. One or more anti-cancer agents can be put in contact with one or more cancer cells for the purpose of determining what anti-cancer agent(s) have the most desirable effect(s) on the cancer cells.

The anti-cancer agents may be put in contact with one or more cancer cells for the purpose of determining the anti-cancer properties. Even though the phrase "anti-cancer agent" may be written as a singular noun, for example, "an anti-cancer agent" or "the anti-cancer agent," the phrase "anti-cancer agent" can be interpreted as referring to one or more anti-cancer agents.

Anti-cancer agents can include agents that may be hypothesized to have an anti-cancer effect. That is to say, anti-cancer agents can include agents where the anti-cancer properties are not known or not well characterized. The anti-cancer agents may be put in contact with one or more cancer cells for the purpose of determining if the agent has any anti-cancer properties.

Anti-cancer agents can be agents that are known in the art to affect cancer cells. Some non-limiting examples of anti-cancer agents can include: hormone ablation agents, anti- androgen agents/anti-androgens, differentiating agents, anti-neoplastic agents, kinase inhibitors, anti-metabolite agents, alkylating agents, antibiotic agents, immunological agents, interferon-type agents, intercalating agents, growth factor inhibitors, cell-cycle inhibitors, enzymes, topoisomerase inhibitors, biological response modifiers, mitotic inhibitors, matrix metalloprotease inhibitors, anti-resorptives, monoclonal antibodies, adhesion molecules, growth factors, pro-apoptotic agents, antisense agents, vitamin D analogs, RNAi agents, modified peptides, enzyme inhibitors, agents ameliorating the side effects of therapy, genetic therapeutics and any combination thereof.

An anti-cancer agent can be a hormonal ablation agent (e.g., deslorelin, leuprolide, goserelin and/or triptorelin). A hormonal ablation agent can be an anti-androgen agent (e.g., bicalutamide, flutamide, spironolactone, cyproterone acetate, finasteride, dutasteride, abiraterone, and/or nilutamide). A hormonal ablation agent can be an anti-estrogen agent (e.g., tamoxifen).

An anti-cancer agent can be a differentiating agent (e.g., polyamine inhibitors; vitamin D and its analogs, such as calcitriol, doxercalciferol, ergocalciferol, 22-oxacalcitriol, dihydrotachysterol, paricalcitol, and seocalcitol; inhibitors of vitamin A metabolism, such as RAMBAS, e.g. liarozole, metabolites of vitamin A, such as ATRA; retinoic acid; retinoids; short- chain fatty acids; phenylbutyrate; and nonsteroidal anti-inflammatory agents).

An anti-cancer agent can be an anti-neoplastic agent. Non-limiting examples of anti-neoplastic agents include tubulin interacting agents, topoisomerase inhibitors and agents, acitretin, alstonine, amonafide, amphethinile, amsacrine, ankinomycin, anti-neoplaston, aphidicolin glycinate, asparaginase, baccharin, batracylin, benfluron, benzotript, bromofosfamide, caracemide, carmethizole hydrochloride, chlorsulfaquinoxalone, clanfenur, claviridenone, crisnatol, curaderm, cytarabine, cytocytin, dacarbazine, datelliptinium, dihaematoporphyrin ether, dihydrolenperone, dinaline, distamycin, docetaxel, elliprabin, elliptinium acetate, epothilones, ergotamine, etoposide, etretinate, fenretinide, gallium nitrate, genkwadaphnin, hexadecylphosphocholine, HDAC inhibitors, homoharringtonine, hydroxyurea, ilmofosine, isoglutamine, isotretinoin, leukoregulin, lonidamine, merbarone, merocyanlne derivatives, methylanilinoacridine, minactivin, mitonafide, mitoquidone, mitoxantrone, mopidamol, motretinide, N-(retinoyl)amino acids, N-acylated-dehydroalanines, nafazatrom, nocodazole derivative, octreotide, oquizanocine, paclitaxel, pancratistatin, pazelliptine, piroxantrone, polyhaematoporphyrin, polypreic acid, probimane, procarbazine, proglumide, razoxane, retelliptine, spatol, spirocyclopropane derivatives, spirogermanium, strypoldinone, superoxide dismutase, teniposide, thaliblastine, tocotrienol, topotecan, ukrain, vinblastine sulfate, vincristine, vindesine, vinestramide, vinorelbine, vintriptol, vinzolidine, and withanolides.

An anti-cancer agent can be a kinase inhibitor. Non-limiting examples of kinase inhibitors include p38 inhibitors; CDK inhibitors; TNF inhibitors; matrixmetallo proteinase (MMP) inhibitors; COX-2 inhibitors, including celecoxib, rofecoxib, parecoxib, valdecoxib, and etoricoxib; SOD mimics; and αvβ3-inhibitors.

An anti-cancer agent can be an anti-metabolite agent. Non- limiting examples of anti-metabolite agents include 5 -FU- fibrinogen, acanthifolic acid, aminothiadiazole, brequinar sodium, carmofur, cyclopentyl cytosine, cytarabine phosphate stearate, cytarabine conjugates, dezaguanine, dideoxycytidine, dideoxyguanosine, didox, doxifluridine, fazarabine, floxuridine, fludarabine phosphate, 5-fluorouracil, N-(2'-furanidyl)-5- fluorouracil, inhibitors of essential amino acids, isopropyl pyrrolizine, methobenzaprim, methotrexate, norspermidine, ornithine decarboxylantion inhibitors, pentostatin, piritrexim, plicamycin, thioguanine, tiazofurin, trimetrexate, tyrosine kinase inhibitors, and uricytin.

An anti-cancer agent can be an alkylating agent. Non-limiting examples of alkylating agents include aldo-phosphamide analogues, altretamine, anaxirone, bestrabucil, budotitane, carboplatin, carmustine, chlorambucil, cisplatin, cyclophosphamide, cyplatate, diphenylspiromustine, diplatinum cytostatic, elmustine, estramustine phosphate sodium, fotemustine, hepsul-fam, ifosfamide, iproplatin, lomustine, mafosfamide, mitolactol, oxaliplatin, prednimustine, ranimustine, semustine, spiromustine, tauromustine, temozolomide, teroxirone, tetraplatin and trimelamol.

An anti-cancer agent can be an antibiotic agent. Non-limiting examples of antibiotic agents include aclarubicin, actinomycin D, actinoplanone, adriamycin, aeroplysinin derivative, amrubicin, anthracycline, azinomycin-A, bisucaberin, bleomycin sulfate, bryostatin-1, calichemycin, chromoximycin, dactinomycin, daunorubicin, ditrisarubicin B, doxorubicin, doxorubicin-fibrinogen, elsamicin-A, epirubicin, erbstatin, esorubicin, esperamicin-Al, esperamicin-Alb, fostriecin, glidobactin, gregatin-A, grincamycin, herbimycin, idarubicin, illudins, kazusamycin, kesarirhodins, menogaril, mitomycin, neoenactin, oxalysine, oxaunomycin, peplomycin, pilatin, pirarubicin, porothramycin, pyrindanycin A, rapamycin, rhizoxin, rodorubicin, sibanomicin, siwenimycin, sorangicin-A, sparsomycin, talisomycin, terpentecin, thrazine, tricrozarin A, and zorubicin.

An anti-cancer agent can be a steroid, corticosteroid, or glucocorticoid. Non-limiting examples steroids include eplerenone, hydrocortisone, prednisone, and dexamethasone.

An anti-cancer agent can be an enzyme inhibitor. An enzyme inhibitor can be an imidazole, and azole, and/or an antifungal agent (e.g., ketoconazole).

An anti-cancer agent can be a monoclonal antibody. A monoclonal antibody can have anti-cancer activity. Non- limiting examples of monoclonal antibodies can include: rituximab, trastuzumab, gemtuzumab, ozogamicin, alemtuzumab, ibritumomab, tiuxetan, tositumomab, cetuximab, bevacizumab, panitumumab, and ofatumumab.

An anti-cancer agent can be an antisense agent. Some non-limiting examples of antisense agents can include: Genasense™ (oblimersen), Affinitak (ISIS 3521), ISIS 112989 (OGX 011), ISIS 23722, AP 12009, GEM 231, GEM 240, IGF-1R/AS ODN, MG98, LErafAON, Ki-67 antisense oligonucleotide, GTI-2040, ISIS 2503, and API 1014.

An anti-cancer agent can be a peptide and/or a modified peptide. A peptide or modified peptide can be a ligand. The peptide and/or modified peptide can be an inhibitor. The peptide and/or modified peptide can a vaccine. The peptide and/or modified peptide can trigger an immune response. The immune response can be a T-cell response against cancer cells. The peptide or modified peptide can have anti-cancer activity.

An anti-cancer agent can be an RNAi therapy having anti-cancer activity.

An anti-cancer agent can be an agent ameliorating the side effects of a cancer therapy. The agent ameliorating the side effects of therapy can be: a diuretic, a pain reliever, an analgesic, an anti-inflammatory agent, eplerenone, prednisone, proton pump inhibitors, H₂ receptor antagonists, lipid lowering agents, antiresorptive agents, antipsychotic agents, and/or dexamethasone.

Other non-limiting examples of anti-cancer agents include acemannan, aclarubicin, aldesleukin, alemtuzumab, alitretinoin, altretamine, amifostine, amsacrine, anagrelide, anastrozole, ancestim, bexarotene, broxuridine, capecitabine, celmoleukin, cetrorelix, cladribine, clotrimazole, daclizumab, dexrazoxane, dilazep, docosanol, doxifluridine, bromocriptine, carmustine, cytarabine, diclofenac, edelfosine, edrecolomab, eflornithine, emitefur, exemestane, exisulind, fadrozole, erythropoietin, filgrastim, finasteride, fludarabine phosphate, formestane, fotemustine, gallium nitrate, gemcitabine, glycopine, heptaplatin, ibandronic acid, imiquimod, iobenguane, irinotecan, irsogladine, lanreotide, leflunomide, lenograstim, lentinan sulfate, letrozole, liarozole, lobaplatin, lonidamine, masoprocol, melarsoprol, metoclopramide, mifepristone, miltefosine, mirimostim, mitoguazone, mitolactol, molgramostim, nafarelin, nartograstim, nedaplatin, nilutamide, noscapine, oprelvekin, osaterone, oxaliplatin, pamidronic acid, pegaspargase, pentosan polysulfate sodium, pentostatin, picibanil, pirarubicin, porfimer sodium, raloxifene, raltitrexed, rasburicase, rituximab, romurtide, sargramostim, sizofiran, sobuzoxane, sonermin, suramin, tasonermin, tazarotene, tegafur, temoporfm, temozolomide, teniposide, tetrachlorodecaoxide, thalidomide, thrombopoietin, thymalfasin, thyrotropin alfa, topotecan, toremifene, trastuzumab, treosulfan, tretinoin, trilostane, trimetrexate, ubenimex, valrubicin, verteporfin, and vinorelbine.

The term "biological sample" generally refers to a sample or part isolated from a biological entity. The biological sample may show the nature of the whole and examples include, without limitation, bodily fluids, dissociated tumor specimens, cultured cells, and any combination thereof. Examples of biological samples can include but are not limited to, blood, serum, plasma, nasal swab or nasopharyngeal wash, saliva, urine, gastric fluid, spinal fluid, tears, stool, mucus, sweat, earwax, oil, glandular secretion, cerebral spinal fluid, tissue, semen, vaginal fluid, interstitial fluids derived from tumor tissue, ocular fluids, spinal fluid, throat swab, breath, hair, finger nails, skin, biopsy, tumor, placental fluid, amniotic fluid, cord blood, emphatic fluids, cavity fluids, sputum, pus, micropiota, meconium, breast milk and/or other excretions. The samples may include nasopharyngeal wash. Examples of tissue samples of the subject may include but are not limited to, connective tissue, muscle tissue, nervous tissue, epithelial tissue, cartilage, cancerous sample (e.g., tumor sample), or bone. The sample may be provided from a human or animal. The sample may be provided from a mammal, vertebrate, such as murines, simians, humans, farm animals, sport animals, or pets. The sample may be collected from a living or dead subject. The sample may be collected fresh from a subject or may have undergone some form of pre-processing, storage, and/or transport.

A "bodily fluid" can refer to fluids or secretions originating from a subject. Exemplary bodily fluids include, e.g., blood, serum, plasma, saliva, urine, gastric fluid, spinal fluid, tears, mucus, sweat, oil, glandular secretion, cerebral spinal fluid, semen, vaginal fluid, interstitial fluids derived from tumor tissue, ocular fluids, spinal fluid, placental fluid, amniotic fluid, cord blood, emphatic fluids, cavity fluids, meconium, breast milk, bone marrow, pleural fluid, lymphatic fluid, and/or other excretions. Bodily fluids can comprise a complex and heterogeneous cell population, and may contain cell types such as CTCs, CSCs, HSCs, and/or EPCs. In some instances, bodily fluids can be a mixture of more than one type of bodily fluid.

The term "cancer" generally refers to a disease that is characterized by the uncontrolled, abnormal growth of cells. In some instances, cancer can spread. As described herein, a "cancer" can encompass any neoplasm or tumor.

"Cancer stem cells (CSCs)" generally refer to a subset of cancer cells that can self-renew. CSCs generally comprise a small proportion of a tumor responsible for its sustained growth and can be responsible for metastasis by giving rise to new tumors. CSCs can express different cell markers than other cells from the same tumor and can contribute to treatment-resistance.

The term "cell subpopulation" generally refers to a grouping of cells of similar type that can be found in a larger, heterogeneous cell population. Non-limiting examples of cell subpopulations can include: pre-cancerous cells, stem cells, fetal stem cells, undifferentiated stem cells, fetal cells, bone marrow cells, progenitor cells, foam cells, mesenchymal cells, epithelial cells, epithelial progenitor cells, endothelial cells, endothelial progenitor cells (EPCs), endometrial cells, hematopoietic stem cells (HSCs), trophoblasts, cancer cells, circulating tumor cells (CTCs), cancer stem cells (CSCs), red blood cells, white blood cells, immune system cells, and connective tissue cells, or any combination thereof.

"Circulating tumor cells" (CTCs) generally refer to cancer cells originating from a primary tumor that can circulate in the blood vasculature and/or lymph system.

The terms "Culturing" and "incubating" cells are used interchangeably herein to generally refer to a process by which cells are grown under controlled conditions. The conditions may vary depending on the type of cells to be cultured.

The term "primary cell", as used herein, generally refers to a cell that is obtained from a living tissue (e.g., a biopsy) and established for growth in vitro (e.g., by culturing).

The term "cell morphology" or "morphological characteristic" can be used to refer to the observable physical characteristics of a cell, such as size, shape, or the presence or absence of certain features.

Endothelial progenitor cells (EPCs) generally refers to a population of cells with the ability to differentiate into endothelial cells.

The term enrichment generally refers to increasing the ratio of the number of cells of a target cell subpopulation present to the total number of cells present in a mixture of cells. Enrichment can occur, for example, by capturing target cell subpopulations or by proliferation (i.e. propagation) of target cell subpopulations or by a combination thereof.

Hematopoietic stem cells (HSCs) generally refer to multipotent stem cells that can give rise to different blood cell types.

Heterogeneous cell population generally refers to a mixture of two or more cell types. Examples of heterogeneous cell populations can include: bodily fluids, biological specimens, dissociated tumor specimens, cultured cells, and any combination thereof.

Human serum generally refers to a biological fluid originating from humans, whereby the majority of genetic material has been removed and the fluid largely is comprised of, for example, water, enzymes, metabolites, growth factors, signaling peptides, and other proteins native to the host. Some non-limiting examples of sources of human serum include: blood plasma collected form peripheral blood, interstitial fluid, intracellular fluid, and/or transcellular fluid

The kinetic properties of a cell generally refer to a cell's ability to move, invade, and/or divide over time.

Micromanipulation, as used herein, generally describes any method for handling single cells. Typical micromanipulation systems can include, for example: an inverted microscope plus a joystick operated, motorized micromanipulation platform; a micropipetter; or laser capture microdissection.

The terms "Enrichment medium" and "cell culture medium" are used interchangeably herein to generally refer to a solution in which to bathe and incubate cells that can be used to supply the cells with nutrients, such that enrichment of a target cell subpopulation can be achieved. Enrichment media can contain specific upstream ligand-based signaling cues that can potentiate cell division. The exact composition of the enrichment medium can vary based on the cell type to be captured and enriched.

Subject generally refers to an organism. A subject can be an animal. Exemplary animals include, but are not limited to humans, laboratory animals such as mice, rats, monkeys, and chimpanzees; domestic animals such as dogs and cats, agricultural animals such as cows, horses, pigs, sheep, goats; and wild animals such as bears, pandas, lions, tigers, leopards, elephants, zebras, giraffes, gorillas, dolphins, and whales.

"Substrate", as used herein, generally refers to the substance that can enhance enrichment, retrieval, capture, and/or proliferation of target cell subpopulations from a heterogeneous cell population. The exact composition of the substrate can be tailored for the enrichment of specific types of target subpopulations of cells. The substrate can be comprised of a single layer or many layers.

The term "vector" generally refers to a nucleic acid molecule, which may or may not be self-replicating, which transfers an inserted nucleic acid molecule into and/or between host cells. The term includes vectors that function primarily for insertion of DNA or RNA into a cell, replication of vectors that function primarily for the replication of DNA or RNA, and expression vectors that function for transcription and/or translation of the DNA or RNA. Also included are vectors that provide more than one of the above functions.

The term "expression vector" generally refers to a polynucleotide which, when introduced into an appropriate host cell, can be transcribed and translated into a polypeptide(s). An "expression system" usually connotes a suitable host cell comprised of an expression vector that can function to yield a desired expression product.

The term tumor generally refers to an abnormal mass of tissue resulting from abnormal cell growth and division. Tumors can be cancerous.

The terms, treat or treating, or palliating or ameliorating are generally used interchangeably and can mean administering a substance to achieve: a therapeutic benefit; to cure or reduce the severity of an existing disease, disorder, or condition; and/or to achieve a prophylactic benefit, prevent, or reduce the likelihood of onset or severity the occurrence of a disease, disorder or condition.

A therapeutic effect or therapeutic benefit, as used herein, generally refers to a physiologic effect, including but not limited to the cure, mitigation, amelioration, or prevention of disease in humans or other animals, or to otherwise enhance physical or mental wellbeing of humans or animals. Therapeutic benefit can mean treatment of the underlying disorder being treated or treatment of one or more of the physiological symptoms associated with the underlying disorder such that an improvement is observed in the subject, notwithstanding that the subject may still be afflicted with the underlying disorder.

The term "operably linked" generally refers to a juxtaposition wherein two or more components so described as being "operably linked" are in a relationship permitting them to function in their intended manner.

The terms "polynucleotides", "nucleic acid", and "oligonucleotides" are used interchangeably herein. They refer to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, ribozymes, cDNA, recombinant polynucleotides, branched polynucleotides, plasmids, vectors, DNA of any sequence, RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

### General Overview

Described herein are methods, apparatuses, compositions, and kits that can detect, isolate, capture, enrich, culture, and/or characterize one or more target cell subpopulations from a heterogeneous cell population, as well as applications thereof. Target cell subpopulations can comprise viable cells of any type. The viable cells can comprise adherative properties. Adherative properties can be evidenced by an ability of the cells to adhere to a solid or semi-solid surface, such as, e.g., an extracellular matrix, other cell, or to a substrate as described herein. Cells with adherative properties can express one or more adhesion molecules. Exemplary adhesion molecules can include, e.g., selectins, integrins, cadherins, syndecans, epithelial cell adhesion molecule (EpCAM), cd133, cd44, cd166, among others. Expression of an adhesion molecule can be determined by any means known in the art, including but not limited to gene expression studies, immunohistochemistry, immunoblotting, and the like. Target cell subpopulations can comprise primary cells of any type. The primary cells may be mammalian primary cells, such as, e.g., primary neurons, primary glia, primary cardiomyocytes, primary hepatocytes, primary lung cells, primary chondrocytes, primary endothelial cells, primary fibroblasts, primary gastrointestinal cells, primary immune cells, primary keratinocytes, primary osteoblasts, primary pancreatic cells, primary prostate cells, primary renal cells, primary retinal cells, primary muscle cells, primary sertoli cells, fetal cells, among others. Target cell subpopulations can have high proliferative and renewal properties. Some non-limiting examples of target cell subpopulations include, e.g., circulating tumor cells (CTCs), cancer stem cells (CSCs), hematopoietic stem cells (HSCs), endothelial progenitor cells (EPCs), fetal cells, stem cells, immune cells, epithelial cells, endothelial cells, cancer cells, white blood cells, adult stem cells, primary cells (e.g., primary cells described herein), cells from a solid tumor, and/or epithelial progenitor cells. In some embodiments, the target cell subpopulation is selected from: CTCs, CSCs, EPCs, stem cells, epithelial cells, and cancer cells. The target cell subpopulation can comprise CTCs and/or CSCs. The CTCs and/or CSCs may be tumor-initiating cells. The CTCs and/or CSCs may be metastatic cells. In some cases wherein the target cell subpopulation comprises cells from a solid tumor, the cells may still be in the form of the solid tumor (e.g., the methods, apparatuses, compositions, and kits described herein can be used for the purposes of culturing a solid tumor biopsy). In some embodiments, the target cell subpopulation does not comprise a cell from an immortalized cell line. In some embodiments, the target cell subpopulation does not comprise white blood cells. The target cell subpopulation may be a primary target cell subpopulation.

It will be recognized further that illustrations herein are primarily with reference to isolation and enrichment of cell subpopulations from blood, but the invention could readily be applied to isolation and enrichment of cell subpopulations from any heterogeneous cell populations. Such examples can include bodily fluids, mixtures of bodily fluids, disassociated tumor samples, solid tissue samples (e.g., solid tumor samples), cell cultures, or any combination thereof. Furthermore, the bodily fluids, disassociated tumor samples, and cell cultures may be from one or more subjects, or a combination thereof.

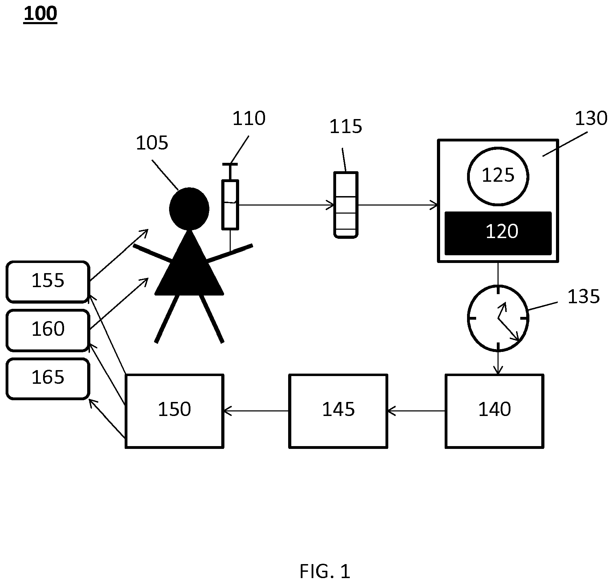

**FIG 1** shows an exemplary method according to the disclosure. **FIG 1** shows an exemplary process **100** for capturing and enriching target cell subpopulations, in accordance with an embodiment of the invention. The process **100** comprises obtaining a sample **110** from a subject **105;** separating one or more components of the sample **115** to obtain a heterogeneous cell population; contacting the heterogeneous cell population with a substrate **120** (e.g., plating the heterogeneous cell population onto the substrate; incubating the heterogeneous cell population under conditions sufficient to allow growth of a target cell subpopulation, wherein the conditions sufficient to allow growth of a target cell subpopulation can comprise incubating with enrichment media **125** in an apparatus **130;** incubating the samples for a time **135,** wherein the time **135** is sufficient for cell division to occur. The cells may be monitored using an imaging system **140** (e.g. a microscope). Data from monitoring with an imaging system can be processed using analysis software **145.** Analysis software **145** can create an output of results **150.** The cells and/or results **150** can be used for diagnosis, prognosis, or the monitoring of a disease condition **155,** to direct a treatment regimen for a subject **160,** and/or for research **165.**

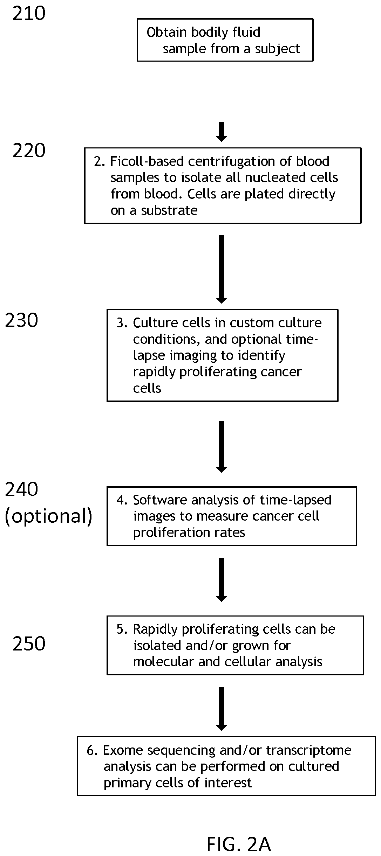

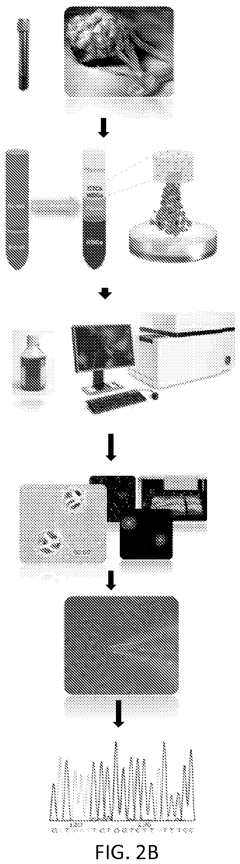

**FIGS. 2A** and **2B** depict a workflow of an exemplary method of the invention. The exemplary method can comprise step **210** of obtaining a biological sample from a subject, e.g., a human subject. In some embodiments, the biological sample is a bodily fluid sample (e.g., a blood sample). The exemplary method can comprise a subsequent step **220** of Ficoll-based centrifugation of blood samples to isolate all nucleated cells from blood. The nucleated cells may then be plated directly onto a substrate. The exemplary method can comprise a subsequent step **230** of culturing the cells in custom culture conditions. Step **230** may optionally comprise time-lapse imaging to identify rapidly proliferating cancer cells. The exemplary method can comprise a subsequent optional step **240** of using software (e.g., a computer readable medium) to analyze the time-lapse images. The analysis may comprise measuring cancer cell proliferation rates. The exemplary method can further comprise step **250** of isolating rapidly proliferating cells and/or growing the rapidly proliferating cells for further molecular/cellular analysis and/or imaging. The isolating can comprise use of a micromanipulation apparatus. In some embodiments, the further molecular and cellular analysis comprises exome sequencing and/or transcriptome analysis. The exemplary method may be useful for identification of, e.g., new biomarkers and/or therapeutic targets for the treatment of one or more cancers.

### Exemplary Subjects

The subject **105** (as depicted in **FIG. 1**) generally refers to a living organism. The subject can comprise one or more target cell subpopulations. It may be unknown if the subject comprises target cell subpopulations. In some embodiments, the methods of the disclosure may be used to determine if a subject comprises one or more target cell subpopulation(s). For example, subject may be screened for the presence or absence of one or more target cell subpopulation(s).

The subject can be a subject having cancer, a subject in remission from cancer, a subject undergoing treatment for cancer, a subject undergoing cancer screening, a subject who is suspected of having cancer, a subject that is being tested (e.g., screened) for the presence of cancer, a subject that is cancer-free, a subject who has an autoimmune disease, a subject who is suspected of having an autoimmune disease, a subject donating cells for a bone marrow transplant, a subject receiving bone marrow cells, a subject having a cardiovascular disease, a subject who has experienced a heart attack or stroke, a subject who is suspected of experiencing a heart attack or stroke in the future, a subject undergoing treatment for wound healing, a subject who may need treatment for wound healing, and any combination thereof. It is understood that the subject can have or can be suspected of having any disease.

The cancer can be, e.g., a tumor, a leukemia such as acute leukemia, acute t-cell leukemia, acute lymphocytic leukemia, acute myelocytic leukemia, myeloblastic leukemia, promyelocytic leukemia, myelomonocytic leukemia, monocytic leukemia, erythroleukemia, chronic leukemia, chronic myelocytic (granulocytic) leukemia, or chronic lymphocytic leukemia, polycythemia vera, lymphomas such as Hodgkin's lymphoma, follicular lymphoma or non-Hodgkin's lymphoma, multiple myeloma, Waldenstrom's macroglobulinemia, heavy chain disease, solid tumors, sarcomas, carcinomas such as, e.g., fibrosarcoma, myxosarcoma, liposarcoma, chondrosarcoma, osteogenic sarcoma, lymphangiosarcoma, mesothelioma, Ewing's tumor, leiomyosarcoma, rhabdomyosarcoma, colon carcinoma, colorectal cancer, pancreatic cancer, breast cancer, ovarian cancer, prostate cancer, including castration resistant prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic, carcinoma, renal cell carcinoma, hepatoma, bile duct carcinoma, choriocarcinoma, seminoma, embryonal carcinoma, Wilms' tumor, cervical cancer, uterine cancer, testicular tumor, lung carcinoma, small cell lung carcinoma, bladder carcinoma, epithelial carcinoma, glioma, craniopharyngioma, ependymoma, pinealoma, hemangioblastoma, acoustic neuroma, oligodendroglioma, meningioma, melanoma, neuroblastoma, retinoblastoma, endometrial cancer, non small cell lung cancer, head and neck cancer, or kidney cancer.

The autoimmune disease can be a disease or disorder arising from and directed against an individual's own tissues or a co-segregate or manifestation thereof or resulting condition therefrom. Examples of autoimmune diseases or disorders include, but are not limited to: Acute Disseminated Encephalomyelitis (ADEM), Arthritis, Acute necrotizing hemorrhagic leukoencephalitis, Addison's disease, Agammaglobulinemia, Alopecia areata, Amyloidosis, Ankylosing spondylitis, Anti-GBM/Anti-TBM nephritis, Antiphospholipid syndrome (APS), Autoimmune angioedema, Autoimmune aplastic anemia, Autoimmune dysautonomia, Autoimmune hepatitis, Autoimmune hyperlipidemia, Autoimmune immunodeficiency, Autoimmune inner ear disease (AIED), Autoimmune myocarditis, Autoimmune pancreatitis, Autoimmune retinopathy, Autoimmune thrombocytopenic purpura (ATP), Autoimmune thyroid disease, Autoimmune urticaria, Axonal & neuronal neuropathies, Balo disease, Behcet's disease, Bullous pemphigoid, Cardiomyopathy, Castleman disease, Celiac disease, Chagas disease, Chronic fatigue syndrome, Chronic inflammatory demyelinating polyneuropathy (CIDP), Chronic recurrent multifocal ostomyelitis (CRMO),-Strauss syndrome, Cicatricial pemphigoid/benign mucosal pemphigoid, Crohn's disease, Cogans syndrome, Cold agglutinin disease, Congenital heart block, Coxsackie myocarditis, CREST disease, mixed cryoglobulinemia, neuropathies, Dermatitis herpetiformis, Dermatomyositis, Devic's disease (neuromyelitis optica), Discoid lupus, Dressler's syndrome,Endometriosis, Eosinophilic esophagitis, Eosinophilic fasciitis, Erythema nodosum, Experimental allergic encephalomyelitis, Evans syndrome, Fibromyalgia, Fibrosing alveolitis, Giant cell arteritis (temporal arteritis), Glomerulonephritis, Goodpasture's syndrome, Granulomatosis with Polyangiitis (GPA), Graves' disease, Guillain-Barre syndrome, Hashimoto's encephalitis, Hashimoto's thyroiditis, Hemolytic anemia, Henoch-Schonlein purpura, Herpes gestationis, Hypogammaglobulinemia, Idiopathic thrombocytopenic purpura (ITP), IgA nephropathy, IgG4-related sclerosing disease, Immunoregulatory lipoproteins, Inclusion body myositis, Insulin-dependent diabetes (typel), Interstitial cystitis, Juvenile arthritis, Type I diabetes, Kawasaki syndrome, Lambert-Eaton syndrome, Leukocytoclastic vasculitis, Lichen planus, Lichen sclerosus, Ligneous conjunctivitis, Linear IgA disease (LAD), Lupus (SLE), Lyme disease, Meniere's disease, Microscopic polyangiitis, Mixed connective tissue disease (MCTD), Mooren's ulcer, Mucha-Habermann disease, Multiple sclerosis, Myasthenia gravis, Myositis, Narcolepsy, Neuromyelitis optica (Devic's), Neutropenia, Ocular cicatricial pemphigoid, Optic neuritis, Palindromic rheumatism, PANDAS (Pediatric Autoimmune Neuropsychiatric Disorders Associated with Streptococcus), Paraneoplastic cerebellar degeneration, Paroxysmal nocturnal hemoglobinuria (PNH), Parry Romberg syndrome, Parsonnage-Turner syndrome, Pars planitis (peripheral uveitis), Pemphigus, Peripheral neuropathy, Perivenous encephalomyelitis, Pernicious anemia, POEMS syndrome, Polyarteritis nodosa,Type I, II, or III autoimmune polyglandular syndromes, Polymyalgia rheumatic, Polymyositis, Postmyocardial infarction syndrome, Postpericardiotomy syndrome, Progesterone dermatitis, Primary biliary cirrhosis, Primary sclerosing cholangitis, Psoriasis, Psoriatic arthritis, Idiopathic pulmonary fibrosis, Pyoderma gangrenosum, Pure red cell aplasia, Raynauds phenomenon, Reflex sympathetic dystrophy, Reiter's syndrome, Relapsing polychondritis, Restless legs syndrome, Retroperitoneal fibrosis, Rheumatic fever, Rheumatoid arthritis, Sarcoidosis, Schmidt syndrome, Scleritis, Scleroderma, Sjogren's syndrome, Sperm & testicular autoimmunity, Stiff person syndrome, Subacute bacterial endocarditis (SBE), Susac's syndrome, Sympathetic ophthalmia, Takayasu's arteritis, Temporal arteritis/Giant cell arteritis, Thrombocytopenic purpura (TTP), Tolosa-Hunt syndrome, Transverse myelitis, Ulcerative colitis, Undifferentiated connective tissue disease (UCTD), Uveitis, Vasculitis, Vesiculobullous dermatosis, Vitiligo, and Wegener's granulomatosis.

Cardiovascular disease (CVD) can be manifest by many disorders involving the heart and vasculature throughout the body. Some non-limiting examples can include: aneurysms, angina, atherosclerosis, cerebrovascular accident (e.g., stroke), cerebrovascular disease, congestive heart failure, coronary artery disease, myocardial infarction, and peripheral vascular disease.

A subject may be a healthy subject with none of the diseases disclosed herein.

### Exemplary Biological Samples

A biological sample **110** (as depicted in FIG. 1) may be acquired from a subject. One or more samples may be acquired from a subject at one or more time points. A single sample can be taken from a subject once. Multiple samples can be taken from a subject over a period of time.

Exemplary biological samples are described herein. The biological sample 110 can be, e.g., any bodily fluid, biological specimen, a solid tissue sample, a cell culture, a heterogeneous population of cells, or any combination thereof. The source of the sample can be known. The source of the sample can be unknown. The identity of a subject from which a sample was taken can be associated with the identity of a sample. The association of the identity of a subject with a sample can be accessed by a caregiver.

The biological sample can comprise a bodily fluid sample. Exemplary bodily fluid samples are described herein. In some embodiments, the bodily fluid sample is blood. Blood (e.g., peripheral blood) can be obtained from a subject by any means known to those of skill in the art, e.g., by venous puncture, arterial puncture, and/or isolation from an umbilical cord. A bodily fluid sample volume may range, for example, approximately between 1 to 10 mL, approximately between 5 to 20 mL, or approximately between 15 to 35 mL in volume. The sample volume may be approximately 4, 5, 6, 7, 8, 9, 10, 12, 15, 17, 20, 25, 30 or 35 mL in volume but other volumes are contemplated. A larger sample of blood can be taken and a portion of the larger sample can be used.

The biological sample **110** may be mixed with one or more biological samples (e.g., cells) from the same subject or a different subject. The biological sample **110** can, e.g., be spiked with one or more cells. Spiking cells into a sample can provide a positive or negative control, and/or may improve enrichment of a target cell subpopulation. By way of example only, the biological sample **110** can be spiked with cells from an immortalized cell line. The immortalized cell line may originate from a cancer (e.g., a human cancer). Exemplary tumor or cancer cell lines are available through, e.g., ATCC, the National Cancer Institute, and other sources. Known immortalized cell lines can be used to spike a sample. Some non-limiting examples of known immortalized cell lines that can be used to spike a sample can include: HeLa, LnCAP, F47D, THP-1, U87, SHSY5Y, Saos02, Vero, DU145, MCF-7, MDA-MB-438, PC3, GH3, PC12, MC3T3, 293-T, HL-60, NCI-H322M, SW620, UACC-62, HCC2998, UACC-257, SN12C, CAKI-1, ACHN, LOXIMVI, EKVX, A549, 786-0, Hs-578-T, RPMI-8226, SR M14, A498, OVCAR-4, NCI-ADR-RES, MDA-MB-231, HOP-92, DU-145, CCRF-CEM, HT-29, IGROV-1, SF295, TK10, SK-MEL-2, SK-OV-3, MDA-MB-435, NCI-H522, T47D, SNB75, NCI-H226, SK-MEL-28, SK-MEL-5, HOP-62, RXF393, COLO-205, SF268, U031, HCT-15, BT-549, U251, OVCAR-5, SF539, Malme-3M, KM12, HCT-116, SNB19, OVCAR-3, NCI-H23, NCI-H460, MOLT-4, OVCAR-8, K-562.

The biological sample **110** may be spiked with one or more marked cells. The term "marked cell", as used herein, generally refers to a cell that comprises a detectable label. Marked cells can act as a control. For example, marked cells can be used to determine the effectiveness of cell capture, the effectiveness of cell proliferation, determine the rate of cell proliferation, or any combination thereof. A marked cell can be a transformed cell that expresses a gene or a protein. A marked cell can be a cell that expresses a cell surface antigen that can bind to a marked antibody.

Some non-limiting examples of detectable labels include, e.g.,: dyes, fluorescent markers, fluorescent proteins (e.g., green fluorescent protein (GFP), red fluorescent protein (RFP), yellow fluorescent protein (YFP), and etc.), bioluminescent proteins (e.g. luciferase), tagged proteins; radioactive molecules, biotin, horseradish peroxidase, fluorescently-conjugated molecules (e.g. dextran, β-galactosidase), and/or genetic alterations to a cell line or an animal (e.g. cre-lox recombinase system, FLP recombinase, and etc.).

In some embodiments, the sample is not spiked with additional cells.

### Optional Separation of Sample

The step of separating one or more components of the sample **115** (as depicted in **FIG. 1**) can increase a concentration of a target cell subpopulation by a factor of 1.5, 2, 4, 6, 8, 10, 20, 50, 100, 200, 500, 1,000, 2,000, 5,000, 10,000, 20,000, 50,000, 100,000, 200,000, 500,000, 1,000,000, 2,000,000, 5,000,000, 10,000,000, 20,000,000, 50,000,000, 100,000,000, 200,000,000, 500,000,000, 1,000,000,000, 2,000,000,000, or 5,000,000,000 fold over its concentration in the original sample. The step of separating one or more components of the sample **115** can also increase concentration of the cell subpopulations by reducing volume of the original sample (e.g., by removal of fluid). By way of example only, a bodily fluid sample (e.g., a blood sample) of greater than 10, 15, 20, 50, or 100 mL total volume comprising the subpopulation cells can be concentrated into a solution of less than 0.5, 1, 2, 3, 5, or 10 mL total volume, respectively.

Gentle handling of samples that comprise target subpopulation cells be used to reduce any mechanical damage to the cells. Gentle handling may serve to preserve the small number of subpopulation cells in the sample and/or the integrity of nucleic acids or macromolecules. Integrity of the target cell subpopulation can be important for later enrichment steps.

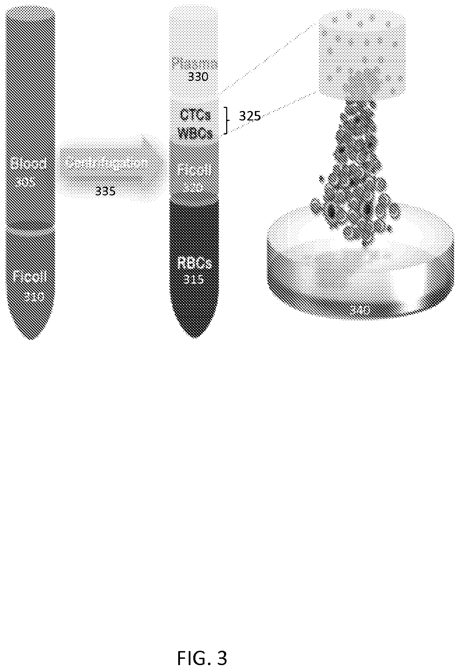

In some instances wherein the original sample is a bodily fluid (e.g., whole blood), the step of separating one or more components of the sample **115** can comprise use of a Ficoll reagent, described in detail Fuss et al, Curr Protoc Immunol (2009) Chapter 7:Unit7.1, which is incorporated herein by reference. **FIG. 3** shows an exemplary method **300** for separating blood components using a Ficoll reagent (e.g., Ficoll-Paque PLUS, GE Healthcare). The exemplary method **300** includes layering a whole blood sample **305** on top of Ficoll reagent **310.** The exemplary method further comprises subjecting the whole blood/Ficoll sample to density based centrifugation **335.** Centrifugation can result in a separation of the whole blood/Ficoll sample into distinct layers: (1) an anucleated cell layer **315,** comprising red blood cells; (2) a Ficoll reagent layer **320,** (3) a nucleated cell layer **325,** comprising, e.g., white blood cells (WBCs) and other nucleated cells (e.g. CTCs, CSCs, HSCs, EPCs, and the like) ; and (4) a plasma layer **330.** For target cell subpopulation capture, the nucleated cell layer can be isolated and exposed to a substrate **340.**

The step of separating one or more components of the sample **115** can also be achieved using one or more size-based separation techniques. Some non-limiting examples of size-based separation techniques can include use of a size based separation module, e.g., filtration modules, sieves, matrixes, lateral displacement from obstacles and the like. Size-based separation can remove red blood cells and platelets from blood (e.g. WO 2004/113877, hereby incorporated by reference). Particles can be separated using obstacles or a network of gaps (e.g. US 20040144651, hereby incorporated by reference). In some embodiments, separation and/or enrichment of the subpopulation cells does not comprise a size-based separation module.

A size-based separation module can comprise one or more arrays of obstacles forming a network of gaps. The obstacles can be configured to direct particles as they flow through the array/network of gaps into different directions or outlets based on the particle's hydrodynamic size. For example, a blood sample comprised of nucleated cells having a predetermined size (e.g. greater than 8 microns) can be directed towards a separate outlet than other blood components.

An array can be configured to separate cells smaller or larger than a predetermined size by adjusting the size of the gaps, obstacles, and offset in the period between each successive row of obstacles. Obstacles or gaps between obstacles can be up to 10, 20, 50, 70, 100, 120, 150, 170, or 200 microns in length or about 2, 4, 6, 8 or 10 microns in length. An array for size-based separation can include more than 100, 500, 1,000, 5,000, 10,000, 50,000 or 100,000 obstacles arranged into more than 10, 20, 50, 100, 200, 500, or 1000 rows. Obstacles in a first row of obstacles can be offset from a previous (i.e. upstream) row of obstacles by up to 50% the period of the previous row of obstacles. Obstacles in a first row of obstacles can be offset from a previous row of obstacles by up to 45, 40, 35, 30, 25, 20, 15 or 10% the period of the previous row of obstacles. The distance between a first row of obstacles and a second row of obstacles can be up to 10, 20, 50, 70, 100, 120, 150, 170 or 200 microns. A particular offset can be continuous (e.g. repeating for multiple rows) or non-continuous. A separation module can include multiple discrete arrays of obstacles fluidly coupled such that they are in series with one another. Each array of obstacles can have a continuous offset. But each subsequent (i.e. downstream) array of obstacles has an offset that can be different from the previous (i.e. upstream) offset. Each subsequent array of obstacles can have a smaller offset that the previous array of obstacles. This can allows for a refinement in the separation process as cells migrate through the array of obstacles. A plurality of arrays can be fluidly coupled in series or in parallel, (e.g., more than 2, 4, 6, 8, 10, 20, 30, 40, 50). Fluidly coupling separation modules (e.g., arrays) in parallel allows for high-throughput analysis of the sample, such that at least 1, 2, 5, 10, 20, 50, 100, 200, or 500 mL per hour can flow through the enrichment modules or at least 1, 5, 10, or 50 million cells per hour can be sorted or flow through the device.

Obstacles can be coupled to a flat substrate to form an array of gaps. The obstacles can form a two-dimensional array with each successive row shifted horizontally with respect to the previous row of obstacles, wherein the array of obstacles can direct a component having a hydrodynamic size smaller than a predetermined size in a first direction and another component having a hydrodynamic size larger that a predetermined size in a second direction. By way of example only, enriching epithelial or circulating tumor cells from enucleated cells, the predetermined size of an array of obstacles can be about 6-12 µm or 6-8 µm. The flow of sample into the array of obstacles can be aligned at a small angle (i.e. flow angle) with respect to a line-of-sight of the array. Optionally, the array can be coupled to an infusion pump to perfuse the sample through the obstacles. The flow conditions of the size-based separation module described herein can be such that subpopulation cells are sorted by the array with minimal damage. This allows for downstream analysis of intact cells and intact nuclei to be more efficient and reliable.

The step of separating one or more components of the sample **115** can comprise use of one or more capture techniques that selectively inhibit the mobility of one or more cells. A capture technique can be used with one or more other separation and/or enrichment techniques. A capture technique can involve affinity purification. The target cell subpopulation can be enriched based on positive selection, e.g. based on affinity of target cell subpopulations to a capture reagent.

The target cell subpopulations can be enriched based on affinity to a capture reagent, e.g., a capture antibody. For example, target cell subpopulations can be enriched based on affinity to a positive selection reagent. The positive selection reagent can be a positive selection antibody, e.g., an antibody to a peptide that is selectively expressed in the target cell subpopulation as compared to in non-target cell subpopulations. The peptide may or may not be a cell surface peptide. Exemplary positive selection antibodies include, but are not limited to antibodies to epithelial cell adhesion molecule (EPCAM), aldehyde dehydrogenase 1 (ALDHl), CK19, cytokeratin 8,18, Twist, EGFR, CD133, vimentin, among others. Accordingly, in some cases the target cell subpopulation (e.g., CTCs) can be enriched using an EPCAM antibody. A capture reagent (e.g., an antibody) can be conjugated to a solid or semi-solid support, e.g., a well, a bead, a magnetic bead, a chamber. Enrichment of target cell subpopulations by positive selection can comprise, by way of example only, contacting a biological sample with a solid or semi-solid phase immobilized thereon a positive selection antibody, such that a target cell binds to the positive selection antibody. The method can further comprise removing the unbound fraction of the sample (e.g., by one or more wash steps). The method can further comprise collecting the bound fraction. Enrichment of target cell subpopulations may not require use of a solid or semi-solid phase immobilized thereon a positive selection reagent. For example, enrichment via affinity to a positive selection reagent (e.g., a positive selection antibody) can comprise use of flow cytometry, e.g., FACS sorting. In some embodiments, target cell subpopulations are labeled with, e.g., a detectably labeled EpCAM antibody, and are enriched by FACS-based capture of EpCAM+ cells.

The target cell subpopulations can be enriched based on negative selection, e.g., based on affinity of non-target cell subpopulations to a capture reagent, e.g., a capture antibody. The target cell subpopulations can have lower affinity to the capture antibody as compared to the non-target cell subpopulations. For example, a capture antibody can be an antibody to a peptide that is typically expressed in non-target cell subpopulations but is typically not expressed in target cell subpopulations. For example, a capture antibody can be an antibody to a peptide that is selectively expressed in white blood cells, monocytes, and/or macrophages. An exemplary capture antibody that can be used for negative selection can include an anti-CD45 antibody, an anti-CD 14 antibody, an anti-CD34 antibody, an anti-fibroblast surface protein antibody, among others. Enrichment of target cell subpopulations by negative selection can comprise, by way of example only, contacting a biological sample with a solid or semi-solid phase immobilized thereon a negative selection antibody, such that a non-target cell binds to the negative selection antibody. The method can further comprise collecting the unbound fraction of the biological sample. Enrichment of target cell subpopulations may not require use of a solid or semi-solid phase immobilized thereon a negative selection reagent. For example, enrichment via affinity to a negative selection reagent (e.g., a negative selection antibody) can comprise use of flow cytometry, e.g., FACS sorting. In some embodiments, target cell subpopulations are labeled with, e.g., a detectably labeled negative selection antibody (e.g., CD45 antibody), and are enriched by FACS-based capture of CD45- cells.

Capture techniques may comprise use of one or more capture modules. A capture module can be a device configured for capture of one or more analyte (e.g., cells of interest or not of interest). In one non-limiting example, an affinity-based separation module can capture cells or can include an array of obstacles adapted for permitting sample flow through, wherein the obstacles can comprise surface binding moieties that can selectively bind one or more cell populations of interest (e.g., target cell subpopulation cells, epithelial cells, nucleated cells, CTCs, CSCs and the like) or analytes not-of-interest (e.g., non-target cells such as, by way of example only, white blood cells). Binding moieties can be coupled to the obstacles. Binding moieties can include e.g., proteins (e.g., ligands/receptors), nucleic acids having hybridizable counterparts in retained cells, antibodies, etc.

A capture technique can utilize a magnetic field to separate and/or enrich the cells based on a magnetic property or magnetic potential in such cells or in cells not of interest. For example, red blood cells which can be slightly diamagnetic (i.e. repelled by magnetic field) can be made paramagnetic (i.e. attracted by magnetic field) by deoxygenation of the hemoglobin into methemoglobin. This magnetic property can be achieved through physical or chemical treatment of the red blood cells. Thus, for a blood sample comprising cells of interest, the cells of interest can be separated from red blood cells by first inducing a magnetic property in the red blood cells and then separating the red blood cells from the cells of interest by flowing the sample through a magnetic field. A magnetic field can be uniform or non-uniform.

### In Vitro Systems for Selective Adherence and Growth of Target Cell Subpopulations

The in vitro systems described herein can promote the selective adherence and growth of target cell subpopulations. The target cell subpopulations can remain viable and undergo cell propagation. The in vitro systems can be configured to promote selective adherence and growth of target cell subpopulations by, for example, providing conditions that selectively promote adherence and growth of the target cell subpopulations. The conditions may be physiologically relevant conditions (e.g. environmental conditions that resemble in vivo environmental conditions).

### Exemplary Substrates

A substrate **120** (as depicted in **FIG. 1**) can capture target cell subpopulations from a sample comprising a heterogeneous population of cells.
A substrate can promote selective adherence and growth of the target cell subpopulations (e.g., adherative and/or viable cells) as compared to non-target cell subpopulations (e.g., cells that are not adherative and/or are not viable). For example, a substrate can promote the selective adherence and growth of circulating tumor cells (CTCs), cancer stem cells (CSCs), hematopoietic stem cells (HSCs), endothelial progenitor cells (EPCs), primary cells, fetal cells, stem cells, immune cells, epithelial cells, endothelial cells, cancer cells, white blood cells, adult stem cells, and/or epithelial progenitor cells (EPCs), as compared to other types of cells. A substrate can be configured to promote the selective adherence and growth of a particular type of target cell subpopulation. A substrate can promote selective adherence and growth of CTCs, CSCs, EPCs, stem cells, epithelial cells, primary cells, or cancer cells, as compared to other types of cells. A substrate can promote the selective adherence and growth of CTCs and/or CSC's as compared to other types of cells. Accordingly, the substrate may further enrich the target cell subpopulation. The substrate may enrich the ratio of target cell subpopulations/non-target cell populations by a factor of at least 1.5, 2, 4, 6, 8, 10, 20, 50, 100, 200, 500, 1,000, 2,000, 5,000, 10,000, 20,000, 50,000, 100,000, 200,000, 500,000, 1,000,000, 2,000,000, 5,000,000, 10,000,000, 20,000,000, 50,000,000, 100,000,000, 200,000,000, 500,000,000, 1,000,000,000, 2,000,000,000, or 5,000,000,000 fold over the original ratio.

The substrate can be configured to allow the target cell subpopulation to adopt one or more distinct morphological characteristics. In one non-limiting example, adoption of one or more distinct morphological characteristics can allow the target cell subpopulation to be distinct from non-target cell subpopulations. A substrate can thus enable the selection of target cell subpopulations from a heterogeneous population of cell subpopulations, based on the one or more morphological characteristics.

By way of example only, after plating on a substrate, CTCs and/or CSCs can be morphologically distinct from other cell subpopulations by a large surface area as compared to the surface area of non-target cell subpopulations. By way of example only, about 20 minutes to 1 hour after plating onto the substrate, the CTCs and/or CSCs spread out onto the substrate, assuming a diameter that is at least 1.5X, 2X, 3X, 4X, 5X, 6X, 7X, 8X, 9X, 10X, 15X, 20X, 25X, 30X, 35X, 40X, 45X, 50X, or more than 50X the diameter of the cells before contacting the substrate. The diameter of a CTC and/or CSC after contacting a substrate can be about 20 µm or greater, 25 µm or greater, 30 µm or greater,35 µm or greater, 40 µm or greater, 45 µm or greater, 50 µm or greater, 55 µm or greater, 60 µm or greater, 65 µm or greater, 70 µm or greater, 75 µm or greater, 80 µm or greater, 85 µm or greater 90 µm or greater, 95 µm or greater, 100 µm or greater, or more than 100 µm. Accordingly, the invention provides a method of contacting a heterogeneous cell population with a substrate, incubating the heterogeneous cell population under conditions sufficient to allow growth of a target cell subpopulation (e.g., CTCs and/or CSCs), and identifying a member of a target cell subpopulation if the member of the target cell subpopulation exhibits a large diameter.

In some embodiments, the CTCs and/or CSCs assume a "pancake" like shape after plating on a substrate, characterized by a cytoplasm with a very large surface area. The surface area of the cytoplasm of the CTC and/or CSC can be larger than the surface area of the nucleus of the CTC and/or CSC. For example, the surface area of the cytoplasm of the CTC and/or CSC can be 2-4X larger than the surface area of the nucleus. By contrast, the surface area of a cytoplasm of a non-CTC or non-CSC cell can be less than 2X the surface area of the nucleus. The surface area of a CTC and/or CSC after plating on a substrate can be at least 1.5X, 2X, 3X, 4X, 5X, 6X, 7X, 8X, 9X,10X, 12X, 15X, 20X, 25X, 30X, 40X, 50X, 60X, 70X, 80X, 90X, 100X, or greater than 100X larger than the surface area of a non-CTC or non-CSC cell. The surface area of a CTC and/or CSC after plating on the substrate can be at least 900 µm², 1000 µm², 1100 µm², 1200 µm², 1300 µm², 1400 µm², 1500 µm², 1600 µm², 1700 µm², 1800 µm², 1900 µm², 2000 µm², 2100 µm², 2200 µm², 2300 µm², 2400 µm², 2500 µm², 2600 µm², 2700 µm², 2800 µm², 2900 µm², 3000 µm², 3100 µm², 3200 µm², 3300 µm², 3400 µm², 3500 µm², 3600 µm², 3700 µm², 3800 µm², 3900 µm², 4000 µm², 4500 µm², 5000 µm², 5500 µm², 6000 µm², 6500 µm², 7000 µm², 7500 µm², 8000 µm², 8500 µm², 9000 µm², 9500 µm², 10000 µm², or more than 10000 µm². By contrast, the surface area of a non-CTC or non-CSC cell after plating on the substrate is about 500-700 µm². The size difference may not be detectable until cells have adhered to the substrate. Accordingly, the invention provides a method of contacting a heterogeneous cell population with a substrate, incubating the heterogeneous cell population under conditions sufficient to allow growth of a target cell subpopulation (e.g., CTCs and/or CSCs), and identifying a member of a target cell subpopulation if the member of the target cell subpopulation exhibits a surface area that is larger than a surface of area of a non-target cell.

CTCs and/or CSCs may exhibit multiple nuclei per cell after plating on the substrate. The CTCs and/or CSCs exhibit 2, 3, 4, 5, 6, 7, 8, 9, 10, or more than 10 nuclei. By contrast, non-CTC or non CSC cells generally exhibit one nucleus per cell. Accordingly, the invention provides a method of contacting a heterogeneous cell population with a substrate, incubating the heterogeneous cell population under conditions sufficient to allow growth of a target cell subpopulation (e.g., CTCs and/or CSCs), and identifying a member of a target cell subpopulation if the member of the target cell subpopulation exhibits more than one nucleus per cell.

The substrate can allow for EPCs to be identified based on morphological characteristics. EPCs, can form a spindle-like appearance once in contact with the substrate. The width of an EPC in contact with the substrate can be at least 2-5X smaller than the length of the EPC. Accordingly, the invention provides a method of contacting a heterogeneous cell population with a substrate, incubating the heterogeneous cell population under conditions sufficient to allow growth of a target cell subpopulation (e.g., EPCs), and identifying a member of a target cell subpopulation if the member of the target cell subpopulation exhibits a width that is at least 2-5X smaller than the length of the member of the target cell subpopulation.

The substrate can allows HSCs to be identified based on morphological characteristics. HSCs can form a round, compact appearance once in contact with the substrate. In some instances, the diameter of HCSs in contact with the substrate may not exceed 20 µm. In some instances the diameter of the HSCs after plating do not exceed 2X the diameter of HSCs when suspended in a culture medium. Accordingly, the invention provides a method of contacting a heterogeneous cell population with a substrate, incubating the heterogeneous cell population under conditions sufficient to allow growth of a target cell subpopulation (e.g., HSCs), and identifying a member of a target cell subpopulation if the member exhibits a diameter after plating that does not exceed 2X the diameter of the cell before adherence to the substrate.

The substrate can be configured to allow the target cell subpopulation to adopt one or more distinct kinetic features. In one non-limiting example, adoption of one or more distinct kinetic features can allow the target cell subpopulation to be distinct from non-target cell subpopulations. A substrate can thus enable the selection of target cell subpopulations from a heterogeneous population of cell subpopulations, based on the one or more kinetic features.

Exemplary kinetic features can include cell proliferation rates. For example, the substrate can be configured to promote the proliferation of different cell subpopulations in a heterogeneous population of cells at different rates. For example, a heterogeneous population of cells (comprising a plurality of cell subpopulations, e.g. non cancerous cells, CTCs, CSCs, and white blood cells) may be plated onto the substrate. When the heterogeneous population of cells is incubated for a period of time sufficient for proliferation, the different cell subpopulations can undergo proliferation at different rates. For example, non cancer cells may not undergo proliferation. For other example, CTCs may form subcolonies wherein each of the subcolonies undergo proliferation at different rates. The different rates of proliferation of the CTCs and/or CSCs may be more rapid than a proliferation rate of a non-cancer cell. Accordingly, the invention provides a method of contacting a heterogeneous cell population with a substrate, incubating the heterogeneous cell population under conditions sufficient to allow growth of a target cell subpopulation (e.g., CTCs and/or CSCs), and identifying a member of a target cell subpopulation if the member of the target cell subpopulation exhibits rapid cell division. Rapid cell division may be evidenced by a 30X increase in cell confluency within 3 days of plating on the substrate. Rapid cell division may be evidenced by a 100X increase in cell confluency within 5 days of plating on the substrate. Rapid cell division may be evidenced by a 100X increase in cell confluency within 10 days of plating on the substrate. Rapid cell division may be evidenced by a 2X or greater increase in proliferation of the target cell subpopulation as compared to proliferation of a non-target cell subpopulation.

Exemplary kinetic features can also include colony formation. A cell colony can be a cluster of cells growing on the surface of or within a solid medium. The colony can originate from a single cell. The cells within the cluster may grow on top of each other. The substrate can be configured to promote colony formation of target cell subpopulations without appreciable colony formation of non-target cell subpopulations. For example, upon plating onto a substrate, white blood cells may proliferate but may not form colonies. CTCs and/or CSCs may undergo colony formation upon plating onto a substrate. The colonies may be 3-dimensional colonies. The 3-dimensional colonies can be formed by the growth of CTCs and/or CSCs on top of each other. By contrast, non-CTC or non-CSC cells generally do not form 3-dimensional colonies. Accordingly, the invention provides a method of contacting a heterogeneous cell population with a substrate, incubating the heterogeneous cell population under conditions sufficient to allow growth of a target cell subpopulation (e.g., CTCs and/or CSCs), and identifying a member of a target cell subpopulation if the member of the target cell subpopulation exhibits 3-dimensional colony formation.

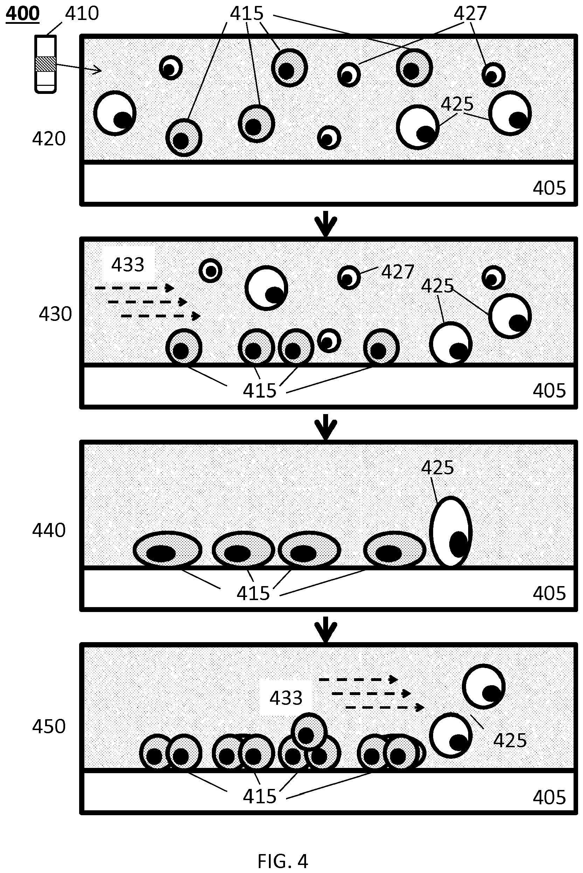

**FIG. 4** is an illustration diagramming an exemplary method **400** by which a target cell subpopulation **415** can be plated on a substrate **405** and adopt one or more distinct morphological characteristics and/or kinetic properties on the substrate. The mononuclear layer from a Ficoll-based density separation can be put in contact with a substrate. Panel **420** illustrates a sample **410** that contains a heterogeneous population of cells incubated with a substrate **405.** In this illustration, the heterogeneous population of cells contain: a target cell subpopulation **415,** white blood cells **425,** and other cells **427.**

Panel **430** illustrates the target cell subpopulation **415** adhering to the substrate **405** with a higher affinity than white blood cells **425** or other cells **427.** Cells that are not adhered to the substrate **405** can be removed, e.g., by washing with media **433** or other substance.

Panel **440** shows cells from the target cell subpopulation **415** spreading and beginning a round of cell division on the substrate **405.** A captured white blood cell **425** may divide but white blood cells **425** generally do not have the same spreading morphology or kinetic properties as target cell subpopulations **415.** Panel **450** depicts captured target cell subpopulations **415** that have undergone one or more rounds of cell division at different rates. The target cell subpopulations **415** may have formed colonies on the substrate. Captured white blood cells **425** may proliferate; however white blood cells generally not form colonies on the substrate **405.** Cells that are not in a colony nor adhered to the substrate **405** may be washed with media **433** or other substance.

### Exemplary Substrate Configurations

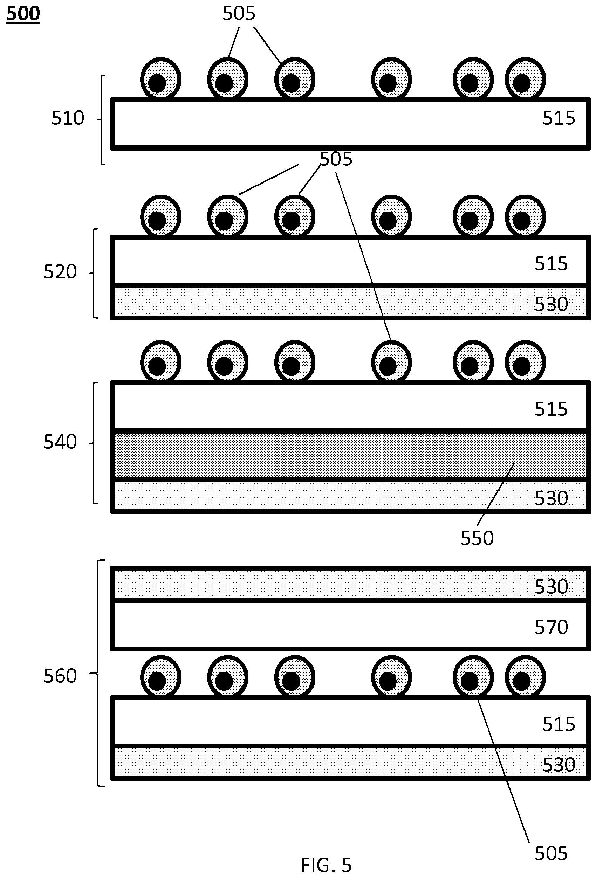

Many configurations of a substrate are encompassed in the invention. **FIG. 5** illustrates several different exemplary configurations of substrates illustrated with captured target cell subpopulations **505.** Panel **510** depicts an exemplary "single layer" configuration of a substrate, wherein the binding surface layer **515** is the only layer. Panel **520** depicts an exemplary "two layer" configuration of a substrate comprising a base layer **530** adjacent to a binding surface layer **515.** Panel **540** depicts an exemplary "middle-layer" configuration of a substrate comprising a base layer **530,** one or more middle layer(s) **550,** and a binding surface layer **515.** Panel **560** depicts an exemplary "inverted" configuration of a substrate comprising two base layers **530,** a binding surface layer **515,** and a feeding layer **570.** The inverted configuration can create a semi-sealed chamber wherein the outer layers are facing each other and the distance between the two substrates may range from around 0.1-20 mm, around 1-10 mm, or around 1-5 mm and can be approximately 0.1, 1, 2, 3, 4, 5, 6, 7, 8, 9, 10, 12, 15, 17, or 20 mm.

### Exemplary Base Layers

The base layer **530** can be made of plastic, glass, gelatin, components comprising the basal lamina, polyacrylamide or any combination thereof. Exemplary components comprising the basal lamina include, but are not limited to: laminin, type IV collagen, entanctin, and perlecan. The base layer can be configured to have a stiffness. In some cases, the stiffness of the base layer mimics the stiffness of soft tissue, such as brain tissue. In such cases, the stiffness of the base layer is 0.02-20 kilopascals (kPa), 0.05-1.5 kPa, or 0.1-1.0 kPa. In some cases, the stiffness of the base layer mimics the stiffness of bone tissue. In such cases, the stiffness can be 30-150 kPa, 40-125 kPa, or 50-100 kPa. In some instances, the material of the base layer may be configured as an apparatus. Examples of such commercially available apparatuses include: microscope slides, culture plates/dishes, Petri dishes, microscope coverslips, sealed culture dishes, and multi-well culture dishes (e.g., Millicell Cell Culture Inserts™; EMD Millipore Corporation).

### Exemplary Binding Surface Layers

The binding surface layer **515** can comprise one or more of, e.g., a cell monolayer, intracellular components (e.g., obtained from a cell lysate), a biological material associated with an extracellular matrix (ECM), gelatin, or any combination thereof.

The binding surface layer can comprise a cell monolayer. Cell monolayers can comprise, wholly or in part, mammalian cultured cells. The mammalian cultured cells can comprise a mammalian cell line. A mammalian cell line may be, for example, of an endothelial nature and may be of vascular, venous, or capillary origin. Non-limiting examples of mammalian cultured cells include mouse embryonic fibroblasts (MEFs), human umbilical vein endothelial cells (HUVEC) and human lung microvascular endothelial cells (HLMVEC). The cell line can be obtained from a primary source or from an immortalized cell line. In some instances, the monolayer of cells may be irradiated by ultraviolet light or X-ray sources to cause senescence of cells (e.g., to halt further proliferation of the monolayer cells). In some instances, the cell monolayer may contain a mixture of one or more different cell types (e.g. cells originating from different lineages or different cell lines). The different cell types may be co-cultured together. One non-limiting example is a combination of primary human endothelial cells co-cultured with transgenic mouse embryonic fibroblasts mixed to form a cell monolayer.

The binding surface layer can comprise a mixture of intracellular components. One method to obtain a mixture of intracellular components is by lysing cells (e.g. disrupting the cellular plasma membrane) and collecting the cytosolic components. The lysed cells may be primary or immortalized. Furthermore, the lysed cells may be from either mono- or co-cultures. Examples of cell lines from which to obtain lysates include, but are not limited to MEFs, HUVEC and HLMVEC cells. Cell lysates may be concentrated in their composition by removing excess solution. Excess solution may be removed in several different ways known by those skilled in the art, including: centrifugation, membrane-based dialysis, gas-aided evaporation, or any combination thereof.

The binding surface layer can comprise a biological material associated with an extracellular matrix (ECM) or binding moieties. Such biological materials associated with the ECM can include: collagen type I, collagen type IV, laminin, fibronectin, elastin, reticulin, hygroscopic molecules (glycosaminoglycanse, roteoglycans, glycocalyx), bovine serum albumin, Poly-L-lysine, Poly-D-lysine, and poly-ornithine (e.g., Poly-L-ornithine). In some embodiments, the biological material associated with an ECM is positively charged (e.g., poly-ornithine). There are also commercially available sources of biological materials associated with the ECM, one example of a commercially available source is Matrigel™ (BD Biosciences). Exemplary binding moieties are described herein.

The binding surface layer can comprise gelatin. The gelatin can be from an animal source. For example, the gelatin may be a porcine and/or bovine gelatin. In some instances, the binding surface layer can comprise gelatin (e.g. porcine gelatin) mixed with collagens. In some instances, gelatin can be mixed directly with one or more cell monolayers, intracellular components, binding moieties, biological materials associated with an ECM, or any combination thereof, to make a binding surface layer. In some instances the binding surface layer can comprise a commercially available product (e.g. Matrigel™ (BD Biosciences); Vita-Assay™ (Vitatex, NY)). In some cases, the binding surface layer can comprise serum.

### Exemplary Optional Middle Layer(s)

The substrate can have one or more middle layers when the substrate is in the "middle-layer" configuration **540.** In a "middle-layer" configuration **540,** there may be one or more middle layer(s) **550** between the base layer **530** and the binding surface layer **515.** The middle layer(s) of the sandwich may be one or more monolayers of cells. The cells of the monolayer may be of varying origin. One non-limiting example of a middle layer is made by growing a confluent monolayer of mouse embryonic fibroblasts on the base layer and then growing another layer of cells, the binding surface layer, on top of the confluent mouse embryonic fibroblasts.

### Exemplary Feeder Layer

A substrate having an "inverted" configuration **560** can comprise a feeder layer **570.** A feeder layer **570** can be adjacent to a base layer. The feeder layer can be separate from the binding surface layer. In some embodiments, a target cell subpopulation **505** preferentially binds to the binding surface layer **515** as compared to the feeder layer **570.** The feeder layer **570** may comprise a monolayer of feeder cells. The cells of the monolayer may be of any origin. Exemplary feeder cells are described herein. One non-limiting example of a feeder layer is made by growing a monolayer of human endothelial cells or mouse embryonic fibroblasts on a base layer.

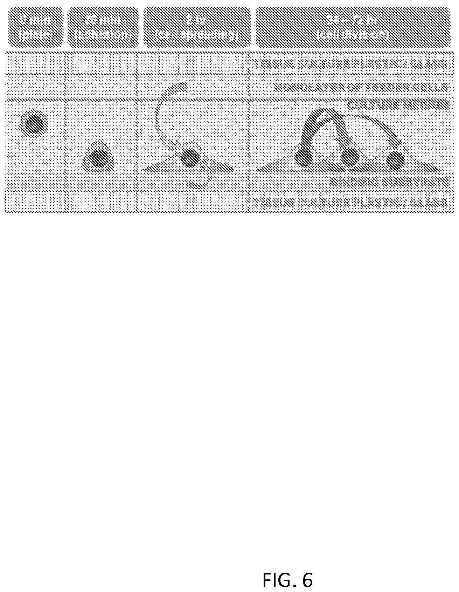

An exemplary "inverted layer" substrate is depicted in **FIG. 6****.** The exemplary "inverted layer" substrate comprises two base layers comprising tissue culture plastic and/or glass, a binding surface layer comprising a binding substrate (e.g., a biological material associated with an ECM), and a feeder layer comprising a monolayer of feeder cells. **FIG. 6** also depicts exemplary morphological and kinetic properties of a target cell after plating on the inverted layer substrate. Within 30 minutes of plating a target cell may exhibit adherence to the binding surface layer. Within 2 hours of plating, a target cell may exhibit cell spreading. Within 24-72 hours, a target cell may exhibit cell division, resulting in a colony of target cells.

An exemplary embodiment of a substrate, referred to herein as "GOL substrate", can comprise gelatin, a biological material associated with an ECM, and a serum. The gelatin may be a porcine gelatin. The biological material associated with an ECM may be poly-ornithine (e.g., poly-L-ornithine). The serum may be fetal bovine serum. In some embodiments, GOL substrate comprises porcine gelatin, poly-ornithine, and fetal bovine serum. For example, GOL substrate can comprise 0.01-10% porcine gelatin, 0.001-0.5 mg/ml poly-L-ornithine, and 0.5-20% fetal bovine serum. In one embodiment, GOL substrate comprises about 0.1% porcine gelatin, approximately 0.05 mg/mL Poly-L-ornithine hydrochloride, and approximately 5% fetal bovine serum (FBS).

Another exemplary embodiment of an invention substratum referred to herein as "GEL substrate" can comprise gelatin and one or more biological materials associated with an ECM. GEL substrate can comprise, for example, gelatin and two or more biological materials associated with an ECM. GEL substrate can comprise gelatin and three or more biological materials associated with an ECM. GEL substrate can comprise, for example, gelatin and collagen. GEL substrate can comprise gelatin, collagen, and/or laminin. GEL substrate can comprise gelatin, collagen, laminin, and/or fibronectin. In some instances, GEL substrate can comprise 0.01-10% porcine gelatin, 0.01-1 mg/ml collagen, 0.01-1 mg/ml laminin, and/or 0.01-1 mg/ml fibronectin. In one embodiment, GEL Substrate comprises about 0.1% porcine gelatin, and one or more of the following proteins: approximately 0.1 mg/mL of collagen (e.g. Sigma C7774); approximately 0.1 mg/mL laminin (e.g. Sigma L2020); and/or approximately 0.1 mg/mL fibronectin (e.g. Sigma F0895).

A substrate can be prepared by conjugation of one or more layers as described herein. Conjugation of layers may be accomplished by growing cells in a monolayer atop the base layer or middle layer. Conjugation of layers may also be accomplished by pre-treating the surface with a surface of either net positive, net negative, or net neutral charge. The conjugation procedure may be aided by chemical moieties, linkers, protein fragments, nucleotide fragments, or any combination thereof.

As one skilled in the art will recognize, the exact configuration and composition of the substrate can be tailored for enrichment of a particular target cell subpopulation. The composition of the substrate may vary based on patient type, cancer type, stage of cancer, patient medical history, and genomic and proteomic analysis of the patient tumor(s).

### Environmental Conditions Suitable for Growth of Target Cell Subpopulations

One of the challenges in providing sufficient numbers of target subpopulations cells, such as, e.g., CTCs or CSCs, for analysis is the inability to maintain cell viability long term. Accordingly, the invention provides culture methods to promote long-term in vitro viability and propagation of the target cell subpopulations. Culture methods can comprise incubating the target cell subpopulations in environmental conditions suitable for growth of the target cell subpopulations. Accordingly, the invention also provides a cell culture incubator comprising an enclosed environmental chamber, wherein the incubator is configured to maintain environmental conditions suitable for growth of a target cell subpopulation. Environmental conditions can be configured to mimic the in vivo vasculature system. Incubation of target cell subpopulations under one or more environmental conditions as described herein can increase adherence of target cell subpopulations to a substrate as compared to a non-target cell subpopulation. Incubation of target cell subpopulations under one or more environmental conditions as described herein can result in adherence of at least 1%, 5%, 10%, 15%, 20%, 25%, 30%, 35%, 40%, 45%, 50%, 55%, 60%, 65%, 70%, 75%, 80%, 85%, 90%, 95%, or more than 95% of the target cell subpopulations to the substrate. Incubation of target cell subpopulations under one or more environmental conditions as described herein can promote viability of the target cell subpopulations. For example, target cell subpopulations, upon incubation under one or more environmental conditions described herein, may remain viable for 1, 2, 3, 4, 5, 6, 7, 8, 9, 10, 11, 12 , or more than 12 weeks in culture. Incubation of target cell subpopulations under one or more environmental conditions as described herein can result in at least 15%, 20%, 25%, 30%, 35%, 40%, 45%, 50%, 55%, 60%, 65%, 70%, 75%, 80%, 85%, 90%, 95%, or more than 95% of the target cell subpopulations remaining viable for at least 1 week in culture. Incubation of target cell subpopulations under one or more environmental conditions as described herein can result in at least 15%, 20%, 25%, 30%, 35%, 40%, 45%, 50%, 55%, 60%, 65%, 70%, 75%, 80%, 85%, 90%, 95%, or more than 95% of the target cell subpopulations remaining viable for at least 2 weeks in culture. Incubation of target cell subpopulations under one or more environmental conditions as described herein can result in at least 15%, 20%, 25%, 30%, 35%, 40%, 45%, 50%, 55%, 60%, 65%, 70%, 75%, 80%, 85%, 90%, 95%, or more than 95% of the target cell subpopulations remaining viable for at least 4 weeks in culture. Incubation of target cell subpopulations under one or more environmental conditions as described herein can result in at least 15%, 20%, 25%, 30%, 35%, 40%, 45%, 50%, 55%, 60%, 65%, 70%, 75%, 80%, 85%, 90%, 95%, or more than 95% of the target cell subpopulations remaining viable indefinitely in culture.

Another challenge in providing sufficient numbers of target subpopulations cells, such as, e.g., CTCs or CSCs, for analysis is the inability to propagate the target cell subpopulations long-term in culture. Utilizing the methods of the invention, the target cell subpopulations may be propagated for 1, 2, 3, 4, 8, 12, or more than 12 weeks in culture. Utilizing the methods of the invention, at least 15%, 20%, 25%, 30%, 35%, 40%, 45%, 50%, 55%, 60%, 65%, 70%, 75%, 80%, 85%, 90%, 95%, or more than 95% of the target cell subpopulations after adhering to the substrate subpopulations may be propagated for 1, 2, 3, 4, 8, 12, or more than 12 weeks in culture. By utilizing the methods of the invention, the target cell subpopulations may remain viable and/or may be propagated indefinitely. In some embodiments, the culture methods comprise incubating the target cell subpopulations in environmental conditions suitable for growth. In some cases, the target cell subpopulation can be propagated for over 10, 15, 20, 25, 30, 35, 40, 45, 50, 55, 60, 65, 70, 75, 80, 85, 90, 95, 100, or over 100 doubling cycles.

Environmental conditions suitable for growth of a target cell subpopulation can comprise culturing in an enclosed environmental chamber comprising a defined internal ambient temperature. The enclosed environmental chamber may be an inner chamber of a cell culture incubator. The internal ambient temperature of the enclosed environmental chamber can be approximately 34 to approximately 42 degrees Celsius, including approximately 34, 35, 36, 37, 38, 39, 40, 41, or 42 degrees Celsius. Accordingly, a cell culture incubator comprises an enclosed environmental chamber, wherein the incubator is configured to maintain an internal ambient temperature of the enclosed environmental chamber.

Environmental conditions suitable for growth of a target cell subpopulation can comprise culturing in an enclosed environmental chamber comprising an internal gas composition. The gas composition of the enclosed environmental chamber can comprise a defined O₂ level. The defined O₂ level can range from a negligible amount of O₂ up to about 20% O₂ (e.g., O₂ accounting for about 20% of the internal gas composition), including approximately 0%, 0.1%, 0.5%, 1%, 2%, 3%, 4%, 5%, 6%, 7%, 8%, 9% or 10% O₂. The defined O₂ level can be configured to provide a hypoxic environment for culturing the target cell subpopulation. In some embodiments, the defined O₂ level is no more than 10%, no more than 5%, no more than 4%, no more than 3%, no more than 2%, no more than 1%, no more than 0.5%, no more than 0.4%, no more than 0.3%, no more than 0.2%, no more than 0.1%. In some instances, the O₂ level of the enclosed environmental chamber ranges from 0.5-2%. The O₂ level of the enclosed environmental chamber can be about 1%.

The gas composition can comprise a defined CO₂ level. For example, a target cell subpopulation can be grown in an enclosed environmental chamber comprising CO₂ levels between about 1% to about 15% CO₂, including approximately 1%, 2%, 3%, 4%, 5%, 6%, 7%, 8%, 9%, 10%, 11%, 12%, 13%, 14% or 15%. The CO₂ level of the enclosed environmental chamber can be between about 2-10%. The CO₂ level of the enclosed environmental chamber can be between about 3-8%. The CO₂ level of the enclosed environmental chamber can be about 5%.

The gas composition can comprise a nitrogen gas level. The nitrogen gas level can be between about 50-99.5%, including approximately 50%, 60%, 70%, 80%, 85%, 86%, 87%, 88%, 89%, 90%, 91%, 92%, 93%, 94%, 95%, 96%, 97%, 98%, 99%, 99.5%, or more than 99.5%. The nitrogen gas level can be about 90-99%, about 92-98% about 93-96%, or about 94%. In some embodiments, the nitrogen gas level is about 93%-94%. In particular cases, a target cell subpopulation is grown in an environment having about 1% O₂, 5% CO₂, and 94% nitrogen gas. In particular cases, a target cell subpopulation is grown in an environment having about 2% O₂, 5% CO₂, and 93% nitrogen gas.

Environmental conditions suitable for growth of a target cell subpopulation can comprise culturing under pressurized conditions. For example, environmental conditions suitable for growth of a target cell subpopulation can comprise culturing in an enclosed environmental chamber comprising a defined atmospheric pressure. The defined atmospheric pressure of the enclosed environmental chamber can be set by a user. For example, the defined atmospheric pressure of the enclosed environmental chamber can be set to produce a force equal or greater than the average pressure present in capillaries of the human vasculature system. The defined atmospheric pressure of the enclosed environmental chamber can be 15 PSIA or greater, can be 15.7 PSIA or greater, can be 16.7 PSIA or greater, can be 17.7 PSIA or greater, can be 18.7 PSIA or greater, or can be 19.7 PSIA or greater. The defined atmospheric pressure of the enclosed environmental chamber can be between about 15.7-19.7 PSIA, between about 15.7-18.7 PSIA, between about 15.7-17.7 PSIA, or can be about 16.7 PSIA. The incubation conditions may vary depending on the type of cell to be proliferated.

The environmental conditions suitable for growth of the target cell subpopulations comprise growing in hypoxic conditions in a pressurized environmental chamber. In some cases, the target cell subpopulations can be grown in an enclosed environmental chamber comprising a defined gas composition, a defined internal ambient temperature, and a defined internal atmospheric pressure. In particular cases, the defined gas composition comprises 1% O₂, 5% CO₂, 94% and nitrogen gas, the defined internal atmospheric pressure is 16.7 PSIA, and the defined internal ambient temperature is about 37°C.

The target cell subpopulations can be incubated in an environmental chamber that is configured for growth of anaerobic cells. Such environmental chambers can include, e.g., the Bactron™ anaerobic chamber from Shel Labs (www.shellab.com).

Accordingly, described herein is a cell culture incubator that is configured to provide environmental conditions suitable for growth of a target cell subpopulation. The cell culture incubator can be configured to maintain within an enclosed environmental chamber a defined gas composition, a defined internal ambient temperature, and a defined internal atmospheric pressure. The cell culture incubator can also be configured to maintain a defined internal humidity within the enclosed environmental chamber. Exemplary defined internal ambient temperatures, defined internal atmospheric pressure, and defined gas compositions are described herein. The cell culture incubator can comprise one or more control units. The one or more control units can be configured to maintain one or more of the defined gas composition, defined internal ambient temperature, and/or defined internal atmospheric pressure. Any one of the defined gas composition, defined internal ambient temperature, and defined internal atmospheric pressure can be controlled by a user. In some cases, the defined internal atmospheric pressure is controlled by a user. The cell culture incubator can comprise a user interface. The user interface can be configured to control any one of the defined gas composition, defined internal ambient temperature, and defined internal atmospheric pressure.

The cell culture incubator can be operably linked to one or more gas tanks. The one or more gas tanks can comprise a CO₂ tank, a nitrogen gas tank, an oxygen gas tank, a gas tank comprising a defined mixture or one or more gases, or any combination thereof. The cell culture incubator can be operably linked to the one or more gas tanks via a pressurized pump and/or pressure sensor (e.g., pressure gauge). The pressurized pump and/or pressure sensor can be configured to maintain a controlled flow of gas from the one or more gas tanks to the enclosed environmental chamber of the cell culture incubator. The controlled flow of gas from the one or more tanks can have a set inlet pressure, the set inlet pressure of the one or more tanks configured to maintain the desired internal gas composition and/or internal atmospheric pressure of the enclosed environmental chamber.

The cell culture incubator can comprise one or more sensors. The one or more sensors can be configured to provide a detectable alarm upon deviation of one or more of the gas composition, internal ambient temperature, and/or internal atmospheric pressure from the defined gas composition, defined internal ambient temperature, and/or defined internal atmospheric pressure. For example, the one or more sensors can provide a detectable alarm upon detection of an O₂ level of the enclosed environmental chamber if the detected O₂ level differs by more than ±0.5% from the defined O₂ level. The one or more sensor can provide a detectable alarm upon detection of an internal atmospheric pressure of the enclosed environmental chamber if the detected internal atmospheric pressure differs by more than ±0.5% from the defined atmospheric pressure. The one or more sensor can provide a detectable alarm upon detection of an internal ambient temperature of the enclosed environmental chamber if the detected internal ambient temperature differs by more than ±0.5% from the defined internal ambient temperature.

The cell culture incubator can comprise an oxygen removal catalyst. The oxygen removal catalyst may or may not be operably linked to the one or more gas tanks. Any oxygen removal catalyst known in the art or otherwise described herein may be used. Exemplary oxygen removal catalysts are commercially available from, e.g., BASF. Exemplary oxygen removal catalysts are described in US Patent Nos. 7,735,670 and 4,034, 062, and US Patent Application Publication No. 20090041646.

The cell culture incubator of the invention can be configured such that the enclosed environmental chamber of the incubator occupies a small volume of space. For example, the enclosed environmental chamber of the incubator can occupy no more than 3.5 cubic feet of space, no more than 22 cubic feet of space, no more than 1.5 cubic feet of space, or no more than 1 cubic foot of space. In some instances, the enclosed environmental chamber of the incubator occupies less than 1 cubic feet of space, e.g., occupies 0.5 ft X 0.5 ft X 0.5 ft of space. The cell culture incubator can be configured to occupy no more than 3.5 cubic feet of space, no more than 22 cubic feet of space, no more than 1.5 cubic feet of space, or no more than 1 cubic foot of space. In some instances, the cell culture incubator occupies less than 1 cubic feet of space, e.g., occupies 0.5 ft X 0.5 ft X 0.5 ft of space.

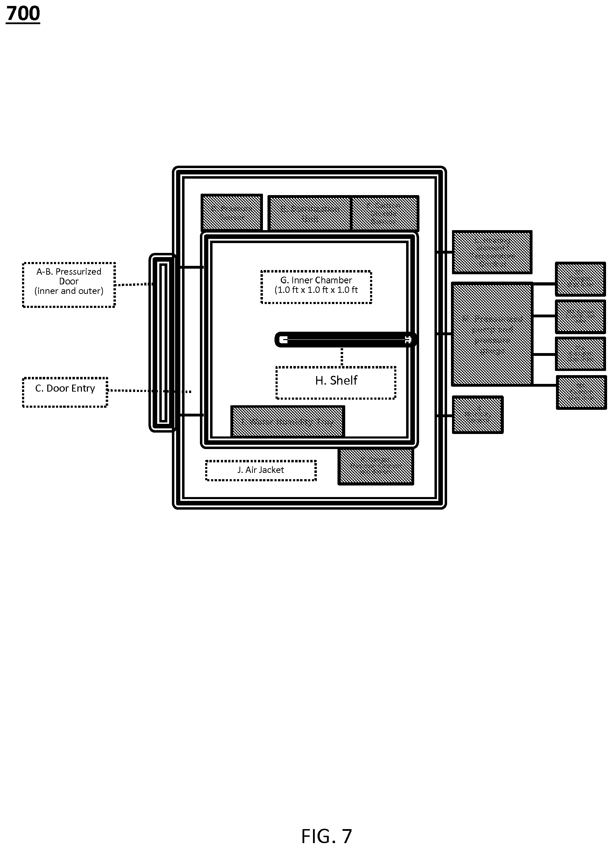

**FIG. 7A** depicts an exemplary cell culture incubator **700.** The incubator **700** can comprise a pressurized door. In some instances the incubator comprises an outer pressurized door and an inner pressurized door. The outer pressurized door and/or inner pressurized door can be, e.g., a double-walled door. The double-walled door can have a pressure-sealed latch. The outer pressurized door may include an integrate pressure sensor on the door. The incubator 700 can comprise a door entry. The door entry can provide an entrance into an enclosed environmental chamber. Dimensions of the door entry opening can be less than pressurized door. The door entry may comprise a rubber gasket. The rubber gasket can create a pressurized seal. The cell culture incubator can comprise an integrated pressure sensor. The integrated pressure sensor can be a manifold pressure sensor. The integrated pressure sensor can be a water-based or silicon based pressure sensor. The incubator 700 can comprise a sterilization unit. The sterilization unit can be a UV-based sterilization unit. The UV-based sterilization unit can be configured to provide UV rays to the entire space of an enclosed environmental chamber of the incubator. The incubator 700 can comprise a CO₂ sensor. The CO₂ sensor can be configured to provide a detectable alarm upon deviation of +/- 0.5% from a defined CO₂ level of the enclosed environmental chamber. The incubator 700 can comprise an enclosed environmental chamber. The volume occupied by the enclosed environmental chamber can be 1 cubic feet or less. The enclosed environmental chamber can comprise a shelf. The shelf can be removable from the enclosed environmental chamber. The shelf can comprise a metal material. The metal material can be stainless steel, copper, or other suitable metal. The incubator 700 can comprise a water humidity tray. The water humidity tray can promote sterility of the enclosed environmental chamber. The water humidity tray may be tethered directly to humidity sensor / regulator. The incubator 700 can comprise an air jacket. The air jacket can maintain optimal temperature and proper gas regulation. The air jacket may be a physically distinct compartment. The air jacket may house electrical controllers and circuit boards. The incubator 700 can comprise an oxygen removal catalyst and sensor for regulating oxygen levels within the incubator.

The incubator 700 can comprise a heating element and/or temperature control. The heating element and/or temperature control can comprise silent fan-based heating elements. The silent fan-based heating elements can dispense a constant flow of heated air into the air-jacket compartment. Passive heating can then warm the inner chamber in an evenly distributed and constant manner. The incubator can comprise a pressurized pump and regulator configured to provide a defined gas composition and internal atmospheric pressure. A motor based pump can dispense defined gas mixtures to maintain chamber pressure and gas composition levels (e.g., 1% oxygen, 5% CO₂, 94% N₂). The incubator can comprise a user interface. A user can use the user interface to set gas levels and pressures. The user interface can be integrated to the sensor and pump for direct control. The pressurized pump and regulator can comprise one or more gas inlets. The one of more gas inlets can be configured to allow flow of any gas into the enclosed environmental chamber. For example, the one or more gas inlets can be connected to an oxygen tank. The one or more gas inlets can be connected to a CO₂ tank. The one or more gas inlets can be connected to a nitrogen tank. In some instances, the incubator comprises a gas inlet connected to an oxygen tank, a gas inlet connected to a CO₂ tank, and a gas inlet connected to a nitrogen tank. The one or more gas inlets can be connected to a tank containing a custom gas mixture. In some instances, a user can control a flow of gas through any and/or each of the gas inlets. Any one of the gas inlets may be connected to a flow meter. The flow meter can regulate an inlet gas pressure. The cell culture incubator can comprise a humidity control unit/sensor. The humidity control unit/sensor may or may not be directly connected to the water humidity tray.

Incubating the target cell subpopulations in the specific environmental conditions described herein can enhance viability and proliferation of the target cell subpopulations (e.g., CTCs and/or CSCs). The in vivo microenvironment of various tissues and organs can have a unique pressure, stiffness, and/or gas composition that is amenable for new cancer growth. Without wishing to be bound by theory, it is possible that the culture methods of the present invention can mimic the in vivo microenvironmental conditions by the combination of growing the target cell subpopulations on the substrate having a particular stiffness as described herein, applying an external pressure as described herein, and setting the gas conditions (e.g., O₂, CO₂ conditions) as described herein. The environmental conditions as described herein can regulate the expression of genes and proteins that promote cell adhesion and subsequent cell division. For example, hypoxic conditions (e.g., 1% O₂) can induce the expression of Hypoxia Inducible Factor I (HIF-1), which has been shown to be highly expressed in proliferating CTCs.

Without wishing to be bound by theory, the environmental conditions as described herein may modulate the internal tension or stiffness of the target cell subpopulations (e.g., the CTCs and/or CSCs). By way of example only, with respect to CTC tension or stiffness, an increase in CTC stiffness may provide protective capabilities to withstand the shear forces found in the vasculature system. Conversely, a CTC that binds to a tissue that decreases its overall stiffness may be more likely to metastasize. The environmental conditions described herein can reduce cell tension, which can lead to an increase in cell size, a reduction in adhesive cell strength, and/or increase in cell motility. The cell tension and/or substrate stiffness that can promote cancer growth, can depend on the cancer type of the CTC. Accordingly, the environmental conditions as described herein can be modulated depending on the type of CTC(s) to be targeted.

### Exemplary Cell Culture Medium

Described are one or more cell culture media **125** (as depicted in **FIG. 1****,** also referred to herein as "enrichment media") to specifically enrich target cell subpopulations. For example, the cell culture medium can be formulated to selectively promote the growth of circulating tumor cells (CTCs), cancer stem cells (CSCs), hematopoietic stem cells (HSCs), endothelial progenitor cells (EPCs), fetal cells, stem cells, immune cells, epithelial cells, endothelial cells, cancer cells, white blood cells, adult stem cells, and/or epithelial progenitor cells, as compared to other types of cells. In particular instances, the cell culture medium promotes the selective growth of CTCs, CSCs, EPCs, stem cells, epithelial cells, or cancer cells, as compared to other types of cells. The cell culture medium may enrich the ratio of target cell subpopulations/non-target cell populations by a factor of at least 1.5, 2, 4, 6, 8, 10, 20, 50, 100, 200, 500, 1,000, 2,000, 5,000, 10,000, 20,000, 50,000, 100,000, 200,000, 500,000, 1,000,000, 2,000,000, 5,000,000, 10,000,000, 20,000,000, 50,000,000, 100,000,000, 200,000,000, 500,000,000, 1,000,000,000, 2,000,000,000, or 5,000,000,000 fold over the original ratio before culturing in the media.

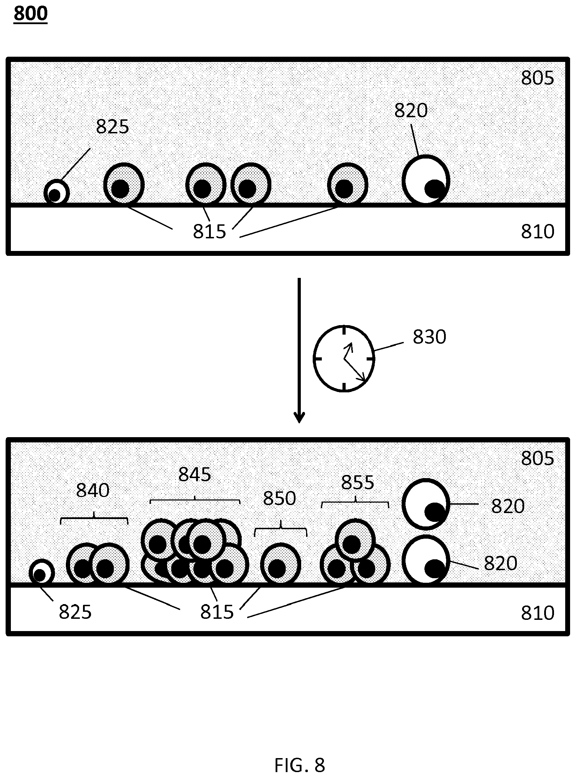

**FIG 8** depicts an exemplary instance **800** of enrichment media **805** formulated to promote proliferation of the target cell subpopulation, in this illustration the target cell subpopulation are CTCs **815.** The composition of the enrichment medium **805** can be tailored to the type of cell to be targeted for enrichment. There may be one or more formulations of enrichment media **805** used to enrich cells and the enrichment medium may be changed during the course of cell enrichment. The enrichment media **805** can be in contact with the substrate **810.** In one non-limiting example, the enrichment medium formulation is tailored to cancer type, stage of cancer, patient medical history, and genomic and proteomic analysis of the patient tumor(s). A heterogeneous population of cells (comprising a plurality of cell subpopulations, e.g. non cancerous cells **825,** CTCs **815,** and white blood cells **820**) may be plated on the substrate **810.** When the cells are incubated in enrichment media **805** for a period of time sufficient for proliferation **830,** the cell subpopulations can undergo proliferation at different rates. In one example, non cancer cells **825** may not undergo proliferation; CTCs **815** may form subcolonies that undergo proliferation at different rates (e.g., compare: **840, 845, 850,** and **855**); and white blood cells may undergo proliferation but do not form colonies **820.** In some embodiments, the enrichment media is formulated to promote colony formation of target cell subpopulations without appreciable colony formation of non-target cell subpopulations. A cell colony can be a cluster of cells growing on the surface of or within a solid medium. The colony can originate from a single cell. CTCs **815** may be classified based on their rate of proliferation. One example of a CTC **815** classification scheme is depicted as: non-dividing **850,** slow-dividing **840,** intermediate-dividing **855,** and fast-dividing **845.** Classification of cells based on their proliferation capacity in media can be related to the metastatic properties of the CTCs. In some embodiments, the enrichment media, substrate, and pressure conditions work synergistically to improve enrichment of target cells.

The cell culture medium can comprise a commercially available cell culture medium. Exemplary commercially available cell culture media that are suitable for use in the invention include, but are not limited to, RPMI medium, DMEM, MEM, F-12, DMEM/F12, F10 Nutrient Mixture, Media 199, Advanced™ Media, Opti-MEM®, IMDM, SensiCell™ Media, Fluorobrite™ DMEM media.

The cell culture medium **805** can be comprised of a primary nutrient source with specific additives to promote cell adherence to the substrate and to ensure long-term cell viability. The primary nutrient source can be derived from mammalian sources, or a mixture of mammalian sources. Some mammalian nutrient sources include but are not limited to: serum derived from calves, horses, goat, mice, rats, and/or humans. In some embodiments, one or more animal serums are mixed. Fetal calf serum and human serum can be mixed together with a mixture ratio of human serum to fetal calf serum of: approximately 1 to 1; approximately 1.5 to 1; or approximately 2 to 1. Additives that may be added include: ions, elements, calcium, glutamate, magnesium, zinc, iron, potassium, sodium, amino acids, vitamins, glucose, growth factors, hormones, tissue extracts, proteins, small molecules, or any combination thereof. In some instances, media culture may be supplemented or made with culture media that has been collected from cell cultures, blood plasma, or any combination thereof. In some instances, more than one type of enrichment media may be used. Exemplary amino acids include essential amino acids such as, e.g., phenylalanine, valine, threonine, tryptophan, isoleucine, methionine, leucine, lysine, and histidine, and other amino acids, such as, e.g., arginine, cysteine, glycine, glutamine, proline, serine, tyrosine, alanine, asparagines, aspartic acide, glutamic acid. Exemplary growth factors that may be included in the cell culture medium include Epidermal Growth Factor (EGF), Nerve Growth Factor (NGF), Brain Derived Neurotrophic Factor (BDNF), Fibroblast Growth Factor (FGF), Stem Cell Factor (SCF), Insulin-like Growth Factor (IGF), Transforming Growth Factor-beta (TGF-β). Exemplary hormones that may be included in the cell culture medium include, but are not limited to: peptide hormones such as, e.g., insulin, steroidal hormones such as, e.g., hydrocortisone, progesterone, testosterone, estrogen, dihydrotestosterone. Exemplary tissue extracts include, e.g., pituitary extract. Exemplary small molecule additives include, e.g., sodium pyruvate, Endothelin-1, transferrin, cholesterol, and the like.

In some embodiments, the cell culture medium comprises: RPMI-1640 (e.g., Sigma-Aldrich; R1145), approximately 20% fetal bovine serum; approximately between 0.2 to 1.0 g/L L-glutamine, approximately between 2 to 5 g/L high glucose; approximately 2 g/L sodium bicarbonate; and 25 approximately mM HEPES (e.g., Invitrogen; 15630106).

The cell culture medium can be a Plating Culture Medium. Plating Culture Medium can comprise: F12 medium (e.g., Sigma-Aldrich; N6658), approximately 5 g/L or between approximately 1 to 10 g/L D-(+)-Glucose (e.g., Sigma-Aldrich; G7021); 1X or optionally between 0.1 to 2X Minimum Essential Medium (MEM); approximately 10 mM or between 10 to 25 mM L-glutamine (e.g., Sigma-Aldrich; G7513); approximately 10mM or between 10 to 25 mM HEPES (e.g., Invitrogen; 15630106); and approximately 20% mammalian serum (e.g. fetal bovine serum, fetal calf serum, horse serum, human serum, human plasma, and etc.).

The cell culture medium can be a Type R Long Term Growth Medium. Type R Long Term Growth Medium may comprise: RPMI-1640 based medium (e.g., Sigma-Aldrich; R4130); approximately 5g/L or approximately 1 to 10 g/L D-(+)-Glucose (e.g., Sigma-Aldrich; G7021); approximately 1X MEM non-essential amino acids (e.g., Sigma-Aldrich; M7145); approximately 1X RPMI vitamins (e.g., Sigma-Aldrich; R7256); approximately 10 mM or between approximately 1 to 20 mM L- glutamine (e.g., Sigma-Aldrich; G7513); approximately 20% or between 5% to 50% mammalian serum (e.g., fetal bovine serum, fetal calf serum, horse serum, human serum, human plasma, and etc.). Optimally, the pH of the Type R Long Term Growth Medium can be adjusted to approximately 7.2 but the pH can range from approximately 6 to 8. Optional Type R Long Term Growth Medium supplements can include: 5 µg/mL or approximately 1 to 10 µg/mL insulin (e.g., recombinant human insulin (rh insulin)); 50 µg/mL or approximately 10 to 100 µg/mL ascorbic acid; 5 ng/mL or approximately 1 to 20 ng/mL epidermal growth factor (EGF) (e.g. recombinant human (rhEGF)); 0.75 units/ mL heparin sulfate; 1 µg/mL or approximately 0.5 to 2 µg/mL hydrocortisone hemisuccinate; 15 ng/mL or approximately 5 to 30 ng/mL insulin-like growth factor 1 (IGF-1) (e.g. rhIGF-1); 1 unit/mL or approximately 1 to 10 units/mL penicillin; 1 µg/mL or approximately 1 to 10 µg/mL streptomycin; approximately 0.1 to 25 ng/mL Amphotericin B; or any combination thereof.

The cell culture medium can be Type DF Long Term Growth Medium. Type DF Long Term Growth Medium may comprise: DMEM/F12 medium (e.g., Sigma-Aldrich; 51445C); 5 g/L or approximately 1 to 10 g/L D-(+)- Glucose (Sigma-Aldrich; G7021); approximately 1X MEM non-essential amino acids (Sigma-Aldrich; M7145); 10 mM or approximately 1 to 20 mM L-glutamine (e.g., Sigma-Aldrich; G7513); and 20% or approximately 5% to 50% mammalian serum (e.g. (e.g. fetal bovine serum, fetal calf serum, horse serum, human serum, human plasma, and etc.). Optimally, the pH of the Type DF Long Term Growth Medium can be adjusted to approximately 7.2 but the pH can range from approximately 6 to 8. Optional Type DF Long Term Growth Medium supplements can include: approximately 10mM or between 10 to 25 mM HEPES (e.g., Invitrogen; 15630106); 5 µg/mL or approximately 1 to 10 µg/mL insulin (e.g. recombinant human insulin (rh insulin)); 50 µg/mL or approximately 10 to 100 µg/mL ascorbic acid; 5 ng/mL or approximately 1 to 20 ng/mL epidermal growth factor (EGF) (e.g. recombinant human (rhEGF)); 0.75 units/ mL heparin sulfate; 1 µg/mL or approximately 0.5 to 2 µg/mL hydrocortisone hemisuccinate; 15 ng/mL or approximately 5 to 30 ng/mL insulin-like growth factor 1 (IGF-1) (e.g. rhIGF-1); 1 unit/mL or approximately 1 to 10 units/mL penicillin; 1 µg/mL or approximately 1 to 10 µg/mL streptomycin; approximately 0.1 to 25 ng/mL Amphotericin B; or any combination thereof.

The cell culture medium can be a Type D Long Term Growth Medium. Type D Long Term Growth Medium may comprise: DMEM medium, high glucose (Sigma-Aldrich; D6429); 5 g/L or approximately 1 to 10 g/L D-(+)- Glucose (Sigma-Aldrich; G7021); approximately 1X MEM non-essential amino acids (Sigma-Aldrich; M7145); 10 mM or approximately 1 to 20 mM L-glutamine (e.g., Sigma-Aldrich; G7513); and 20% or approximately 5 to 50% mammalian serum (e.g. fetal bovine serum, fetal calf serum, horse serum, human serum, human plasma, and etc.). Optimally, the pH of the Type D Long Term Growth Medium can be adjusted to approximately 7.2 but the pH can range from approximately 6 to 8. Optional Type D Long Term Growth Medium supplements can include: 10mM or between 10 to 25 mM HEPES (e.g., Invitrogen; 15630106); 5 µg/mL or approximately 1 to 10 µg/mL insulin (e.g. recombinant human insulin (rh insulin)); 50 µg/mL or approximately 10 to 100 µg/mL ascorbic acid; 5 ng/mL or approximately 1 to 20 ng/mL epidermal growth factor (EGF) (e.g. recombinant human (rhEGF)); 0.75 units/ mL heparin sulfate; 1 µg/mL or approximately 0.5 to 2 µg/mL hydrocortisone hemisuccinate; 15 ng/mL or approximately 5 to 30 ng/mL insulin-like growth factor 1 (IGF-1) (e.g. rhIGF-1); 1 unit/mL or approximately 1 to 10 units/mL penicillin; 1 µg/mL or approximately 1 to 10 µg/mL streptomycin; approximately 0.1 to 25 ng/mL Amphotericin B; or any combination thereof.

The cell culture medium can be pre-conditioned by exposing the medium to a conditioning cell line. Exemplary conditioning cell lines can include any of the feeder cells described herein. For example, the cell culture medium can be a Mouse Embryonic Fibroblast (MEF) - Conditioned Medium. MEF-Conditioned medium can comprise: DMEM medium, high glucose (Sigma-Aldrich; D6429); 5 g/L or approximately 1 to 10 g/L D-(+)- Glucose (Sigma-Aldrich; G7021); approximately 1X MEM non-essential amino acids (Sigma-Aldrich; M7145); 10 mM or approximately 1 to 20 mM L-glutamine (e.g., Sigma-Aldrich; G7513); 10mM or between 10 to 25 mM HEPES (e.g., Invitrogen; 15630106); approximately 10% mammalian serum (e.g. fetal bovine serum, fetal calf serum, horse serum, human serum, human plasma, and etc.). MEF medium can become "conditioned" after being cultured in the presence of MEF cells. In some embodiments, the MEF cells are cultured for a minimum of 48 hours, until the cell confluency is at least 70%.

In any of the cell culture media described herein, small molecules and drugs regulating cell tension may be added as a supplement to enhance cell viability and propagation. For example, rho kinase is important for regulating the contractile machinery of a cell, through its action on myosin II, which is a cytoskeleton-associated protein that binds to filamentous actin (F-actin) (the two proteins are commonly referred to as actomyosin bundles or filaments). Actomyosin bundles are the major contractile machines found in skeletal muscle. The presence of a rho kinase inhibitor in an enrichment medium can reduce cell tension, leading to an increase in cell size, reduction in adhesive cell strength, and increase in cell motility - mimicking a substrate surface elasticity that can be described as softer (e.g. 1000 to 2000 Pa). Such commercially available compounds/drugs include Blebbistatin (Straight et al. 2003), ML-7 and 9 (Saitoh et al. 1987), Y-27623 (Ishizaki et al. 2000 Pharmacological Properties of Y-27632, a specific inhibitor of rho-associated kinases. Mol Pharmacol. 57(5):976-83), Calyculin A (Kato et al. 1988), and or equivalents that control intracellular forces generated by the cytoskeleton and its upstream regulators (Rho Kinase I and II, Myosin Light chain Kinase, LIM Kinase). All of the above references are hereby incorporated by reference in their entirety.

The structure of Blebbistatin is as follows:

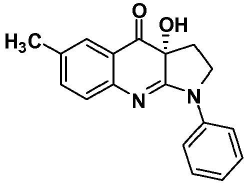

The structure of ML-7 is as follows:

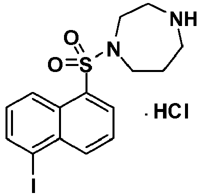

The structure of ML-9 is as follows:

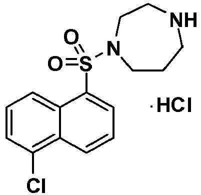

The structure of Y27623 is as follows:

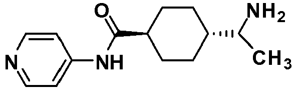

The structure of Calyculin A is as follows:

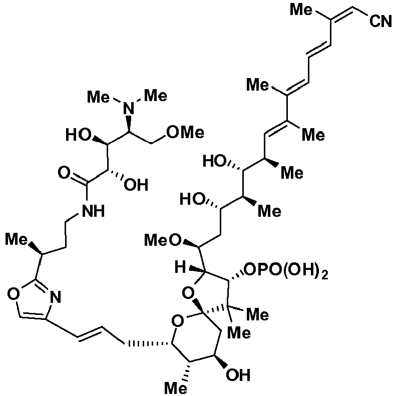

Such cell-tension mediating supplements can affect intermediate filaments, microtubules and actomyosin filaments. The small molecules/drugs as described herein can regulate the expression of genes and proteins that are regulated by pressure and/or hypoxic environmental conditions. In some embodiments, supplementation of the enrichment media with the small molecules and/or drugs as described herein can be used in lieu of the external pressure and/or hypoxic conditions as described herein, enabling the target cell subpopulations to be cultured under normoxia conditions and at normal atmospheric pressure.

Any of the foregoing methods can comprise culturing with an agent that increases expression of a hypoxia-inducible factor in the cell. Hypoxia-inducible factors generally refer to transcription factors that are activated by hypoxic environmental conditions. Exemplary hypoxia-inducible factors include, but are not limited to Hypoxia Inducible Factor-1α (HIF-1α), Hypoxia Inducible Factor-1β (HIF-1β), Hypoxia Inducible Factor-2α (HIF-2α), Hypoxia Inducible Factor-2β (HIF-2β), Hypoxia Inducible Factor-3α (HIF-3α), and Hypoxia Inducible Factor-3β (HIP-3β). In particular embodiments, the agent increases expression of HIF-1α.
The agent can comprise a polynucleotide encoding the hypoxia-inducible factor. For example, the agent can comprise a coding sequence for any of the hypoxia-inducible factors described herein. The coding sequence can encode HIF-1α polypeptide. The HIF-1α polypeptide can comprise an amino acid sequence that has at least 70% or greater homology to SEQ. ID. NO. 1.

The coding sequence for HIF-1α can comprise a polynucleotide sequence having at least 70% homology or complementarity to SEQ. ID. NO. 2.

The agent can be a vector comprising the polynucleotide which encodes the hypoxia-inducible factor. Vectors can include any plasmid, virus, viral vector, or other vehicle known in the art that can be manipulated by insertion or incorporation of a nucleic acid. For example, "cloning vectors" can be employed for the purposes of genetic manipulation. For another example, "expression vectors" can be employed to transcribe or translate the inserted polynucleotide.

Viral vectors can be based on retroviral, adeno-associated virus (AAV), lentiviral, adenoviral, retrovirus, or rotaviral genomes, simian virus 40 (SV40) or bovine papilloma virus (Cone et al., Proc. Natl. Acad. Sci. USA 81:6349 (1984); Eukaryotic Viral Vectors, Cold Spring Harbor Laboratory, Gluzman ed., 1982; Sarver et al., Mol. Cell. Biol. 1:486 (1981). Additional viral vectors useful for expression include Norwalk virus, coronaviruses, paramyxo and rhabdoviruses, togavirus (e.g., sindbis virus and semliki forest virus), parvovirus, and vesicular stomatitis virus (VSV).

Vectors can also include cytomegalovirus (CMV) based vectors (U.S. Pat. No. 5,561,063). Vectors that efficiently deliver genes to cells of the intestinal tract have been developed (see, e.g., U.S. Pat. Nos. 5,821,235, 5,786,340 and 6,110,456).

A vector generally contains an origin of replication for propagation in a cell. Control elements, including expression control elements as set forth herein, present within a vector, can be included to facilitate transcription and translation.

Suitable transcription or translational control sequences include but are not limited to replication origin, promoter, enhancer, repressor binding regions, transcription initiation sites, ribosome binding sites, translation initiation sites, and termination sites for transcription and translation.

As used herein, a "promoter" is a DNA region capable of binding RNA polymerase and initiating transcription of a coding region located downstream (in the 3' direction) from the promoter. The promoter can be constitutive or inducible. In general, the promoter sequence is bounded at its 3' terminus by the transcription initiation site and extends upstream (5' direction) to include the minimum number of bases or elements necessary to initiate transcription at levels detectable above background. Within the promoter sequence is a transcription initiation site, as well as protein binding domains responsible for the binding of RNA polymerase. Eukaryotic promoters can contain "TATA" boxes and "CAT" boxes.

The choice of promoters will largely depend on the host cells in which the vector is introduced. For animal cells, a variety of robust promoters, both viral and non-viral promoters, are known in the art. Non-limiting representative viral promoters include CMV, the early and late promoters of SV40 virus, promoters of various types of adenoviruses (e.g. adenovirus 2) and adeno-associated viruses. It is also possible, and often desirable, to utilize promoters normally associated with a desired light or heavy chain gene, provided that such control sequences are compatible with the host cell system.

Suitable promoter sequences for other eukaryotic cells include the promoters for 3-phosphoglycerate kinase, or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other promoters, which have the additional advantage of transcription controlled by growth conditions, are the promoter regions for alcohol dehydrogenase 2, isocytochrome C, acid phosphatase, degradative enzymes associated with nitrogen metabolism, and the aforementioned glyceraldehyde-3-phosphate dehydrogenase, and enzymes responsible for maltose and galactose utilization.

A vector can be introduced into a cultured cell by any means known in the art, including transfection, viral transduction, and the like. Transfection methods can comprise opening transient pores or "holes" in a cell membrane to allow the uptake of material. Transfection can be carried out using, e.g., calcium phosphate, by electroporation, by optical transfection, by impalefaction, by hydrodynamic delivery, by gene gun, by magnet assisted transfection, by mixing a cationic lipid with the material to produce liposomes which may fuse with the cell membrane and deposit their cargo inside, by use of cationic polymers such as, e.g., DEAE-dextran or polyethylenimine.

### Identification of Target Cells

The substrates, cell culture media, environmental conditions, and/or cell culture methods described herein can allow the target cell subpopulation to adopt one or more distinct morphological characteristics and/or kinetic features. Any of the distinct morphological characteristics and/or kinetic features can be used to distinguish and/or identify a member of a target cell subpopulation. Exemplary morphological characteristics and/or kinetic features are described herein. Accordingly, the invention provides a method of identifying and/or selecting a member of a target cell subpopulation based on a display of one or more distinct morphological characteristics and/or kinetic features adopted by the target cell upon plating on an invention substrate, upon culturing in a cell culture medium described herein, and/or upon culturing under environmental conditions as described herein.

Such methods of identifying and/or selecting a member of a target cell subpopulation can obviate a necessity for use of a positive selection antibody for target cell identification. Accordingly, in some embodiments, a method of identifying and/or selecting a member of a target cell subpopulation does not comprise use of a positive selection antibody. However, a method of identifying and/or selecting a member of a target cell subpopulation can in some instances comprise use of a positive selection antibody. For example, a method of identifying and/or selecting a member of a target cell subpopulation can comprise identifying said member if said member displays one or more distinct morphological characteristics and/or kinetic features, and/or if said member is detectably labeled with a positive selection antibody.

The methods of identifying and/or selecting a member of a target cell subpopulation described herein can obviate a necessity for use of a negative selection antibody for target cell identification. Accordingly, in some embodiments, a method of identifying and/or selecting a member of a target cell subpopulation does not comprise use of a negative selection antibody. However, a method of identifying and/or selecting a member of a target cell subpopulation can in some instances comprise use of a negative selection antibody. For example, a method of identifying and/or selecting a member of a target cell subpopulation can comprise identifying said member if said member displays one or more distinct morphological characteristics and/or kinetic features, and/or if said member is not detectably labeled with a negative selection antibody.

### Computer Readable Medium

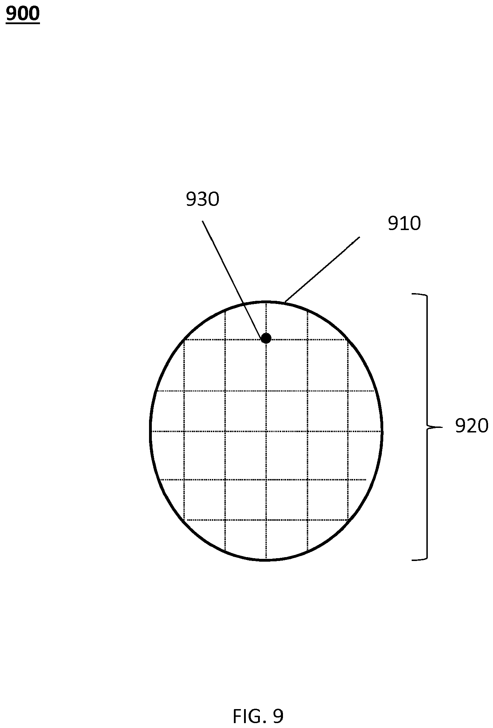

Described herein is a computer readable medium comprising a computer executable code which is configured to implement any of the methods described herein. The computer readable medium can implement a method for semi-automated or automated identification, enumeration, and/or classification of target cell subpopulations. The identification, enumeration, and/or classification of the target cell subpopulations can be based on one or more morphological and/or kinetic properties adopted by the cell upon culturing under conditions described herein. The identification, enumeration, and/or classification of the target cell subpopulations can be based on one or more morphological and/or kinetic properties adopted by the cell upon plating on a substrate described herein. The one or more morphological and/or kinetic properties of the cells may be assessed over time. For example, the one or more morphological and/or kinetic properties of the cells may be assessed over time in a coordinate system **900** as depicted in **FIG. 9****.** The software is configured to create a virtual coordinate system **920** to reference the location of detected target cell subpopulations on a substrate in a culture dish or well **910.** The virtual coordinate system is propagated by aligning the culture dish to a user-defined point of origin ("point of origin") **930** and a virtual grid **920** that is made up of scalable vector quadrants is overlaid **900.** In one instance, the virtual coordinate system is used by the image acquisition system to facilitate an automated time-lapse capture across the entire surface of a culture dish. Some examples of standard-sized culture dishes and/or wells that have been used include: 10 mm, 35 mm, 60 mm, and 100 mm and wells of a high multi-well plate (e.g. 6 well, 12 well, 24 well, 96 well, 384 wells, 1056 wells, 1536 wells, or 3456 wells) but other sizes of culture dishes are also contemplated. In one instance, an operator can revisit the location of specific colonies of particular interest, and cells can be collected through the use of a micromanipulator and transjector. An individual cell may be captured for use in targeted biomedical studies or can be harvested and used for further culturing experiments.

The computer readable medium may use the coordinate system **900** to automatically capture, recombine, realign, and/or optimize images. For example, the computer readable medium may stitch any number of images comprising neighboring regions. This automated stitching process can create a single stitched image for a given time point. In some cases, a stitched image can encompass between approximately 25 to approximately 200 images. Stitched images may undergo an initialization process whereby the images will be compressed to reduce the file size. Contrast-based filters can be applied to the stitched image.

The computer readable medium can determine number of total cells, number of viable and non-viable cells, number of actively dividing cells and the number of cell colonies, ratios between viable and non-viable cells, the ratio between dividing and non-dividing cells. Such analyses can be used to determine the aggressiveness or malignant potential of a patient's CTCs. For example, rapidly dividing cells can be considered an 'aggressive', e.g., metastatic, class of CTCs.

### Identification and Classification Parameters

The computer readable medium can comprise software which uses any of the morphological characteristics and/or kinetic properties of a cell as described herein to identify and characterize target cell subpopulations. For example, the computer readable medium may use the following parameters to identify and classify target cell subpopulations: cell size, cell shape, cell movement, nucleus size and cell division rate. The software can integrate such parameters into an algorithm for classifying a target cell subpopulation. The software can process the parameters in cycles, processed either sequentially or tangentially using, e.g., multicore computer chips. Optionally, a user may assign different weights to each of the said parameters.

### Enhancement of Cell Detection

In some instances, the visualization of target cell subpopulations can be enhanced by use of cell permeable dyes and/or antibodies against cell-adhesion proteins. Cell-permeable fluorescent dyes may be used to aid the analysis software to determine the overall cell and nuclei shape, size, and number. There are several cell-permeable dyes known to those skilled in the art. Some non-limiting examples include: calcium-based dyes (e.g., Calcium-Green-1 AM; Life Technologies) or fluorescently conjugated dextran.

Analysis software may also be aided through the visualization of mitochondria. Mitochondria specific antibodies or permeable dyes (e.g., MitoTracker™; Life Technologies) may be used to enhance the detection of target cell subpopulations. Furthermore, the complete absence or overabundance of mitochondria for given cell subpopulation or colony can be used as an additional parameter for subclassification, however those skilled in the art will recognize there may be other cell-permeable fluorescent dyes that may be used to aid detection.

Antibodies directed against cell surface molecules, such as cell adhesion molecules may also be used to enhance detection of a target cell subpopulation. Analysis software can be aided through the use of known biomarkers that mark a target cell subpopulation. Targeting antibodies that are conjugated to a detection moiety can be used to enhance detection, and subclassify cells based on the presence of specific biomarkers. Known biomarkers for CTCs include EpCAM, EGFR, and CD133, however those skilled in the art will recognizes there have been other biomarkers identified. Analysis software can be aided through use of a known biomarker that is expressed in a non-target cell subpopulation but is not generally expressed, or expressed at lower levels in a target cell subpopulation. Known biomarkers that are selective for non-target cells include CD45, CD14, CD34, and fibroblast surface protein, among others.

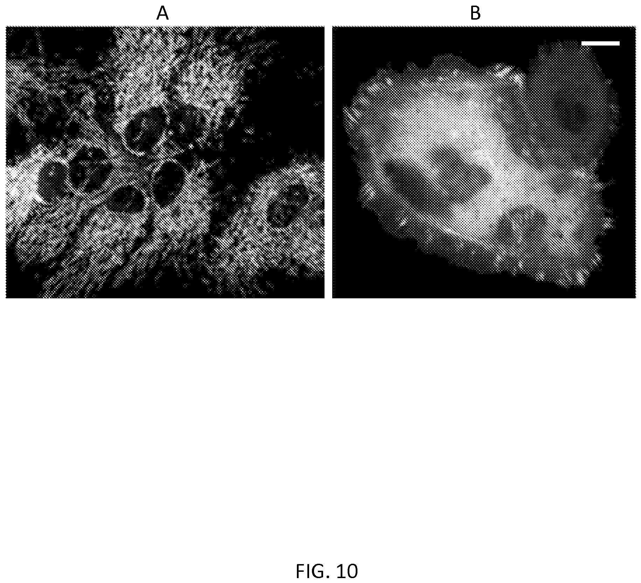

**FIG. 10** is one such example of enhanced cell detection (figure adopted from a color image). Panel **A** shows labeled cancer cells cultured using methods of the invention; the mitochondria of the cells are labeled with MitoTracker. Panel **B** shows a cancer cell labeled with fluorescently conjugated antibodies against the cell adhesion protein, paxillin.

### Computer Systems

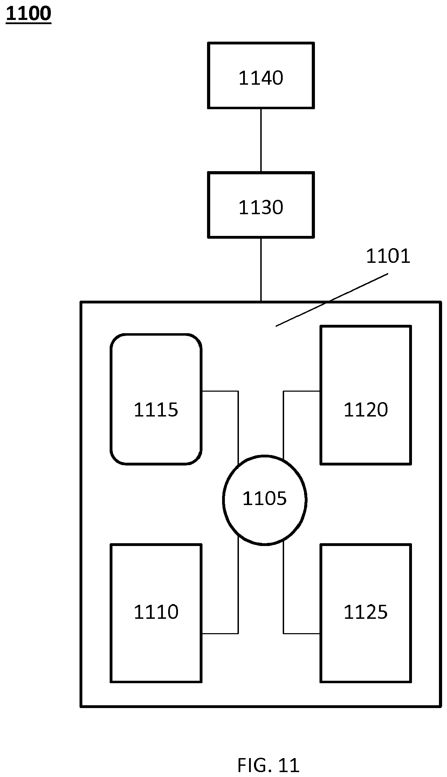

Described herein is a computer system that is configured to implement the methods of the disclosure. The system can include a computer server ("server") that is programmed to implement the methods described herein. **FIG 11** depicts a system **1100** adapted to enable a user to detect, analyze, and process images of cells. The system **1100** includes a central computer server **1101** that is programmed to implement exemplary methods described herein. The server **1101** includes a central processing unit (CPU, also "processor") **1105** which can be a single core processor, a multi core processor, or plurality of processors for parallel processing. The server **1101** also includes memory **1110** (e.g. random access memory, read-only memory, flash memory); electronic storage unit **1115** (e.g. hard disk); communications interface **1120** (e.g. network adaptor) for communicating with one or more other systems; and peripheral devices **1125** which may include cache, other memory, data storage, and/or electronic display adaptors. The memory **1110,** storage unit **1115,** interface **1120,** and peripheral devices **1125** are in communication with the processor **1105** through a communications bus (solid lines), such as a motherboard. The storage unit **1115** can be a data storage unit for storing data. The server **1101** is operatively coupled to a computer network ("network") **1130** with the aid of the communications interface **1120.** The network **1130** can be the Internet, an intranet and/or an extranet, an intranet and/or extranet that is in communication with the Internet, a telecommunication or data network. The network **1130** in some cases, with the aid of the server **1101,** can implement a peer-to-peer network, which may enable devices coupled to the server **1101** to behave as a client or a server. The microscope and micromanipulator can be peripheral devices **1125** or remote computer systems **1140.**

The storage unit **1115** can store files, such as individual images, time lapse images, data about individual cells, cell colonies, or any aspect of data associated with the invention. The data storage unit **1115** may be coupled with data relating to locations of cells in a virtual grid.

The server can communicate with one or more remote computer systems through the network **1130.** The one or more remote computer systems may be, for example, personal computers, laptops, tablets, telephones, Smart phones, or personal digital assistants.

In some situations the system **1100** includes a single server **1101.** In other situations, the system includes multiple servers in communication with one another through an intranet, extranet and/or the Internet.

The server **1101** can be adapted to store cell profile information, such as, for example, cell size, morphology, shape, migratory ability, proliferative capacity, kinetic properties, and/or other information of potential relevance. Such information can be stored on the storage unit **1115** or the server **1101** and such data can be transmitted through a network.

Methods as described herein can be implemented by way of machine (e.g., computer processor) computer readable medium (or software) stored on an electronic storage location of the server **1101,** such as, for example, on the memory **1110,** or electronic storage unit **1115.** During use, the code can be executed by the processor **1105.** In some cases, the code can be retrieved from the storage unit **1115** and stored on the memory **1110** for ready access by the processor **1105.** In some situations, the electronic storage unit **1115** can be precluded, and machine-executable instructions are stored on memory **1110.** Alternatively, the code can be executed on a second computer system **1140.**

Aspects of the systems and methods provided herein, such as the server **1101,** can be embodied in programming. Various aspects of the technology may be thought of as "products" or "articles of manufacture" typically in the form of machine (or processor) executable code and/or associated data that is carried on or embodied in a type of machine readable medium (e.g., computer readable medium). Machine-executable code can be stored on an electronic storage unit, such memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical, and electromagnetic waves, such as used across physical interfaces between local devices, through wired and optical landline networks and over various air-links. The physical elements that carry such waves, such as wired or wireless likes, optical links, or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, tangible storage medium, a carrier wave medium, or physical transmission medium. Non-volatile storage media can include, for example, optical or magnetic disks, such as any of the storage devices in any computer(s) or the like, such may be used to implement the system. Tangible transmission media can include: coaxial cables, copper wires, and fiber optics (including the wires that comprise a bus within a computer system). Carrier-wave transmission media may take the form of electric or electromagnetic signals, or acoustic or light waves such as those generated during radio frequency (RF) and infrared (IR) data communications. Common forms of computer-readable media therefore include, for example: a floppy disk, a flexible disk, hard disk, magnetic tape, any other magnetic medium, a CD-ROM, DVD, DVD-ROM, any other optical medium, punch cards, paper tame, any other physical storage medium with patterns of holes, a RAM, a ROM, a PROM and EPROM, a FLASH-EPROM, any other memory chip or cartridge, a carrier wave transporting data or instructions, cables, or links transporting such carrier wave, or any other medium from which a computer may read programming code and/or data. Many of these forms of computer readable media may be involved in carrying one or more sequences of one or more instructions to a processor for execution.

The results of cell monitoring can be presented to a user with the aid of a user interface, such as a graphical user interface.

### Cellular Imaging System

The invention also provides a cellular imaging system. The cellular imaging system may be configured to generate image data corresponding to a cell of a cell population. The cellular imaging system may be configured to generate image data corresponding to a primary cell. The cellular imaging system may be configured for high content analysis of a target cell subpopulation.

The cellular imaging system may comprise a microscope. The microscope may comprise system hardware. System hardware may comprise hardware for microscopy control. An exemplary microscopy hardware control is developed on top of the open-source, java-based image acquisition software, µManager (Arthur Edelstein, et al. (2010) incorporated herein by reference, Computer Control of Microscopes Using µManager. Current Protocols in Molecular Biology 14.20.1-14.20.17, incorporated herein by reference). Custom drivers and scripts can be used to control any of the following microscopy-essential peripherals: image acquisition camera, camera-shutter, motorized linear-encoded X-Y stage, piezo-driven Z-axis stepper, and near-infrared based autofocus.

The microscope may be an inverted microscope. The inverted microscope may comprise a customized environmental chamber. The custom environmental chamber may be configured to provide environmental conditions (e.g., temperature, CO₂, oxygen, pressure) suitable for maintaining viability of a cultured cell. The environmental conditions may be any of the conditions described herein suitable for growth of a target cell subpopulation. The customized environmental chamber may regulate temperature, CO₂, and oxygen levels with, e.g., 0.1% precision. The microscope can include 5X, 10X, 20X, 40X non-oil objectives with fluorescence capabilities but other objectives are contemplated. The inverted microscope may include a motorized stage and hardware-based auto-focusing with a brightfield light source, with phase-shifted polarization filters to enhance contrast for the detection of cells. The light source, can be halogen, xenon, mercury, solid-state lasers or light-emitting diode.

The cellular imaging system may further comprise a computer system. The computer system can be as described herein. In some embodiments, the computer system comprises a computer readable medium as described herein.

In any of the methods described herein, a member of a target cell subpopulation can be collected or marked using, e.g., micromanipulation. Micromanipulation can be accomplished using any techniques known in the art, including a motorized micromanipulator and transjector or laser microdissection. A user can revisit the location of specific colonies or cells of particular interest. This can allow, for example, the user to individually grab cells for targeted biomedical studies at the single cell level, harvest cells for further culturing experiments, or perform chemosensitivity testing. Accordingly, a cellular imaging system can further comprise a micromanipulator.

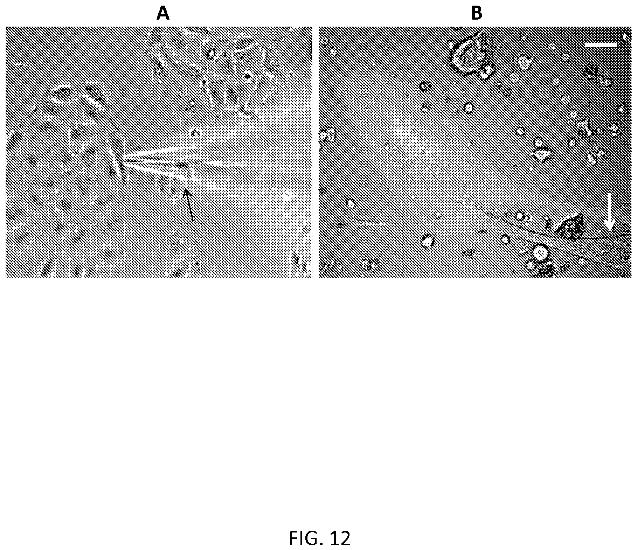

An exemplary motorized micromanipulator and transjector is shown in **FIG 12****.** Panel A depicts a single cell selection; the injection/selection needle of a motor-driven micromanipulator (arrow), isolates a single cancer cell from a colony of cells derived from the cancer cell line (MDA-MB231). Panel B depicts the injection of a single cell with fluorescently conjugated dextran; the white arrow points to an uninjected cancer cell (image adopted from color image).

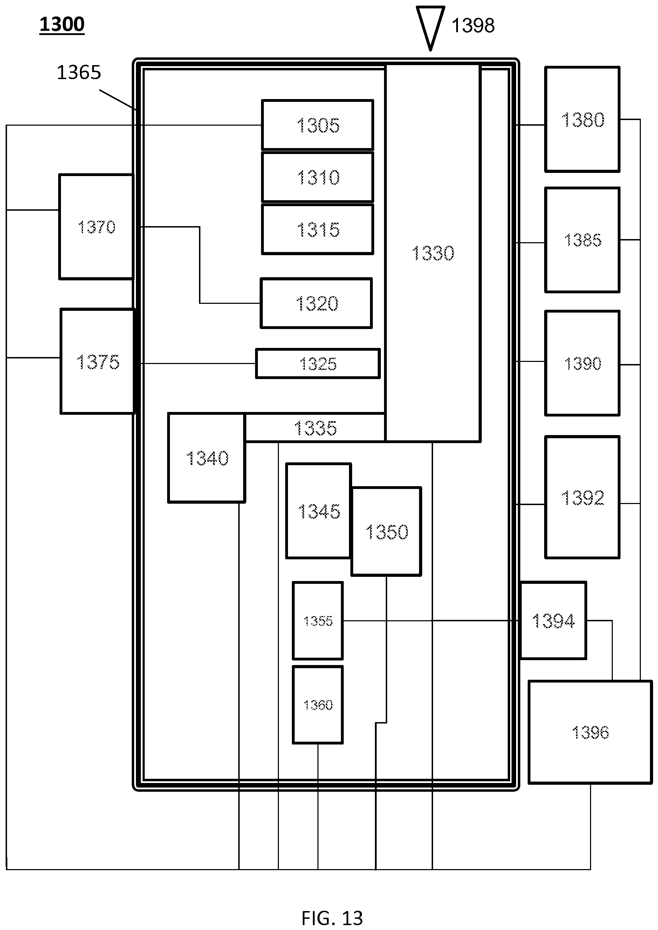

**FIG 13** depicts an exemplary cellular imaging system **1300** configured for automated cell culture and/or high content analysis. The system **1300** includes a light source **1305** which can be bright field and can provide illumination for phase-based light microscopy. The light source **1305** can be filament or light emitting diode (LED) based. The system **1300** can also include a phase contrast ring with a turret **1310.** In some instances, the turret can be motorized and compatible contrast rings can be paired to the proper objective **1345.**

The system **1300** may optionally include a light condenser **1315.** In some instances, a light condenser **1315** may be used to focus the light source which in some instances can improve the contrast of cells. The system may also optionally include a cell selector (e.g. a cell picker) **1320.** In some instances the cell selector can select single cells (e.g. a micromanipulator-based cell selection needle or a laser-aided microdissection tool).

The system **1300** may optionally have a liquid dispenser **1325.** The liquid dispenser **1325** can be used as a mechanism to handle the delivery and transport of media. The liquid dispenser can be syringe-based. The liquid dispenser **1325** can be have one or multiple dispensing ports (e.g. 1, 2, 6, 12, 24, 96 etc.) to dispense liquid into multi-well plates (e.g. 1-well, 2-well, 6-well, 12-well, 24-well, 96-well plates, etc.). In some instances, the liquid dispenser can be a modular add-on with automated liquid dispensing capabilities (e.g. Eppendorf's epMotion, GE's IN CELL Analyzer 6000).

The system **1300** may optionally have a plate loader **1330.** The plate loader **1330** may comprise a motorized plate container that can have a capacity to hold a minimum of 1, 2, 3, 4, 5, 6, 10, 15, 20, 25, or 30 plates. The plate loader can have a modular design to allow for additional plate capacity. In some instances, the plate loader can robotically handle plates for example, to place a plate on the X-Y stage of a microscope for imaging (e.g. ASI's robot plate loader).

The system **1300** may have an X-Y-Stage **1335.** The X-Y-Stage **1335** may include a linear encoder for precise movement. The X-Y-Stage **1335** can be comprised of a stage that can hold a single plate (e.g. ASI MS-2000 Flat Top XYZ Automated Stage). The X-Y-Stage **1335** can be comprised of a large format stage with capacity to hold one, two, three, four, five, six or more plates simultaneously.

The system **1300** can have a Z-Stepper **1340.** The Z-stepper can be a motorized, piezo-driven, Z-axis control that can, e.g., be used for multiple-plane imaging.

The system **1300** can have one or more objectives. The objectives can be approximately 1X, 5X, 10X, 15X 20X, 30X, 40X, 60X, and/or 100X. In some instances, the objectives **345** will be attached to a motorized objective turret. The objectives **1345** can be phase objectives. The objectives **1345** can be microscope objectives that are suitable for both brightfield and fluorescence imaging (e.g. Zeiss Plan-Neofluar objectives). In some embodiments, the objectives **1345** must be compatible with a hardware-based auto-focus system (e.g. Nikon's Perfect Focus System).

The system **1300** can optionally contain an auto-focus system **1350.** The auto-focus system **1350** can use hardware-based image auto-focusing (e.g. Nikon's Perfect Focus System).

The system **1300** can optionally contain a filter **1355.** The filter **1355** can be an illumination filter. The filter **1355** can be dichroic. Dichroic mirrors can be used in conjunction with appropriate excitation and emission filters to fluorescence imaging-compatible wavelengths can be, for example, approximately 360nm, 488nm, and 568nm.

The system **1300** can have a camera **1360.** The camera **1360** may be charge coupled device (CCD) or complementary metal oxide semiconductor (CMOS). The camera **1360** can be compatible with fluorescence, brightfield or both. The camera **1360** can be liquid cooled. The camera **1360** can be wide-field-of-view. The camera **1360** can have wide angle imaging sensors. The camera **1360** can also comprise back-side illumination based image sensors. The camera **1360** can have the capability to transmit information. The capability to transmit information can be known fast-transfer method (e.g. firewire or USB 3.0 ports).

The system **1300** can contain an environmental chamber **1365.** The environmental chamber **1365** can comprise a sealed and light-controlled (e.g. dark) environment. The environmental chamber **1365** may optionally use an air-lock system. In some instances the use of an air-lock system can be used when access to one or more plates is needed while experiments are in progress. The environmental chamber **1300** can also comprise an ability to support a pressurized environment. In some instances, the pressurized environment can be approximately 1, 2, 3, 4, 5, 6, 7, 8, 9, 10, 11, 12, 13, 14, 15, PSI over standard atmospheric pressure (e.g., a standard atmospheric pressure of 14.7 PSIA). In some instances, the environmental chamber **1365** will be constructed to support sterilization or cleaning protocols. In some instances, the environmental chamber may comprise a de-humidification, heating, vacuum pressure control and/ or any combination thereof.

The system **1300** may optionally have a manipulator **1370.** The manipulator **1370** may be used to control the cell selector **1320.** The manipulator **1370** may be controlled by a user (e.g. joy-stick) or may be controlled by software. The manipulator may be a mechanical and pressure-hub for handling of single cells. (e.g. Eppendorf's transjector, manipulator, Cell TRAM VARIO™).

The system **1300** may optionally have a liquid handling module **1375.** The liquid handling module **1375** can be used as a reservoir for liquids (e.g. media, waste media). The liquid handling module **1375** can be used to support one or more liquids. The liquid handling module **1375** can in some instances support 1, 2, 3, 4, 5, 6, 7, 8, 9 10, 11, 12, 13, 14,15, 16, 17, 18, 19, and/or 20 solutions. In some embodiments, the liquid handling module **1375** can comprise automated sterilization capabilities.

The system **1300** can have a humidification regulator **1380.** The humidification regulator **1380** can be used to dehumidify. In some instances, dehumidification may enhance imaging and may increase the life of internal electronics.

The system **1300** can have a heating element **1385.** The heating element **1385** may be used to warm the contents of the environmental chamber **1365.** In some embodiments, the contents of the environmental chamber may be heated to approximately 26, 27, 28, 29, 30, 31, 32, 33, 34, 35, 36, 37, 38, 39, 40, 41, 42, 43, 44, or 45 degrees Celsius. In some embodiments, the heating element **1385** will comprise an integrated temperature system that can control the temperature with automated feedback.

The system **1300** can have a gas inlet **1390.** The gas inlet **1390** can have integrated gas controls and support for individual gas lines (e.g. dual valve support). In some instances the gas inlet **1390** can control the level of atmospheric gases, such as nitrogen, carbon dioxide, and/or oxygen.

The system **1300** can have a pressurized vacuum pump **1392.** The pressurized vacuum pump **1392** can have a pressure gauge and/or an integrated pressure sensor or manifold. The pressurized vacuum pump **1392** can have the capability to supply a pressure of approximately 1, 2, 3, 4, 5, 6, 7, 8, 9, or 10 PSI over atmospheric pressure. In some embodiments, the pressurized vacuum pump **1392** is capable of supplying pressure to an estimated approximate volume of 10 to 18 cubic feet.

The system **1300** can optionally have a fluorescent light source 1394. The fluorescent light source **1394** can be xenon. The fluorescent light source **1394** can be from a mercury-based lamp. The fluorescent light source **1394** can be from a solid state, or laser-diode based illumination source.

The system **1300** can have a system for data storage **1396.** The system for data storage **1395** can comprise any number of storage type media can include any or all of the tangible memory of the computers, processors or the like, or associated modules thereof, such as various semiconductor memories, tape drives, disk drives and the like, which may provide non-transitory storage at any time for the software programming. The system for data storage can also comprise any number of data communication types e.g. through the Internet or various other telecommunication networks. Data may be transmitted through optical, electrical, and /or electromagnetic waves, such as used across physical interfaces between local devices, through wired and optical landline networks and over various air-links. The physical elements that carry such waves, such as wired or wireless likes, optical links, or the like, also may be part of the system for data storage. As used herein, unless restricted to non-transitory, tangible data storage media, terms such as computer or machine "readable medium" refer to any medium that participates in providing instructions to a processor for execution. Hence data storage, may take many forms, including but not limited to, tangible storage medium, a carrier wave medium, or physical transmission medium. Non-volatile storage media can include, for example, optical or magnetic disks, such as any of the storage devices in any computer(s) or the like, such may be used to implement the system. Tangible transmission media can include: coaxial cables, copper wires, and fiber optics (including the wires that comprise a bus within a computer system). Carrier-wave transmission media may take the form of electric or electromagnetic signals, or acoustic or light waves such as those generated during radio frequency (RF) and infrared (IR) data communications. Common forms of computer-readable media therefore include, for example: a floppy disk, a flexible disk, hard disk, magnetic tape, any other magnetic medium, a CD-ROM, DVD, DVD-ROM, any other optical medium, punch cards, paper tame, any other physical storage medium with patterns of holes, a RAM, a ROM, a PROM and EPROM, a FLASH-EPROM, any other memory chip or cartridge, a carrier wave transporting data or instructions, cables, or links transporting such carrier wave, or any other medium from which a computer may read programming code and/or data. Many of these forms of computer readable media may be involved in carrying data for storage. In some embodiments, the system for data storage can be a multi-core processor.

The system **1300** can have an instrument access door **1398.** The instrument access door **1398** may provide a port of entry to the plate loader **1330** and/or the environmental chamber **1365.** The instrument access door **1398** may integrate an air-lock system.

The system **1300** can provide a platform for high-throughput uses of the invention as described herein. For example, the system 1300 can comprise a microscope. The microscope can be an inverted microscope. The microscope can be configured for live cell imaging and/or high content analysis. Microscopes configured for high content, live cell imaging are commercially available through a number of vendors, e.g., BioTek Instruments (Cytation™), Carl Zeiss Microscopy (Lightsheet Z.1 Live Cell Imaging Microscope, Cell Observer Research Microscope, PrimoVert Monitor Inverted Microscope), Molecular Devices LLC (ImageXpress® Micro XLS Widefield High Content Screening System, CloneSelect™ Imager), EMD Millipore (CellASIC™), Nikon Instruments (Biostation CT), among others. Exemplary high throughput microscopes and imaging systems are described in US Patent Application Pub. No. 20040152188, hereby incorporated by reference.

### Kits

The invention also provides kits and/or cell culture systems useful for carrying out one or more methods of the invention. Kits and/or cell culture systems can comprise any combination of (i) a cell culture plate comprising a substrate described herein, (ii) a cell culture medium as described herein, or component thereof (iii) a cell culture incubator as described herein, (iv) a cellular imaging system as described herein, and/or (v) a substrate described herein, or component thereof. Kits may also comprise instructions for use of one or more of the cell culture plate, cell culture medium, incubator, and imaging system for culturing and/or identifying a target cell subpopulation. The instructions may comprise a directive culture in environmental conditions suitable for growth of a target cell subpopulation. Environmental conditions are described herein. For example, the instructions may comprise a directive to culture the target cell under positive pressure conditions. Exemplary pressure conditions are described herein. For example, the instructions may comprise a directive to culture the target cell under hypoxic conditions. Exemplary hypoxic conditions are described herein.

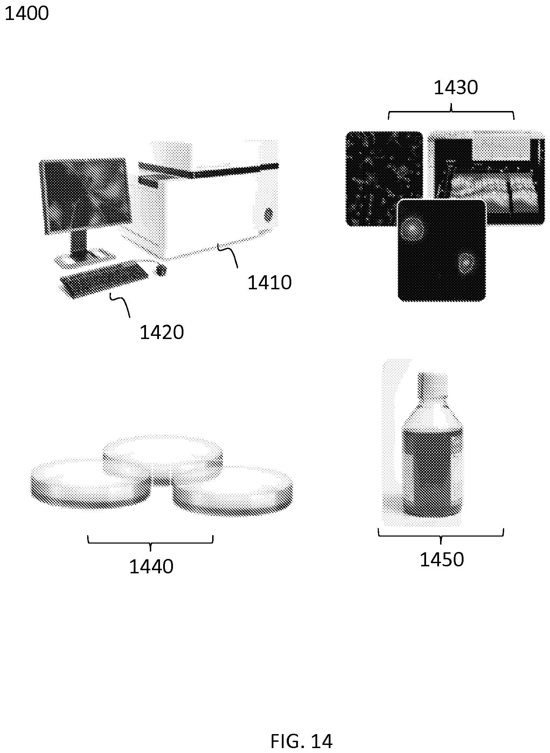

An exemplary instance of a cell culture system **1400** is depicted in **FIG. 14****.** The cell culture system **1400** can comprise a cell incubator **1410** as described herein, the incubator **1410** configured to provide one or more environmental conditions suitable for growth of a target cell subpopulation. The cell incubator may optionally comprise a microscope (not shown) as described herein. The microscope may be configured for imaging one or more members of the target cell subpopulation. The microscope may be within an enclosed environmental chamber of the microscope. The microscope may be operably coupled to a computer system **1420.** The computer system can comprise, e.g., a monitor for visual display and a keyboard. The cell culture system **1400** can comprise computer readable medium **1430** comprising software for target cell identification. The computer readable medium **1430** may be integrated into the computer system **1420.** The system can comprise a substrate **1440** as described herein. The substrate can be configured to promote selective adherence and/or growth of a target cell subpopulation. The system can comprise a cell culture medium **1450** as described herein.

### Exemplary Uses of the Invention

There are several practical applications of the present invention. The ability to enrich rare target subpopulations of cells has value as a commodity to be used for basic research, medical treatments, medical research, development and discovery of drugs and biologics, high throughput screens, antibody development, and vaccine development. CTCs, CSCs, HSCs, and EPCs are examples of cells that circulate in the blood but have been difficult to isolate and enrich in quantities sufficient for practical laboratory or medical purposes. Use of any of the methods, kits, and/or systems of the invention can enable the isolation and enrichment of a target cell subpopulation from a bodily fluid sample or derivative thereof when the target cells are present in the sample at a concentration of 10, 9, 8, 7, 6, 5, 4, 3, 2, or 1 target cell per ml of sample, or less than an average of 1 target cell per ml of sample. For example, any of the methods, kits, and/or systems of the invention can enable the isolation and enrichment of a target cell subpopulation from a bodily fluid sample or derivative thereof when the target cells are present in the sample at a concentration of about 0.8 target cells per ml of sample (e.g., 16 target cells/20 ml blood), or 0.6 target cells per ml blood (e.g., 12 target cells/20 ml blood). In another application of this invention, cultured target cell subpopulations with varying functional traits can provide a platform for highly targeted biomarker discovery. Genomic, proteomic, and metabolic analysis can be conducted on these cultured cells and the ability to generate a commercially available source of these rare cells is important for the identification of novel biomarkers to be used for development of cancer therapies (e.g. drug development), cancer vaccines, cancer screening and diagnostics, personalized antibody development, hematopoietic stem cell transplantation, organ transplantation, and cardiovascular disease treatment.

### Personalized Medicine

Target cell subpopulations can be used for personalized medicine. In the instance of CTCs and CSCs, cells can be used for chemosensitivity testing, whereby standard and exploratory chemotherapy regimens can be tested on cultured CTCs and determination of the effects of chemotherapy drugs on CTCs, including cell viability and cell division rates measurement to determine the efficacy of a given drug. Another application of the present invention is to monitor patient response to a given cancer therapy conducted by serial monitoring of their CTC population as they undergo treatment. Blood samples can be analyzed on a regular basis, before, during and after treatment whereby a positive response to therapy would results in decreased CTC viability and lower division rates.

Another application of this invention is to monitor patients who are currently in remission to investigate the potential of cancer relapse. Serially testing of their blood for CTCs can be conducted on a regular basis, to determine the potential or likelihood for cancer relapse. In some cases, serial testing can result in earlier detection of relapse.

### Drug Discovery/High Throughput Screening

Another application of the disclosure is for high content screening analysis (e.g., high throughput drug discovery) and as a direct research platform for basic research. A large supply of cells is needed to conduct a meaningful study on the efficacy of a drug on a particular cell type. Additionally, comparing the effectiveness of different drug treatments on different types of cells requires a large amount of cells. The disclosure provides for a research platform for basic research allowing for scientists to automate or partially automate the process of isolating cell types; thereby allowing for high throughput experiments to be possible. Such a research platform can have utility as a drug discovery platform. Accordingly, the invention provides a method of developing an anti-cancer therapy, comprising: (a) culturing a target cell subpopulation using methods as described herein; (b) contacting said target cell subpopulation with a test agent; (c) assaying viability and/or growth of said target cell subpopulation; and (d) selecting said test agent as an anti-cancer therapy based on said assaying. In some embodiments, the target cell subpopulation comprises CTCs and/or CSCs. In some embodiments a plurality of test agents are applied to the target cell subpopulation (e.g., testing a combination therapy). In some embodiments, the target cell subpopulation is cultured on a multi-well plate. In some embodiments, the multi-well plate comprises 6, 12, 24, 48, 96, 384, 1536, or 3456 wells. In some embodiments, the method comprises contacting said target cell subpopulation with a plurality of test agents, each test agent applied to a single well of the multi-well plate. In some embodiments, the method comprises assaying the wells of the multi-well plate simultaneously. In some embodiments, the test agent is selected as an anti-cancer therapy if it reduces the viability, propagation, or growth of the target cell subpopulation.

### Treating a Subject

The invention described herein can provide data that can be used for a medical professional to treat a subject. In a preferred embodiment, the classification of cells can direct treatment of a subject. Treatment of a subject can include diagnosis, prognosis or theranosis. Diagnoses can comprise determining the condition of a subject. Diagnosis can be conducted at one time point or on an ongoing basis. For example, a subject can be diagnosed with cancer. In another example a cancer subject who is in remission could be routinely screened to determine if a cancer relapse has occurred. Prognosis can comprise determining the outcome of a subject's disease, the chance of recovery, or how the disease will progress. For example, identifying CTCs of a certain type can provide information upon which a prognosis may be based. Theranosis can comprise determining a therapy treatment. For example, a subject's cancer therapy treatment can be comprised of chemotherapy, radiation, drug treatment, no treatment or any combination thereof. A subject may be monitored, for example by serial blood testing, to measure CTC populations before, during and after a subject undergoes treatment. A positive response to therapy would result in a decreased CTC viability and lower division rates.

In the case of isolated and classified CTCs from a cancer subject, the presence or absence of CTCs may aid or form the diagnosis of cancer. Prognosis of disease may be aided by the types of CTCs present in the subject's blood. Theranosis can be aided based on knowledge of the type of CTCs present. Theranosis may also be aided by using isolated CTCs to test the probability of success of various treatments, such as chemotherapeutic agents most likely to successful.

In some cases, target cells (e.g., isolated CTCs) can be characterized by detecting a presence or absence of one or more biomarkers in the target cells. In some embodiments, the presence or absence of one or more biomarkers can be determined by sequencing nucleic acids isolated from a target cell isolated from a subject in need of a disease therapy. The sequencing can be used to generate a genetic profile of the sequenced target cell. The genetic profile of the target cell can be used to select a personalized disease therapy for the subject. For example, such methods may be used to select a personalized cancer therapy for a subject in need thereof. In some embodiments, nucleic acid is isolated from the CTCs and optionally from normal cells using any methods known in the art. In some embodiments, the nucleic acid is DNA. In some embodiments, the DNA from the tumor sample and normal cells are used to prepare a subject-specific tumor DNA library and/or normal DNA library. DNA libraries can be used for sequencing by a sequencing platform. The sequencing platform can be a next-generation sequencing (NGS) platform. In some embodiments, the method further comprises sequencing the nucleic acid libraries using NGS technology.

Sequencing can be accomplished through classic Sanger sequencing methods which are well known in the art. Sequence can also be accomplished using high-throughput systems some of which allow detection of a sequenced nucleotide immediately after or upon its incorporation into a growing strand, e.g., detection of sequence in real time or substantially real time. In some cases, high throughput sequencing generates at least 1,000, at least 5,000, at least 10,000, at least 20,000, at least 30,000, at least 40,000, at least 50,000, at least 100,000 or at least 500,000 sequence reads per hour; with each read being at least 50, at least 60, at least 70, at least 80, at least 90, at least 100, at least 120 or at least 150 bases per read. Sequencing can be performed using genomic DNA or cDNA derived from RNA transcripts as a template.

In some embodiments, high-throughput sequencing involves the use of technology available by Helicos BioSciences Corporation (Cambridge, Mass.) such as the Single Molecule Sequencing by Synthesis (SMSS) method. SMSS is unique because it allows for sequencing the entire human genome in up to 24 hours. This fast sequencing method also allows for detection of a SNP/nucleotide in a sequence in substantially real time or real time. Finally, SMSS is powerful because, like the MIP technology, it does not require a preamplification step prior to hybridization. In fact, SMSS does not require any amplification. SMSS is described in part in US Publication Application Nos. 20060024711; 20060024678; 20060012793; 20060012784; and 20050100932, hereby incorporated by reference.

In some embodiments, high-throughput sequencing involves the use of technology available by 454 Lifesciences, Inc. (Branford, Conn.) such as the PicoTiterPlate device which includes a fiber optic plate that transmits chemiluminescent signal generated by the sequencing reaction to be recorded by a CCD camera in the instrument. This use of fiber optics allows for the detection of a minimum of 20 million base pairs in 4.5 hours.

Methods for using bead amplification followed by fiber optics detection are described in Marguiles, M., et al. "Genome sequencing in micro fabricated high-density picolitre reactors", Nature, doi:10.1038/nature03959; and well as in US Publication Application Nos. 20020012930; 20030068629; 20030100102; 20030148344; 20040248161; 20050079510, 20050124022; and 20060078909, hereby incorporated by reference.

In some embodiments, sequence analysis of the target cell's genetic material may include a four-color sequencing by ligation scheme (degenerate ligation), which involves hybridizing an anchor primer to one of four positions. Then an enzymatic ligation reaction of the anchor primer to a population of degenerate nonamers that are labeled with fluorescent dyes is performed. At any given cycle, the population of nonamers that is used is structure such that the identity of one of its positions is correlated with the identity of the fluorophore attached to that nonamer. To the extent that the ligase discriminates for complementarity at that queried position, the fluorescent signal allows the inference of the identity of the base. After performing the ligation and four-color imaging, the anchor primer: nonamer complexes are stripped and a new cycle begins. Methods to image sequence information after performing ligation are known in the art.

As a drug screening application, the invention allows for the selective screening of a new class of drugs that will specifically inhibit the 'binding' or adhesive properties of circulating tumor cells. Inhibiting the ability of CTCs to bind to other cell types, tissues, and substrates, will allow investigators to create new drugs to prevent cancer metastasis from occurring. Specific drug targets will include integrins, paxillin, talin, and E-cadherins.

In another aspect of the invention, a subject's HSCs can be cultured in parallel to their CTCs. Both cell types can be isolated together from peripheral blood, lymphatic fluids, and the bone marrow. The propagation of HSCs and their subsequent differentiation into lymphoid lineages that comprise the human immune system (Natural Killer cells, T-cells, and B-cells) can enable 'cellular-reprogramming' of said cells by exposing these cells to CTCs isolated from the same subject, e.g., by exposing the HSCs to the most aggressively growing cancer colonies found in CTC culture. This can be achieved by co-culturing a subject's immune cells to CTCs, e.g., the most aggressively dividing CTCs, in vitro. Introduction of these immune cells will allow investigators to observe their ability to recognize and destroy CTC colonies. Cultured immune cells which are demonstrably effective in perturbing CTC growth or eliminating CTC colonies entirely can be expanded in culture. Such expanded immune cells can be reintroduced into the subject as a therapeutic for cancer.

The inventions herein provide a new approach for the enrichment of target cell subpopulations from a heterogeneous cell population. The method can be applied whenever one has a heterogeneous cell population and enrichment of a target cell subpopulation is needed.

The above description is illustrative and not restrictive. Many variations of the invention will become apparent to those of skill in the art upon review of this disclosure. Merely by way of example, while the invention is illustrated primarily with regard to enrichment of target cell subpopulations, including CTCs, CSCs, HSCs, and EPCs from bodily fluids, the invention is not so limited.

### EXAMPLES

### Example 1: Preparation of an exemplary substrate:

Substrate preparation: A base layer of 0.1% porcine gelatin was pretreated with 0.1 mg/ml of Poly-L-ornithine (e.g. Poly-L-ornithine hydrochloride or Poly-L-ornithine hydrobromide). After pretreatment, the base layer was treated with approximately 0.1 mg/ml to approximately 1.0 mg/ml of collagen type I, collagen type IV, and laminin.

### Example 2: Binding affinity of circulating breast cancer cells to substrate:

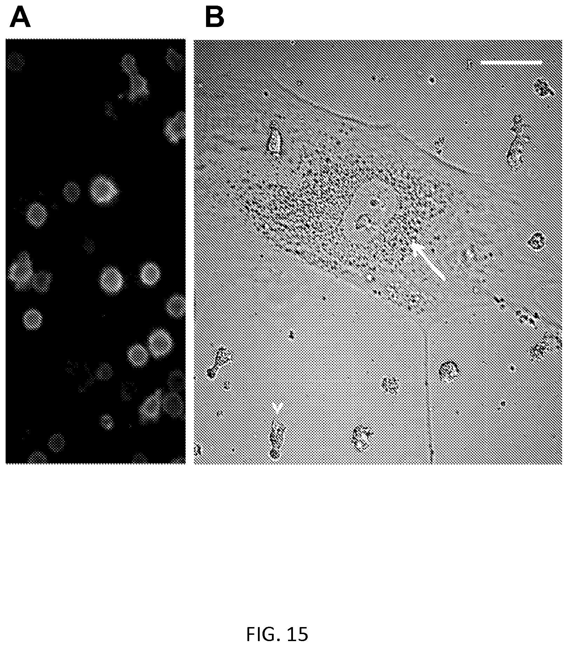

Whole blood samples were spiked with MDA-MB231 breast cancer cells. Spiked blood samples were subjected to Ficoll-based density centrifugation as described herein. The nucleated cell fraction was plated onto the substrate and cultured at 37°C, 2% O₂, and 16.7 PSIA. White blood cells (WBCs) were visualized with a fluorescently-labeled anti-CD45 antibody. FIG. 15 depicts labeled WBCs (panel A) and a circulating breast cancer cell adhered to the substrate (panel B). The adhered breast cancer cell is easily distinguishable from the WBCs (small arrowhead) by morphology.

### Example 3: Preparation of cell culture plates using GOL and GEL substrate:

GOL substrate is prepared as 0.1% porcine gelatin, 0.05 mg/mL Poly-L-ornithine hydrochloride, and 5% fetal bovine serum (FBS). GEL substrate is prepared as 0.1% porcine gelatin, and one or more of 0.1 mg/mL of collagen (e.g. Sigma C7774), 0.1 mg/mL laminin (e.g. Sigma L2020), and 0.1 mg/mL fibronectin (e.g. Sigma F0895). Cell culture plates are coated with GOL Substrate or GEL Substrate, ensuring that the substrate covered the entire surface of the well for four hours or more at 37°C. The substrate solutions are removed from the wells. Wells are washed two times with sterile distilled water. 1x phosphate buffered saline (PBS) was added to each well. Cell culture plates are then UV-sterilized for approximately 4 hours or more at room temperature.

### Example 4: Preparation of substrate comprising feeder cells:

GOL substrate is prepared and coated onto tissue culture plates as described in Example 3. Mouse embryonic fibroblasts (MEFs) are plated onto GOL-coated plates and cultured for about 48 hours, or until cells are at about 70% confluency. In another embodiment, MEFs are cultured on Millicell inserts (Millipore), wherein the inserts are inserted into wells of a tissue culture plate treated with GOL substrate. MEFs are cultured until cells were at 70% or more confluency (e.g. approximately 48 hours).

### Example 5: CTC subpopulation culturing protocol:

20 ml of peripheral blood were collected from human subjects. Peripheral blood nucleated cells (PBNC) were purified by Ficoll-based density centrifugation as described herein and then plated according to the following protocol. Plating Culture Medium was added (e.g. 2 mL/per well in a 6-well plate or 1 mL/well in a 12-well plate) to each well of a cell culture plate coated with a collagen-based substrate, comprising poly-L-ornithine and supplemented with a gelatin-agar mixture that provides substrate rigidity. 300 uL of PBNC solution (obtained from 20 mL of peripheral blood, resuspended in 2 mL of Plating Culture Medium) was added to each well for a 6-well plate (150 uL for 12-well plates). The plates were incubated at 37 degrees Celsius at 5% CO₂ for 1 hour. One hour after initial plating, the cell culture plates were tapped several times to remove loosely attached cells (e.g. white blood cells), and the cell culture medium was exchanged with pre-warmed Plating Culture Medium. The plates were then incubated in a pressurized incubator at 16.7 PSIA, 37 degrees Celsius, with 5% CO₂, 2% O₂, and 93% N₂ for 24 hours or more. After 24 hours had passed, the cell culture plates were tapped several times to remove loosely attached cells and the media was exchanged with pre-warmed Long Term Growth Medium. The cells were incubated in a pressurized incubator at 16.7 PSIA, 37 degrees Celsius, 5% CO₂, 2% O₂, and 93% N₂. Every 72 hours, approximately one third to one half of the Long Term Growth Medium was replaced with pre-warmed fresh media. Using this protocol, CTCs were detected and successfully established in culture.

### Example 6: CTC subpopulation culturing protocol using feeder cells:

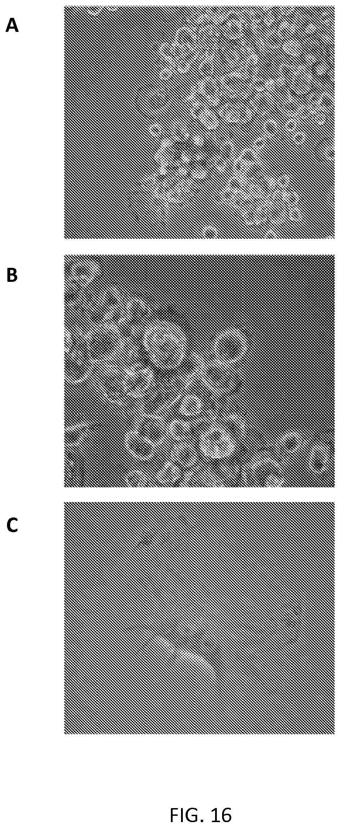

PBNCs spiked with CTC cells from a human subject with cancer were purified using Ficoll-based density centrifugation onto the GOL substrate. Freshly isolated cells were resuspended into Plating Culture Medium. A 6-well or 12-well standard size culture plate was used. The culture plate was pre-treated for a minimum of 48 hours with irradiated or non-irradiated MEF cells, until the MEF cells reached a confluency of at least approximately 70%. The MEF-enriched Culture Medium was exchanged with Plating Culture Medium. The MEF-Conditioned Culture Medium was retained. Peripheral blood nucleated cell (PBNC) Solution was generated using 20 mL of peripheral blood, spun down, and resuspended in 2 mL of Plating Culture Medium. PBNC solution was added to each well of the culture dish and cells were incubated 37 degrees Celsius with 5% CO₂ for 1 hour. One hour after initial plating, the culture plate was tapped several times to remove loosely attached cells and the media was exchanged with the previously retained MEF-Conditioned Culture Medium. The plates were incubated in a pressurized incubator under the following conditions: 16.7 PSIA, 37 degrees Celsius, 5% CO₂, 2% O₂, and 93% N₂ for a minimum of 48 hours. After 48 hours had passed, approximately one third to one half of the MEF-Conditioned Media was exchanged with pre-warmed Type R Long Term Growth Medium. The cells were then incubated again in a pressurized incubator under the following conditions: 16.7 PSIA, 37 degrees Celsius, 5% CO₂, 2% O₂, and 93% N₂ for a minimum of 48 hours. Again, half to one third of the medium was replaced with fresh, pre-warmed Type R Long Term Growth Media. Every 72 hours, approximately one half to one third of the Type R Long Term Growth Medium was replaced with pre-warmed fresh media. CTCs enriched using this protocol are depicted in FIG. 16. Panel A shows CTCs isolated from a patient under 10X magnification; panel B shows the same CTC cells under 20X magnification. Panel C depicts a multi-nucleated CTC cell at 20X magnification. The presence of one more nuclei within a single cell can indicate improper cell cycle control, often associated with cancers. These results demonstrate that such culturing conditions promote selective proliferation of target cell subpopulations (e.g., CTCs) over non-target cell subpopulations (e.g., WBCs). Such selective proliferation was evidenced by well over a two-fold increase in proliferation of the CTCs as compared to the WBCs.

### Example 7: Use of a computer readable medium for image analysis:

An exemplary software package has been developed using ImageJ (Collins (2007) ImageJ for microscopy. BioTechniques 43:S25-S30, incorporated herein by reference) to create new plugins for the detection and measurement of cell proliferation. A push-button JAVA-based plugin for ImageJ automatically detects and measures cell number and cell division rates for a given image series. A graphical user interface-based plugin supports user inputs and batch processing capabilities. The software comprises four distinct scripts (or processes) that are run sequentially to produce a total cell count.

The software program identifies proliferating cells by use of software which integrates one or any combination of the following five parameters: (1) overall cell size; (2) cell shape; (3) cell movement; (4) cell nucleus size; (5) cell division rate into a cell-classifying algorithm. The software identification process cycles through the aforementioned five parameters. The parameters are processed sequentially and/or tangentially through the use of multi-core computer chips. The software program subclassifies cells according to metastatic potential (e.g., to classify CTCs as being of a certain metastatic potential) based on cell division rate. The software program includes a user interface which allows a user to assign a different weight to each of the five parameters.

The software program identifies percentage of viable cells of a given population by determining the exact location or position at each acquisition time point. The software measures the shape and movement of a single cell. Cell shape, deformability, and movement parameters are used to determine if cells are viable. Conversely, cells that do not undergo morphological changes or exhibit movement are considered non-viable.

The software program can measure cell division rates by calculating the overall surface area of a given cell or cell colony for each stitched image at given time point. A single proliferating cell exhibiting a surface area change of at least four times its original surface area during the entire image acquisition step can be considered actively dividing. Cells or cell colonies exhibiting an increase in size can be further analyzed by enumerating the nuclei of said cells to determine their exact number.

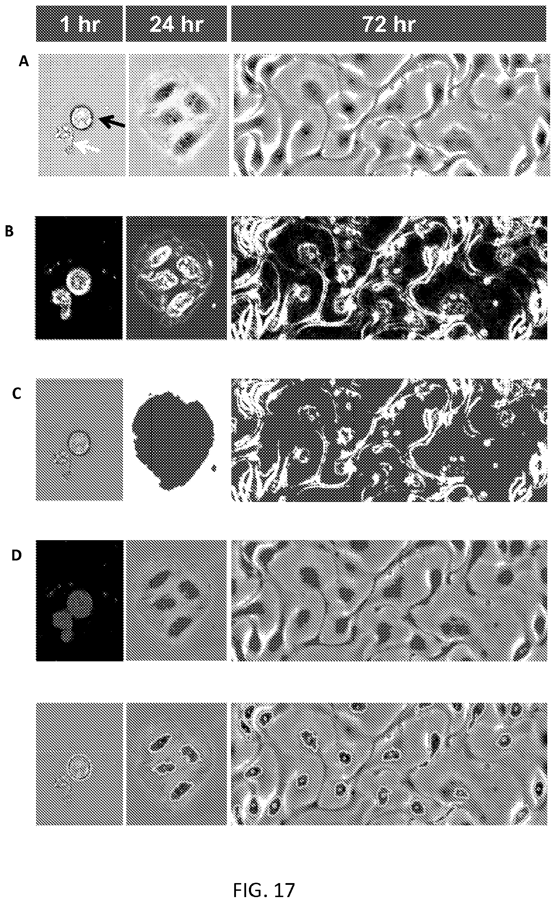

An example of live cell imaging and software analysis of dividing cancer cells can be seen in **FIG. 17****.** Breast cancer cells (MDA-MB231) obtained from cell spiking experiments as described herein (e.g., Example 2 and Example 6) were imaged at one hour, 24 hours, and 72 hours. Raw images of breast cancer cells imaged 1, 24, and 72 hours after plating are depicted in panel A. The white arrow in panel A points to a white blood cell that is attached to the substrate; the black arrow in panel A points to a breast cancer cell attached to the substrate. Panel B depicts the same images as panel A after binning and application of a variance filter. In panel C, images were automatically thresholded and segmented to provide the total surface area of a given cell colony (black and white image adapted from an original color image). Panel D depicts threshold-segmentation of the cell nuclei. Panel E depicts enumeration of the cell nuclei thresholded in panel D.

### Example 8: Long-term culture and molecular characterization of CTCs isolated from human subjects with metastatic castration resistant prostate cancer:

60 subjects with late stage metastatic castration resistant prostate cancer (>stage 3, adenocarcinoma) are recruited. A majority of recruited subjects are 65 years old or older, Caucasian males, with confirmed metastasis to the bone (most commonly associated with prostate cancer). In addition, 10 healthy adult volunteers (5 males, 5 females) are recruited for the study to serve as control subjects.

20 mL of fresh peripheral blood is collected into two CPT Vacutainer tubes from BD (cell preparation tube with sodium citrate) from each subject. Within two hours of blood collection, samples are centrifuged at 1500g for 20 minutes. Nucleated cell fractions are isolated and plated directly onto 12 well plates that are coated with a collagen-based substrate, comprising poly-L-ornithine and supplemented with a gelatin-agar mixture that provides substrate rigidity. About 2 mL of each nucleated cell fraction (containing WBCs and CTCs) is diluted into serum-free, RPMI-based, culture medium (supplemented with EGF, hydrocortisone, progesterone, and dihydrotestosterone) and dispensed equally across all twelve wells.

Freshly plated cells are then transferred into a cell culture incubator. The cell culture incubator delivers a customized gas mixture at a constant pressure of 16.7 PSIA, while maintaining humidity and temperature (37 degrees Celsius) in a completely enclosed and sterile environment. The gas mixture comprises 1% oxygen, 5% carbon dioxide, and 94% nitrogen, with oxygen levels maintained via a mixed-metal catalyst to remove excess oxygen.

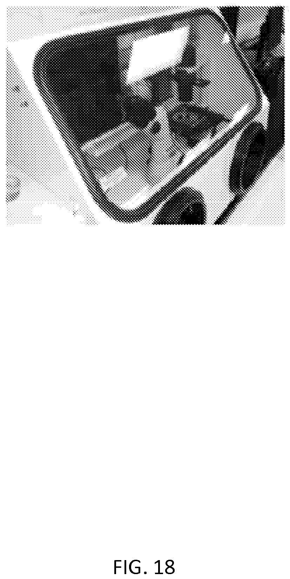

Freshly plated cells are incubated for 1 hour to allow for nucleated cells to adhere onto the substrate. WBCs loosely adhere to the substrate, and are easily detached and removed from each well via a media exchange that occurs 1 hour after cell plating. The media exchange also removes nonviable cells and membrane fragments. WBC-containing fractions are centrifuged and frozen to serve as within-subject control cells. The remaining substrate-bound cells are tightly adhered to the substrate, exhibiting cell-membrane polarization and spreading, in contrast to the spherical appearance of these cells while in suspension or when initially bound. At this point in the protocol, half the wells in the culturing plate are probed for EpCAM expressing cells using an antibody directly conjugated to an Alexa fluorophore (568nm) which is added directly to the culture medium at a concentration of 1:2000. EpCAM+ cells are counted in each well by a technician using a fluorescent microscope that is completely housed within the primary cell culture incubator (depicted in FIG. 18). The antibody containing culture medium is then exchanged for fresh culture medium after EpCAM scoring has been completed to resume cell propagation studies. Preliminary data shows that there are on average 1 to 2 EpCAM+ cells per well, allowing for quick enumeration by a trained technician. Approximately 1.5 hours after cell plating and completion of EpCAM scoring, a monolayer of feeder cells grown on Millipore's Millicell insert system (HUVEC or HLMVEC cell line, at 50% confluency, prepared 48 hours in advance using CTC culture medium) are fitted to each well to provide cell-cell signaling cues and growth factors to promote CTC proliferation, while physically separating the two cultures from potential cell contamination. Cells are then incubated for 72 hours, with fresh culture media added every 24 hours to counter medium evaporation. After 3 days in culture, cell plates are reexamined by a technician to scan for cell colonies using brightfield microscopy (phase optics). Wells containing growing cells will be probed against a cocktail negative selection antibodies directly conjugated fluorescent probe of a defined wavelength (Alexa 488nm) that will identify surface-exposed antigens of WBCs (anti-CD45), monocytes/macrophages (anti-CD14), endothelial progenitor cells (anti-CD34) and fibroblasts (anti-fibroblast surface protein). Plate wells containing cell colonies negative for these control markers are probed for anti-EpCAM, anti-PSA, and anti-Androgen Receptor in a sequential manner to determine the presence of prostate cancer associated markers.

Cell colonies which are positive for prostate cancer markers and/or cell colonies which are negative for all tested markers yet reach a minimum of 50% confluency in a span of 7 days are identified as CTC cells and selected for whole exome sequencing and comprehensive transcriptome analysis. Whole exome sequencing and comprehensive transcriptome analyses are performed by Personalis Inc. (Menlo Park, CA). CTC cultures grown to a minimum of 50% cell confluency provide enough DNA content (1 to 2 µg) to perform these set of analyses using next generation sequencing platforms. The data generated from these cells are used to confirm prostate-associated mutations, while providing comprehensive molecular profiles of rapidly dividing CTCs in culture to be used for novel biomarker studies. The remaining subject samples which may comprise less than 1 µg DNA undergo whole genome amplification (WGA) followed by gene copy number analysis via array comparative genomic hybridization (aCGH). Samples are then tested for amplification of androgen receptor, Myc gene amplification, deletions of tumor suppressor gene PTEN, and presence of gene fusions involving TMPRSS2 and ETS transcription factors ERG and ETV1.

### Example 9: Biomarker-independent selection and culture of CTCs from human subjects:

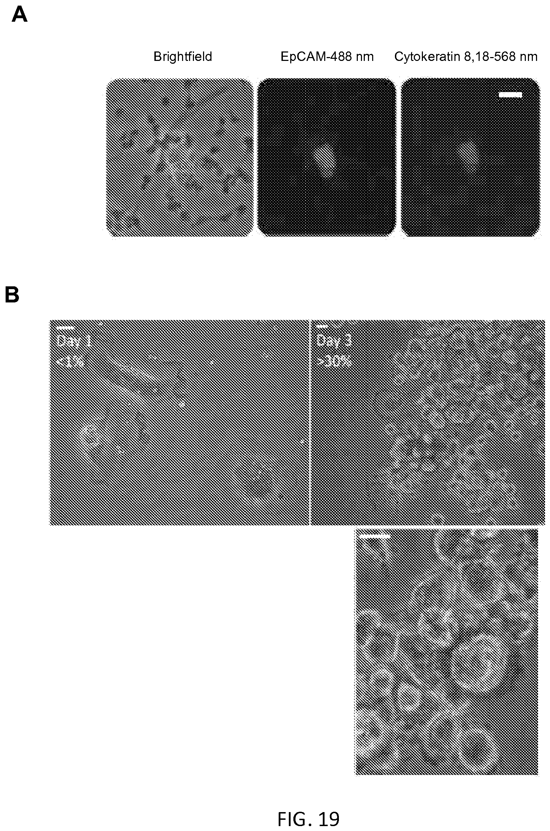

Peripheral blood samples from a diverse range of metastatic cancers were acquired from human subjects with prostate (2), breast (4), lung (4), melanoma (2), colorectal (2), esophageal (2), and pancreatic cancers (2). Patient selection criteria was kept broad, with confirmed metastatic lesions as a key determining factor for enrollment. Blood processing and CTC culture methods were performed as described in Example 8. 10 out of 18 samples contained EpCAM+, cytokeratin+, CD45- cells, while 8 out of 18 samples provided viable CTC colonies. FIG. 19 depicts photomicrographs of CTCs in culture captured from whole blood of prostate cancer patients. Panel A depicts brightfield and fluorescent images of a prostate cancer CTC positive for EPCAM and Cytokeratin 8, 18. Panel B depicts a rapidly-growing CTC culture from a patient with metastatic prostate cancer. The patient had stage IV adenocarcinoma with confirmed bone metastasis. The cell culture depicted in panel B exhibited low to no expression of EpCAM, were negative for CD45, and exhibited high cell division rates, reaching 100% confluency in less than 5 days after plating. Individual cells within these colonies differed in size and shape, while exhibiting high membrane ruffling activity. In addition, cells appeared to divide synchronously, with multiple nearby cells dividing in unison. Dividing cells often extended upward in all directions, taking on a 'bubbling' appearance. This CTC culture will be included our genetic characterization studies with Personalis as part of the 10 samples that will undergo whole exome sequencing and transcriptome analysis.

### Example 10: Chemosensitivity assay of spiked CTCs in blood:

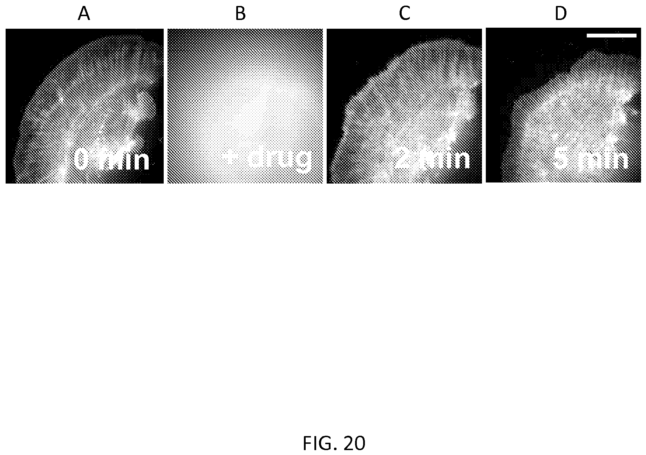

Cancer cells from spiked blood were isolated, enriched, and cultured as described herein (see, e.g., Examples 2 and 6). Cells were treated with the Rho Kinase inhibitor, Y-27632 (Ishizaki et al. 2000 Pharmacological Properties of Y-27632, a specific inhibitor of rho-associated kinases. Mol Pharmacol. 57(5):976-83, incorporated by reference herein). Results of the chemosensitivity assay are depicted in FIG. 20. Panel A shows a single cell before Y-27632 treatment. Panel B shows an image of the cell upon addition of Y-27632. The plasma membrane of the cell retracted upon drug treatment after 2 minutes (shown in Panel C) and 5 minutes (shown in Panel D).

## Claims

1. A method for selective enrichment of circulating tumor cells from a heterogeneous cell population originating from a biological sample comprising a bodily fluid, the method comprising:
(a) contacting said heterogeneous cell population with a substrate; and
(b) incubating said heterogeneous cell population under conditions sufficient to allow growth of said circulating tumor cells, wherein said conditions comprise incubating said heterogeneous cell population under a pressurised condition of 103.4 kPa (15 PSIA) or higher and hypoxic conditions, wherein said incubating results in a selective enrichment of said circulating tumor cells.

2. The method of claim 1, wherein said substrate comprises a coating, and wherein said coating is selected from:
(a) a coating comprising gelatin, a biological material associated with an extracellular matrix (ECM), and a serum;
(b) a coating comprising gelatin and one or more biological materials associated with an extracellular matrix (ECM);
(c) one or more layers of feeder cells; or
(d) any combination thereof.

3. The method of any one of the preceding claims, wherein a configuration of the substrate is selected from the group consisting of: a single layer configuration, a two layer configuration, a middle layer(s) configuration, and an inverted configuration.

4. The method of any one of the preceding claims, wherein said hypoxic conditions comprise culturing under O₂ levels that are less than 10%.

5. The method of any one of the preceding claims, wherein said hypoxic conditions comprise culturing under O₂ levels that are less than 1%.

6. The method of any one of the preceding claims, further comprising identifying a member of said circulating tumor cells upon a display of one or more morphological characteristics and/or kinetic properties by said member of said circulating tumor cells, wherein said one or more morphological characteristics and/or kinetic properties is selected from the group consisting of colony formation, proliferation, large surface area as compared to a surface area of a non-circulating tumor cell, increased adherence to said substrate as compared to an adherence of a non-circulating tumor cell to said substrate, rapid cell division, and multinucleation.

7. The method of claim 6, wherein said identifying does not comprise use of an antibody or polynucleotide marker.

8. The method of claim 6 or claim 7, wherein said biological sample comprises a bodily fluid, or a derivative thereof, and wherein said member of said circulating tumor cells is present at a concentration of 1 circulating tumor cell/ml or less of said bodily fluid or derivative thereof.

9. The method of any one of claims 6 to 8, further comprising individually isolating and culturing said member of said circulating tumor cells.

10. The method of any one of the claims 6 to 9, wherein said conditions comprise culturing said member in a cell culture medium comprising a compound that regulates surface tension of a cell.

11. The method of claim 10, wherein said compound is a Rho kinase inhibitor.

12. The method of claim 10, wherein said compound is selected from the group consisting of

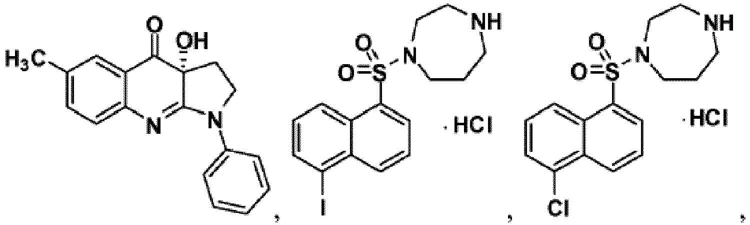

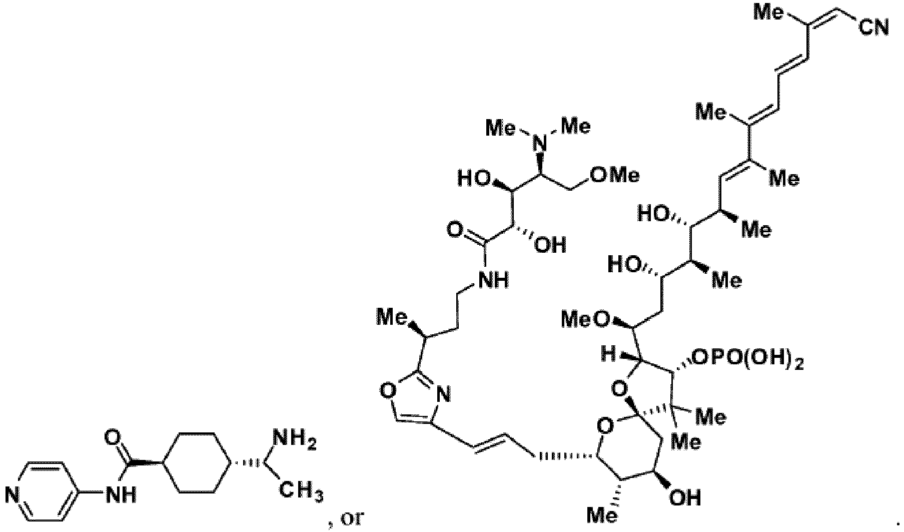

13. The method of any one of claims 6 to 12, wherein said conditions comprise culturing said member with an agent that increases expression of a hypoxia-inducible factor in said member.

## Patentansprüche

1. Verfahren zur selektiven Anreicherung von zirkulierenden Tumorzellen aus einer heterogenen Zellpopulation, die aus einer biologischen Probe umfassend eine Körperflüssigkeit stammt, wobei das Verfahren umfasst:
(a) Inkontaktbringen der heterogenen Zellpopulation mit einem Substrat und
(b) Inkubieren der heterogenen Zellpopulation unter Bedingungen, die ausreichend sind, das Wachstum der zirkulierenden Tumorzellen zu ermöglichen, wobei die Bedingungen Inkubieren der heterogenen Zellpopulation unter einer Druckbedingung von 103,4 kPa (15 PSIA) oder höher und hypoxischen Bedingungen umfassen, wobei das Inkubieren in einer selektiven Anreicherung der zirkulierenden Tumorzellen resultiert.

2. Verfahren nach Anspruch 1, wobei das Substrat eine Beschichtung umfasst, und wobei die Beschichtung ausgewählt ist aus:
(a) einer Beschichtung umfassend Gelatine, ein biologisches Material, das mit einer extrazellulären Matrix (ECM) assoziiert ist und ein Serum;
(b) einer Beschichtung umfassend Gelatine und ein oder mehrere biologische Materialien, die mit einer extrazellulären Matrix (ECM) assoziiert sind;
(c) einer oder mehreren Schichten von Feeder-Zellen oder
(d) einer beliebigen Kombination davon.

3. Verfahren nach einem der vorhergehenden Ansprüche, wobei eine Konfiguration des Substrats ausgewählt ist aus der Gruppe bestehend aus: einer einschichtigen Konfiguration, einer zweischichtigen Konfiguration, einer Mittelschicht(en)-Konfiguration und einer invertierten Konfiguration.

4. Verfahren nach einem der vorhergehenden Ansprüche, wobei die hypoxischen Bedingungen Kultivieren unter O₂-Leveln, die geringer als 10% sind, umfassen.

5. Verfahren nach einem der vorhergehenden Ansprüche, wobei die hypoxischen Bedingungen Kultivieren unter O₂-Leveln, die geringer als 1% sind, umfassen.

6. Verfahren nach einem der vorhergehenden Ansprüche, weiterhin umfassend Identifizieren eines Mitglieds der zirkulierenden Tumorzellen bei einer Anzeige von einem oder mehreren morphologischen Charakteristiken und/oder kinetischen Eigenschaften durch das Mitglied der zirkulierenden Tumorzellen, wobei die eine oder mehreren morphologischen Charakteristiken und/oder kinetischen Eigenschaften ausgewählt sind aus der Gruppe bestehend aus Koloniebildung, Proliferation, große Oberfläche im Vergleich zu einer Oberfläche einer nicht-zirkulierenden Tumorzelle, erhöhtes Anhaften an das Substrat im Vergleich zu einem Anhaften einer nicht-zirkulierenden Tumorzelle an das Substrat, schnelle Zellteilung und Mehrkernigkeit.

7. Verfahren nach Anspruch 6, wobei das Identifizieren nicht die Verwendung eines Antikörpers oder Polynukleotidmarkers umfasst.

8. Verfahren nach Anspruch 6 oder Anspruch 7, wobei die biologische Probe eine Körperflüssigkeit oder ein Derivat davon umfasst, und wobei das Mitglied der zirkulierenden Tumorzellen mit einer Konzentration von 1 zirkulierende Tumorzelle/ml oder weniger der Körperflüssigkeit oder des Derivats davon vorliegt.

9. Verfahren nach einem der Ansprüche 6 bis 8, weiterhin umfassend individuelles Isolieren und Kultivieren des Mitglieds der zirkulierenden Tumorzellen.

10. Verfahren nach einem der Ansprüche 6 bis 9, wobei die Bedingungen Kultivieren des Mitglieds in einem Zellkulturmedium umfassend eine Verbindung, die die Oberflächenspannung einer Zelle reguliert, umfasst.

11. Verfahren nach Anspruch 10, wobei die Verbindung ein Rho-Kinase-Inhibitor ist.

12. Verfahren nach Anspruch 10, wobei die Verbindung ausgewählt ist aus der Gruppe bestehend aus 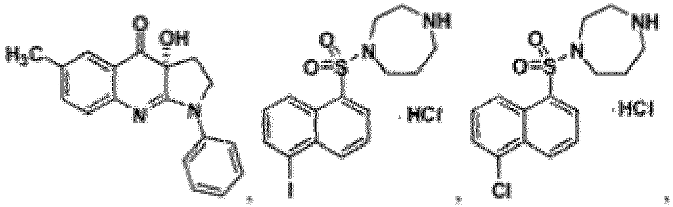 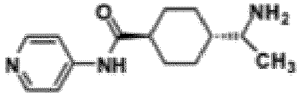 oder

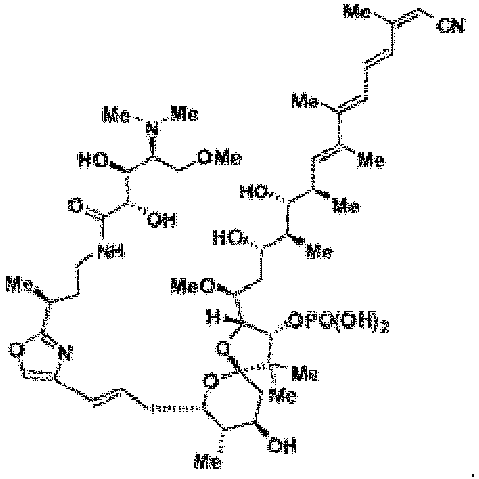

13. Verfahren nach einem der Ansprüche 6 bis 12, wobei die Bedingungen Kultivieren des Mitglieds mit einem Mittel, das die Expression eines Hypoxieinduzierbaren Faktors in dem Mitglied erhöht, umfasst.

## Revendications

1. Procédé d'enrichissement sélectif de cellules tumorales circulantes d'une population cellulaire hétérogène provenant d'un échantillon biologique comprenant un fluide corporel, le procédé comprenant les étapes consistant à :
(a) mettre en contact ladite population cellulaire hétérogène avec un substrat ; et
(b) incuber ladite population cellulaire hétérogène dans des conditions suffisantes pour permettre la croissance desdites cellules tumorales circulantes, où lesdites conditions comprennent l'incubation de ladite population cellulaire hétérogène dans une condition sous pression supérieure ou égale à 103,4 kPa (15 PSIA) et des conditions hypoxiques, où ladite incubation conduit à un enrichissement sélectif desdites cellules tumorales circulantes.

2. Procédé de la revendication 1, dans lequel ledit substrat comprend un revêtement, et dans lequel ledit revêtement est choisi entre :
(a) un revêtement comprenant de la gélatine, un matériel biologique associé à une matrice extracellulaire (MEC) et un sérum ;
(b) un revêtement comprenant de la gélatine et un ou plusieurs matériels biologiques associés à une matrice extracellulaire (MEC) ;
(c) une ou plusieurs couches de cellules nourricières ; et
(d) toute combinaison de ceux-ci.

3. Procédé de l'une quelconque des revendications précédentes, dans lequel une configuration du substrat est choisie dans le groupe consistant en : une configuration à une seule couche, une configuration à deux couches, une configuration à couche(s) intermédiaire(s), et une configuration inversée.

4. Procédé de l'une quelconque des revendications précédentes, dans lequel lesdites conditions hypoxiques comprennent la mise en culture à des niveaux de O₂ qui sont inférieurs à 10 %.

5. Procédé de l'une quelconque des revendications précédentes, dans lequel lesdites conditions hypoxiques comprennent la mise en culture à des niveaux de O₂ qui sont inférieurs à 1 %.

6. Procédé de l'une quelconque des revendications précédentes, comprenant en outre l'étape consistant à identifier un membre desdites cellules tumorales circulantes lors d'un affichage d'une ou plusieurs caractéristiques morphologiques et/ou propriétés cinétiques par ledit membre desdites cellules tumorales circulantes, où lesdites une ou plusieurs caractéristiques morphologiques et/ou propriétés cinétiques sont choisies dans le groupe consistant en la formation de colonies, la prolifération, une grande surface par rapport à une surface d'une cellule tumorale non circulante, une adhérence accrue audit substrat par rapport à une adhérence d'une cellule tumorale non circulante audit substrat, une division cellulaire rapide, et une multi-nucléation.

7. Procédé de la revendication 6, dans lequel ladite identification ne comprend pas l'utilisation d'un anticorps ou d'un marqueur polynucléotidique.

8. Procédé de la revendication 6 ou 7, dans lequel ledit échantillon biologique comprend un fluide corporel, ou un dérivé de celui-ci, et dans lequel ledit membre desdites cellules tumorales circulantes est présent à une concentration inférieure ou égale à 1 cellule tumorale circulante/ml dudit fluide corporel ou d'un dérivé de celui-ci.

9. Procédé de l'une quelconque des revendications 6 à 8, comprenant en outre les étapes consistant à isoler individuellement et à mettre en culture ledit membre desdites cellules tumorales circulantes.

10. Procédé de l'une quelconque des revendications 6 à 9, dans lequel lesdites conditions comprennent la mise en culture dudit membre dans un milieu de culture cellulaire comprenant un composé qui régule la tension superficielle d'une cellule.

11. Procédé de la revendication 10, dans lequel ledit composé est un inhibiteur de Rho kinase.

12. Procédé de la revendication 10, dans lequel ledit composé est choisi dans le groupe consistant en

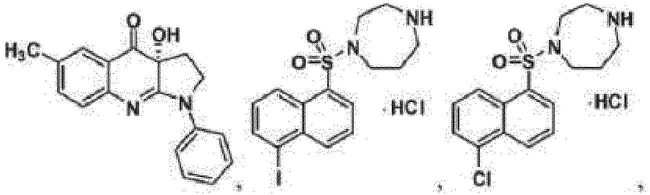

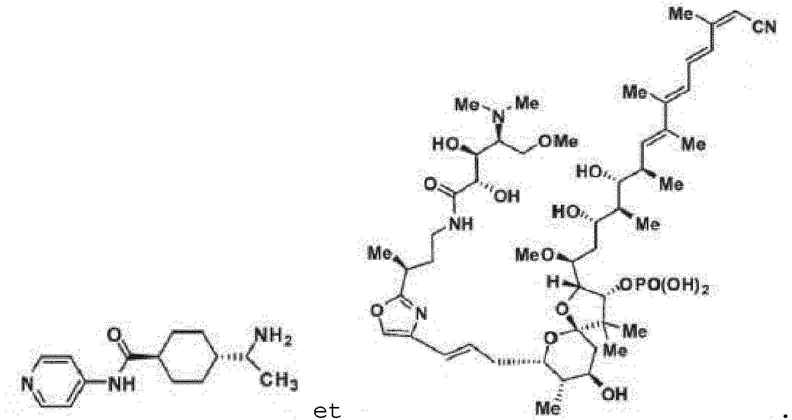

13. Procédé de l'une quelconque des revendications 6 à 12, dans lequel lesdites conditions comprennent la mise en culture dudit membre avec un agent qui augmente l'expression d'un facteur inductible par hypoxie dans ledit membre.
